# EUROPEAN PATENT APPLICATION

(11) **EP 4 850 755 A1**
(43) Date of publication of application: **09.12.2026**
(21) Application number: 25382572.3
(22) Date of filing: 04.06.2025
(51) Int. Cl.: A61P 17/06, A61P 25/28, A61P 29/00, A61P 35/00, C07D 401/06, C07D 403/06, C07D 405/14, A61K 31/416, A61K 31/454

(54) **CB2 RECEPTOR AGONISTS**

(71) Applicant: Universidade de Santiago de Compostela, 15782 Santiago de Compostela (ES)
(72) Inventor: SOTELO PÉREZ, Eddy, E-15782 Santiago de Compostela (ES); GIOÉ, Claudia, E-15782 Santiago de Compostela (ES); ORTIGUEIRA NOYA, Sandra, E-15782 Santiago de Compostela (ES); PRIETO DÍAZ, Rubén, E-15782 Santiago de Compostela (ES); LOZA GARCÍA, María Isabel, E-15782 Santiago de Compostela (ES); BREA FLORIANI, José Manuel, E-15782 Santiago de Compostela (ES)
(74) Representative: ABG Intellectual Property Law, S.L.

(57) **Abstract**

The present invention relates to highly selective CB₂ receptor agonists of formula (I) with potent anti-inflammatory and neuroprotective properties, to their use in the treatment and/or prevention of neuroinflammatory and neurodegenerative diseases, and to processes for the preparation of said compounds of formula (I).

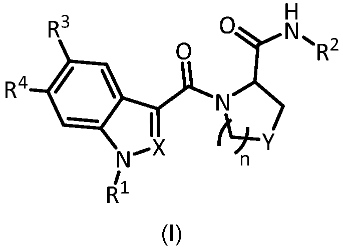

## Description

### FIELD OF INVENTION

The present invention relates to highly selective CB₂ receptor agonists with potent anti-inflammatory and neuroprotective properties, to their use in the treatment and/or prevention of neuroinflammatory and neurodegenerative diseases, to processes for the preparation of said CB₂ receptor agonists, and to intermediate compounds for the synthesis of said highly selective CB₂ receptor agonists.

### BACKGROUND OF INVENTION

The endocannabinoid system (ECS) is a crucial regulatory network responsible for maintaining homeostasis in diverse physiological processes within the human body [Di Marzo et al., Nat. Rev. Neurosci. 2015, 16, 30-42; Pacher et al., Pharmacol. Rev. 2006, 58, 389-462]. The ECS comprises endogenous cannabinoids, cannabinoid receptors (CBRs: CB₁R and CB₂R), and approximately 20 associated enzymes involved in the biosynthesis and degradation of endocannabinoids, as well as hundreds of lipid mediators. It modulates a wide range of physiological functions, including appetite, pain sensation, immune responses, mood, and memory [Busquets-García et al., Nat. Metab. 2022, 4, 848-855; Di Marzo et al., Neurotherapeutics 2015, 12, 692-698. CB₁R and CB₂R, both class A GPCRs with high transmembrane sequence similarity [Munro et al., Nature 1993, 365, 61-65; Latek et al., J. Mol. Model. 2011, 17, 2353-2366], have distinct distributions and physiological roles, making them key regulators of the endocannabinoid system [Hua et al., Cell 2016, 167, 750-762.e14; Li et al., Cell 2019, 176, 459-467.e13; Atwood and Mackie, Br. J. Pharmacol. 2010, 160, 467-479]. Since the discovery and cloning of CBRs, interest in understanding their signaling mechanisms, pharmacology, and therapeutic potential has grown significantly [Maccarrone et al., Pharmacol. Rev. 2023, 75, 885-958; Matsuda et al., Nature 1990, 346, 561-564].

The CB₂R, initially dubbed as the peripheral cannabinoid receptor, due to its early discovery and abundance in immune system cells, plays a crucial role in regulating immune response and inflammation [[Munro et al., Nature 1993, 365, 61-65; Atwood and Mackie, Br. J. Pharmacol. 2010, 160, 467-479; Turcotte et al., Cell. Mol. Life Sci. 2016, 73, 4449-4470]. More recently, high levels of CB₂R expression have been confirmed in different brain regions, including the cortex, striatum, amygdala, cerebellum, and microglia [Li et al., Neuroscience 2015, 311, 253-267; Zhang et al., Addict. Biol. 2017, 22, 752-765]. Notably, under conditions of inflammation or glial activation, CB₂R expression undergoes a significant upregulation. Due to this widespread expression pattern, CB₂R is involved in a multitude of neurological functions within the brain [Atwood and Mackie, Br. J. Pharmacol. 2010, 160, 467-479; Turcotte et al., Cell. Mol. Life Sci. 2016, 73, 4449-4470]. Emerging evidence supports a critical role for CB₂R in mediating neuroprotection and neuroinflammation [Navarro et al., Front. Neurosci. 2016, 10, 406; Vuic et al., Biomedicines 2022, 10, 3000].

Activation of the CB₂R by selective small molecules offers significant neuroprotective potential, particularly for neurodegenerative diseases, such as Alzheimer's disease, Parkinson's disease, multiple sclerosis, amyotrophic lateral sclerosis (ALS), Huntington's disease, and neuropathic pain [Cabral et al., Expert Rev. Mol. Med. 2009, 11, e3]. Primarily expressed in immune cells and microglia in the CNS, CB₂R activation reduces neuroinflammation by lowering pro-inflammatory cytokines and promoting an anti-inflammatory state [Bala et al., Psychopharmacology (Berl.) 2024, 241, 1939-1954].This effect is critical in pathologies such as Alzheimer's, Parkinson's, and Multiple Sclerosis, where inflammation plays a key role [Vuic et al., Biomedicines 2022, 10, 3000; Benito et al., J. Neurosci. 2003, 23, 11136-11141]. Additionally, CB₂R activation has been shown to decrease oxidative stress and apoptosis in neurons, supporting cell survival. Unlike CB₁R, CB₂R modulation avoids psychoactive effects, making it an attractive target for neuroprotective therapies.

Dysregulation of the CB₂R produces immunomodulatory effects, influencing cytokine release, cell migration, and immune cell proliferation and has been implicated in various pathological conditions, highlighting its involvement in chronic inflammation and autoimmune disorders, such as rheumatoid arthritis, inflammatory bowel disease (such as Crohn's disease and ulcerative colitis), systemic lupus erythematosus, and psoriasis [Turcotte et al., Cell. Mol. Life Sci. 2016, 73, 4449-4470; Zhang et al., Addict. Biol. 2017, 22, 752-765].

The CB₂R's involvement in neuronal damage and neuroinflammation underscores its potential as a therapeutic target for neuroprotection and the management of central nervous system pathologies with an inflammatory basis [Rom et al., J. Neuroimmune Pharmacol. 2013, 8, 608-620]. Indeed, by modulating the activity of microglia, the primary immune cells in the brain, CB₂R agonists can suppress the production of pro-inflammatory cytokines and enhance the clearance of toxic protein aggregates implicated in neurodegenerative processes, promoting neurogenesis and neuroplasticity, facilitating neuronal repair and regeneration [Ehrhart et al., J. Neuroinflammation 2005, 2, 29; Intranuovo et al., J. Med. Chem. 2023, 66, 235-250; Graziano et al., Eur. J. Med. Chem. 2023, 248, 115109]. Additionally, preclinical data and clinical studies have demonstrated the efficacy of CB₂R agonists in ameliorating autoimmune responses, reducing inflammation, and alleviating chronic pain without the psychoactive effects associated to CB₁R receptor activation [Whiting et al., JAMA 2015, 313, 2456-2473]. CB₂R activation has been linked to anti-inflammatory and immunomodulatory effects, making it a target in conditions where chronic inflammation plays a central role in disease progression, including cancer, neurodegenerative diseases, autoimmune diseases, and obesity, in particular in cancer-related conditions associated with chronic inflammation and immune dysregulation, such as glioblastoma, pancreatic cancer, breast cancer, and colorectal cancer, as well as chronic inflammatory diseases, such as diabetic retinopathy, chronic obstructive pulmonary disease (COPD), and atherosclerosis [Navarro et al., Front. Neurosci. 2016, 10, 406*;* Han et al., J. Med. Chem. 2013, 56, 8224-8256; André et al., Int. J. Biochem. Cell Biol. 2010, 42, 1788-1801; Morales et al., J. Med. Chem., 2015, 58, 2256-2264; Han et al., J. Med. Chem., 2013, 56, 8224-8256; Chakravarti et al., Oncotarget, 2014, 5, 5852-5872; Capozzi et al., Int. J. Mol. Sci., 2018, 19(7), 1958; Kovalchuk et al., Cell Cycle, 2020, 1-29].

Indole and indazole derivatives are among the earliest identified and most studied synthetic CBR ligands [Shafi et al., Ther. Adv. Psychopharmacol. 2020, 10, 2045125320967197]. While these ligands inspired the early development of CBR agonists, a significant number of derivatives based on these scaffolds have emerged as designer drugs of abuse since the 2000s, under the misleading claim of being synthetic, legal and safe alternatives to marijuana, such as the indole and indazole derivatives depicted below [Shafi et al., Ther. Adv. Psychopharmacol. 2020, 10, 2045125320967197; Zapata et al, Microchem. J. 2021, 163, 105877]. Despite their exceptional potency and efficacy, these compounds have promiscuous effects on CB₁R and CB₂R [Abbate et al., Pure Appl. Chem. 2018, 90, 1255-1282; Banister and Connor, The Chemistry and Pharmacology of Synthetic Cannabinoid Receptor Agonists as New Psychoactive Substances: Origins. In New Psychoactive Substances: Pharmacology, Clinical, Forensic and Analytical Toxicology; Maurer, H. H., Brandt, S. D., Eds.; Springer International Publishing: Cham, 2018; pp 165-190].

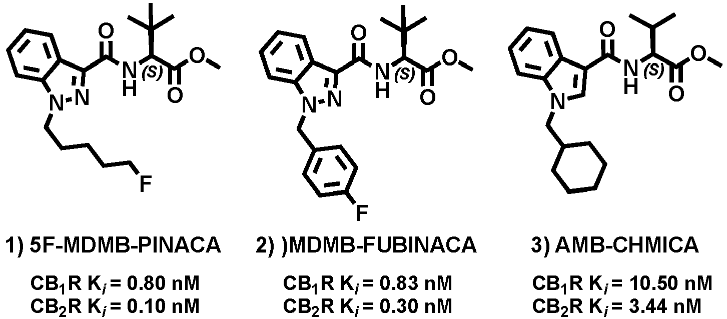

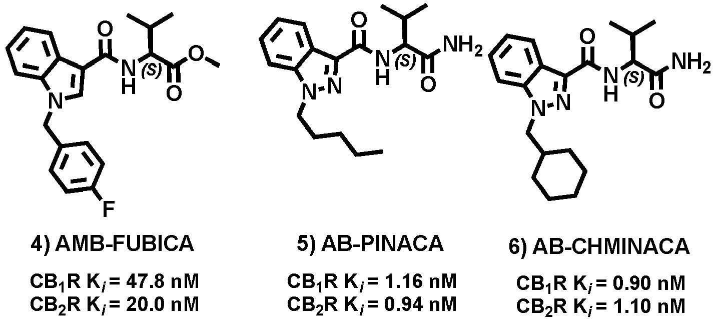

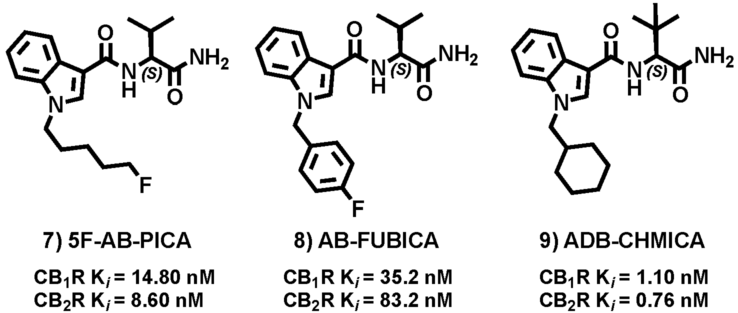

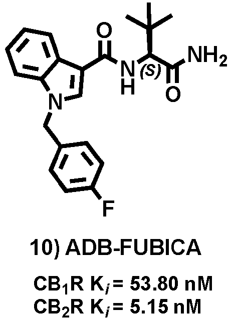

Recent reports have highlighted medicinal chemistry efforts aimed at dissociating the intrinsically promiscuous cannabinomimetic profiles of designer drugs to develop selective CB₂R agonists [Moir et al., Eur. J. Med. Chem. 2019, 180, 291-309]. Despite important advances in structural biology of cannabinoid receptors, the high homology between these receptors continues to pose significant challenges, making this goal elusive.

Thus, there is a need for the development of selective CB₂R agonists.

### SUMMARY OF INVENTION

The inventors have found a family of highly selective CB₂ receptor agonists, in particular with substantially no agonism towards the CB₁ receptor. This selective agonism is evidenced in the examples of the present invention. The compounds of the present invention have excellent CNS permeability and demonstrate remarkable anti-inflammatory and neuroprotective properties.

Thus, in the first aspect, the present invention relates to a compound of formula (I): 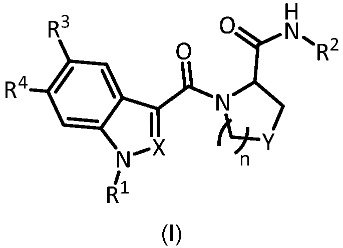 wherein
R¹ is selected from the group consisting of C₁-C₆ alkyl, -CH₂-C₃-C₈ cycloalkyl, and -CH₂-C₆-C₁₂ aryl, wherein each of said groups is optionally substituted by one or more halogen atoms;
R² is selected from the group consisting of H, C₁-C₆ alkyl, -(CH₂)ₚ-C₃-C₈ cycloalkyl, -(CH₂)ₚ-C₆-C₁₂ aryl, -(CH₂)ₚ-CH₂-heteroaryl wherein said heteroaryl is a 6- to 12-memebered monocyclic or bicyclic ring system containing one, two or three heteroatoms selected from N, O and S, -(CH₂)ₚ- heterocyclyl wherein said heterocyclyl is 3- to 8-membered monocyclic ring containing one or two heteroatoms selected from N, O and S, -CH₂C(=O)OR^{a}, and -CH₂-C(=O)NR^{a}; wherein p is selected from 0, 1 and 2; and R^{a} is H or C₁-C₆ alkyl;
R³ and R⁴ are independently selected from H and halogen atom, with the proviso that at least one of R³ and R⁴ is H;
X is CH or N;
Y is selected from the group consisting of CH₂, O, -NR^{b}, and -NC(=O)R^{b}, and - NC(=O)OR^{b}; wherein R^{b} is selected from C₁-C₆ alkyl and H; and
n is 1 or 2;
provided that when R² is H, n is 2;
or a stereoisomer or a pharmaceutically acceptable salt thereof.

In a second aspect, the invention relates to a pharmaceutical composition comprising a compound of formula (I), or a stereoisomer or a pharmaceutically acceptable salt thereof, as defined in the first aspect, and a pharmaceutically acceptable excipient.

In a third aspect, the invention relates to a process for the preparation of a compound of formula (I) according to the first aspect, wherein R² is selected from the group consisting of C₁-C₆ alkyl, -(CH₂)ₚ-C₃-C₈ cycloalkyl, -(CH₂)ₚ-C₆-C₁₂ aryl, -(CH₂)ₚ-CH₂-heteroaryl wherein said heteroaryl is a 6- to 12-memebered monocyclic or bicyclic ring system containing one, two or three heteroatoms selected from the N and O, -(CH₂)ₚ-heterocyclyl wherein said heterocyclyl is 3- to 8-membered monocyclic ring containing one or two heteroatoms selected from N and O, -CH₂C(=O)OR^{a}, and -CH₂-C(=O)NR^{a}; wherein p is selected from 0, 1 and 2; and R^{a} is H or C₁-C₆ alkyl, and
wherein the process comprises the Ugi-Joullié reaction of a compound of formula (II) with a compound of formula (V) and a compound of formula (VI) to give the compound of formula (I), 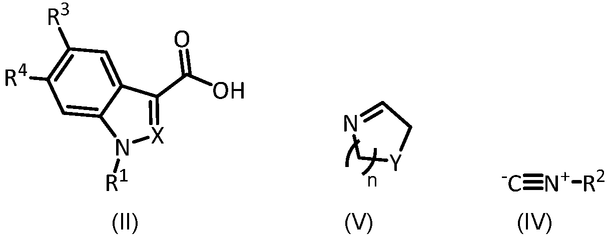 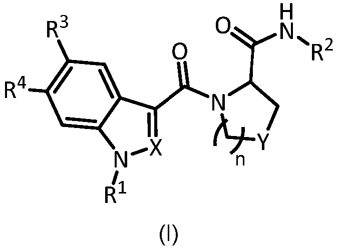
wherein R¹, R³, R⁴, X, Y and n are as defined for the compounds of formula (I) in the first aspect, and R² is as defined above.

In a fourth aspect, the invention relates to a process for the preparation of a compound of formula (I) according to the first aspect, wherein R² is H and n is 2, and wherein the process comprises:
a) amidation of a compound of formula (II) with a compound of formula (III) to give a compound of formula (IV) 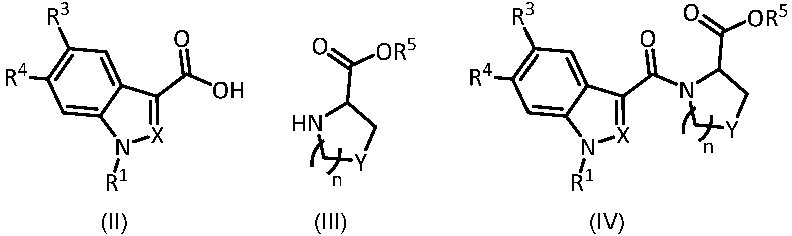 wherein R¹, R³, R⁴, X and Y are as defined for the compounds of formula (I) in the first aspect, n is 2 and R⁵ is C₁-C₃ alkyl;
b) ammonolysis of the compound of formula (IV) obtained in step a) to a compound of formula (I) 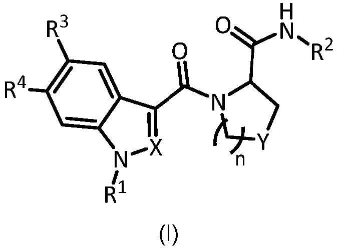 wherein R¹, R³, R⁴, X and Y are as defined for the compounds of formula (I) in the first aspect, n is 2 and R² is H.

In a fifth aspect, the invention relates to a compound of formula (IV) 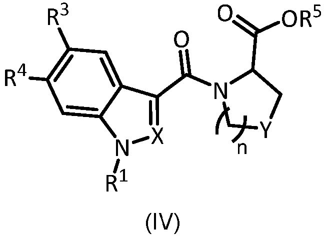 wherein R¹, R³, R⁴, X and Y are as defined for the compounds of formula (I) in the first aspect, n is 2 and R⁵ is C₁-C₃ alkyl.

In a sixth aspect, the invention relates to a compound of formula (I), or a stereoisomer or a pharmaceutically acceptable salt thereof, as defined in the first aspect, for use in medicine.

In a further aspect, the invention relates to a compound of formula (I), or a stereoisomer or a pharmaceutically acceptable salt thereof, as defined in the first aspect, for use in the treatment and/or prevention of a disease selected from the group consisting of pathologies neuroinflammatory and neurodegenerative diseases, such as Alzheimer's disease, Parkinson's disease, multiple sclerosis, amyotrophic lateral sclerosis (ALS), Huntington's disease, and neuropathic pain; peripheral inflammatory diseases, such as rheumatoid arthritis, inflammatory bowel disease (such as Crohn's disease and ulcerative colitis), systemic lupus erythematosus, and psoriasis; cancer-related conditions associated with chronic inflammation and immune dysregulation, such as glioblastoma, pancreatic cancer, breast cancer, and colorectal cancer; chronic inflammatory diseases, such as diabetic retinopathy, chronic obstructive pulmonary disease (COPD), and atherosclerosis.

### DESCRIPTION OF INVENTION

### Definitions

As used herein, the term "alkyl" refers to a straight or branched hydrocarbon chain radical consisting of carbon and hydrogen atoms, containing no unsaturation, having the indicated number of carbon atoms (such as 1 to 6), and which is attached to the rest of the molecule by a single bond. Examples of alkyl are methyl, ethyl, n-propyl, isopropyl, n-butyl, isobutyl, sec-butyl, *tert-butyl,* n-pentyl, 2-mehtylbutan-2-yl, 2,2-dimethylpropyl, 3-methylbutyl, 1-methylbutyl, 1-ethylpropyl, 1,2-dimethylpropyl, 2-methylpropyl, n-hexyl, etc.

As used herein, the term "cycloalkyl" refers to a saturated carbocyclic ring the indicated number of carbon atoms (such as 3 to 8). Examples of cycloalkyl are cyclopropyl, cyclobutyl, cyclopentyl, cyclohexyl, cycloheptyl and cyclooctyl.

As used herein, the term "aryl" refers to aromatic hydrocarbon radical having the indicated number of carbon atoms (such as 6 to 12), such as phenyl and naphthyl.

As used therein, the term "halogen" refers to an atom selected from the group consisting of fluorine, chlorine, bromine and iodine, preferably fluorine.

As used herein, the term "heterocyclyl" refers to a monocyclic ring having the indicated number of ring atoms (such as 3 to 8, preferably 5 to 7, more preferably 6), wherein the ring atoms are carbon atoms and one or more heteroatoms (such as one or two heteroatoms) selected from N, O and S, preferably selected from N and O. Examples of heterocyclyl are morpholinyl, piperidinyl, piperazinyl, and tetrahydrofuranyl; preferably morpholinyl.

As used herein, the term "heteroaryl" refers to a monocyclic or bicyclic ring system containing the indicated number of ring atoms (such as 6 to 12), wherein at least one of the rings is an aromatic ring, wherein the ring atoms are carbon atoms and one or more heteroatoms (such as one, two or three heteroatoms) selected from N, O and S, preferably selected from N and O. Examples of heteroaryl rings are pyridyl, benzodioxanyl, quinolyl, isoquinolyl, indolyl, oxazolyl, and isoxazolyl; preferably pyridyl and benzodioxanyl.

As used herein, the term "heteroatom" refers to an atom selected from the group consisting of N, O and S, preferably selected from the group consisting of N and O.

As used herein, the term "stereoisomer" refers to compounds made up of the same atoms and bonded by the same sequence of bonds but having different three-dimensional structures which are not interchangeable. All stereoisomers of the compounds referred to herein, and mixtures thereof, are considered within the scope of the present invention. Thus, any given compound referred to herein is intended to represent any one of a racemate, one or more enantiomeric forms, one or more diastereomeric forms, and mixtures thereof.

As used herein, the term "pharmaceutically acceptable salt" refers to any salt, which, upon administration to the recipient is capable of providing (directly or indirectly) a compound as described herein. The preparation of salts can be carried out by methods known in the art. For instance, pharmaceutically acceptable salts of compounds provided herein may be acid addition salts, base addition salts or metallic salts, and they can be synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, ammonium, and organic alkali salts such as, for example, ethylenediamine, ethanolamine, N,N-dialkylenethanolamine, triethanolamine, glucamine and basic aminoacids salts. Examples of the metallic salts include, for example, sodium, potassium, calcium, magnesium, aluminium and lithium salts.

### Compounds of formula (I)

The first aspect of the present invention relates to a compound of formula (I): 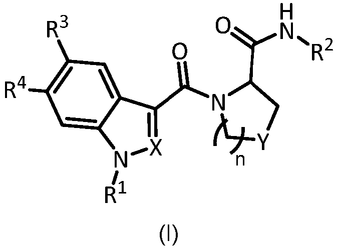 wherein
R¹ is selected from the group consisting of C₁-C₆ alkyl, -CH₂-C₃-C₈ cycloalkyl, and -CH₂-C₆-C₁₂ aryl, wherein each of said groups is optionally substituted by one or more halogen atoms;
R² is selected from the group consisting of H, C₁-C₆ alkyl, -(CH₂)ₚ-C₃-C₈ cycloalkyl, -(CH₂)ₚ-C₆-C₁₂ aryl, -(CH₂)ₚ-CH₂-heteroaryl wherein said heteroaryl is a 6- to 12-memebered monocyclic or bicyclic ring system containing one, two or three heteroatoms selected from N, O and S, -(CH₂)ₚ- heterocyclyl wherein said heterocyclyl is 3- to 8-membered monocyclic ring containing one or two heteroatoms selected from N, O and S, -CH₂C(=O)OR^{a}, and -CH₂-C(=O)NR^{a}; wherein p is selected from 0, 1 and 2; and R^{a} is H or C₁-C₆ alkyl;
R³ and R⁴ are independently selected from H and halogen atom, with the proviso that at least one of R³ and R⁴ is H;
X is CH or N;
Y is selected from the group consisting of CH₂, O, -NR^{b}, and -NC(=O)R^{b}, and - NC(=O)OR^{b}; wherein R^{b} is selected from C₁-C₆ alkyl and H; and
n is 1 or 2;
provided that when R² is H, n is 2;
or a stereoisomer or a pharmaceutically acceptable salt thereof.

In a preferred embodiment, in the compound of formula (I) R¹ is selected from the group consisting of C₃-C₆ alkyl, -CH₂-C₅-C₇ cycloalkyl, and -CH₂-phenyl, wherein each of said groups is optionally substituted by one fluorine atom. More preferably, R¹ is selected from the group consisting of C₄-C₅ alkyl optionally substituted with one fluorine atom, -CH₂- cyclohexyl, and -CH₂-phenyl substituted with one fluorine atom. Still more preferably, R¹ is selected from the group consisting of fluoropentyl, fluorobenzyl and -CH₂-cyclohexyl. Even more preferably, R¹ is selected from the group consisting of fluoropentyl and fluorobenzyl.

In a preferred embodiment, in the compound of formula (I) R² is selected from the group consisting of H, C₁-C₆ alkyl, -(CH₂)ₚ-C₃-C₈ cycloalkyl, -(CH₂)ₚ-C₆-C₁₂ aryl, -(CH₂)ₚ-CH₂- heteroaryl wherein said heteroaryl is a 6- to 12-memebered monocyclic or bicyclic ring system containing one, two or three heteroatoms selected from N and O, -(CH₂)ₚ- heterocyclyl wherein said heterocyclyl is 3- to 8-membered monocyclic ring containing one or two heteroatoms selected from N and O, -CH₂C(=O)OR^{a}, and -CH₂-C(=O)NR^{a}; wherein p is selected from 0, 1 and 2; and R^{a} is H or C₁-C₆ alkyl. More preferably, R² is selected from the group consisting of H, C₄-C₆alkyl, -C₅-C₇ cycloalkyl, -CH₂-phenyl, -CH₂-heteroaryl wherein said heteroaryl is a 6- to 12-membered monocyclic or bicyclic ring system containing one or two heteroatoms selected from N and O, -(CH₂)₂-heterocyclyl wherein said heterocyclyl is a 5- to 7-memebered monocyclic ring containing one or two heteroatoms selected from N and O, and -CH₂-C(=O)N-C₁-C₃ alkyl. Still more preferably, R² is selected from the group consisting of H, *tert-butyl,* cyclohexyl, -CH₂-phenyl, -CH₂-pyridyl, -CH₂-C(=O)NCH₃, -CH₂-benzodioxanyl, and -(CH₂)₂-morpholinyl. Even more preferably, R² is selected from the group consisting of H, *tert-butyl,* cyclohexyl, -CH₂-phenyl, -CH₂-pyridyl, and -CH₂-C(=O)NCH₃. Still more preferably, R² is selected from the group consisting of *tert-butyl,* cyclohexyl, -CH₂-phenyl, -CH₂-pyridyl, and -CH₂-C(=O)NCH₃.

In a preferred embodiment, R³ and R⁴ are independently selected from H and F, with the proviso that at least one of R³ and R⁴ is H. Preferably R³ and R⁴ are both H.

In one embodiment, in the compound of formula (I) X is CH. In another embodiment, in the compound of formula (I) X is N.

In a preferred embodiment, in the compound formula (I) Y is selected from CH₂ and O. In one embodiment Y is CH₂. In another embodiment Y is O.

In a preferred embodiment, in the compound of formula (I) n is 2.

In a preferred embodiment, in the compound of formula (I):
- n is 2 and Y is CH₂,
- n is 2 and Y is O, or
- n is 1 and Y is CH₂.

In a more preferred embodiment, in the compound of formula (I) n is 2 and Y is CH₂ or O.

In a particular embodiment, the compound of the first aspect has formula (I'): 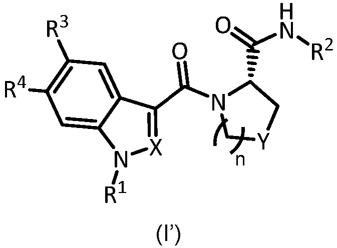 wherein R¹, R², R³, R⁴, X, Y and n are as defined above, or a pharmaceutically acceptable salt thereof.

In a preferred embodiment, the compound of formula (I) is selected from the group consisting of:
1-(1-pentyl-1H-indole-3-carbonyl)piperidine-2-carboxamide,
1-(1-(5-fluoropentyl)-1H-indole-3-carbonyl)piperidine-2-carboxamide,
1-(1-(cyclohexylmethyl)-1H-indole-3-carbonyl)piperidine-2-carboxamide,
1-(1-(4-fluorobenzyl)-1H-indole-3-carbonyl)piperidine-2-carboxamide,
1-(1-pentyl-1H-indazole-3-carbonyl)piperidine-2-carboxamide,
1-(1-(5-fluoropentyl)-1H-indazole-3-carbonyl)piperidine-2-carboxamide,
1-(1-(cyclohexylmethyl)-1H-indazole-3-carbonyl)piperidine-2-carboxamide,
1-(1-(4-fluorobenzyl)-1H-indazole-3-carbonyl)piperidine-2-carboxamide,
*N*-(*tert*-butyl)-1-(1-(4-fluorobenzyl)-1*H*-indole-3-carbonyl)piperidine-2-carboxamide,
*N*-benzyl-1-(1-(4-fluorobenzyl)-1*H*-indole-3-carbonyl)piperidine-2-carboxamide,
*N*-(2,3-dihydrobenzo[*b*][1,4]dioxin-6-yl)-1-(1-(4-fluorobenzyl)-1*H*-indole-3-carbonyl)piperidine-2-carboxamide,
methyl (1-(1-(4-fluorobenzyl)-1H-indole-3-carbonyl)piperidine-2-carbonyl) glycinate,
*N*-cyclohexyl-1-(1-(4-fluorobenzyl)-1*H*-indazole-3-carbonyl)piperidine-2-carboxamide,
*N*-benzyl-1-(1-(4-fluorobenzyl)-1*H*-indazole-3-carbonyl)piperidine-2-carboxamide,
*N*-(2,3-dihydrobenzo[*b*][1,4]dioxin-6-yl)-1-(1-(4-fluorobenzyl)-1*H*-indazole-3-carbonyl)piperidine-2-carboxamide,
1-(1-(4-fluorobenzyl)-1*H*-indazole-3-carbonyl)-*N*-(2-(methylamino)-2-oxoethyl) piperidine-2-carboxamide,
*N*-(*tert*-butyl)-1-(1-(cyclohexylmethyl)-1*H*-indole-3-carbonyl)piperidine-2-carboxamide,
*N*-cyclohexyl-1-(1-(cyclohexylmethyl)-1*H*-indole-3-carbonyl)piperidine-2-carboxamide,
1-(1-(cyclohexylmethyl)-1*H*-indole-3-carbonyl)-*N*-(pyridin-3-ylmethyl)piperidine-2-carboxamide,
methyl (1-(1-(cyclohexylmethyl)-1*H*-indole-3-carbonyl)piperidine-2-carbonyl)glycinate,
*N*-(*tert*-butyl)-1-(1-(cyclohexylmethyl)-1*H*-indazole-3-carbonyl)piperidine-2-carboxamide,
*N*-cyclohexyl-1-(1-(cyclohexylmethyl)-1*H*-indazole-3-carbonyl)piperidine-2-carboxamide,
1-(1-(cyclohexylmethyl)-1*H*-indazole-3-carbonyl)-*N*-(pyridin-3-ylmethyl)piperidine-2-carboxamide,
1-(1-(cyclohexylmethyl)-1*H*-indazole-3-carbonyl)-*N*-(2,3-dihydrobenzo[*b*][1,4]dioxin-6-yl)piperidine-2-carboxamide,
*N*-(*tert*-butyl)-1-(1-(4-fluorobenzyl)-1*H*-indazole-3-carbonyl)pyrrolidine-2-carboxamide,
1-(1-(4-fluorobenzyl)-1*H*-indazole-3-carbonyl)-*N*-(pyridin-3-ylmethyl)pyrolidine-2-carboxamide,
1-(1-(4-fluorobenzyl)-1*H*-indazole-3-carbonyl)-*N*-(2-(methylamino)-2-oxoethyl)pyrrolidine-2-carboxamide,
*N*-cyclohexyl-1-(1-(cyclohexylmethyl)-1*H*-indole-3-carbonyl)pyrrolidine-2-carboxamide,
1-(1-(cyclohexylmethyl)-1*H*-indole-3-carbonyl)-*N*-(2,3-dihydrobenzo[*b*][1,4]dioxin-6-yl)pyrrolidine-2-carboxamide,
methyl (1-(cyclohexylmethyl)-1*H*-indole-3-carbonyl)prolylglycinate,
*N*-(*tert*-butyl)-1-(1-(cyclohexylmethyl)-1*H*-indazole-3-carbonyl)pyrrolidine-2-carboxamide,
*N*-benzyl-1-(1-(cyclohexylmethyl)-1*H*-indazole-3-carbonyl)pyrrolidine-2-carboxamide,
*N*-(*tert*-butyl)-4-(1-(4-fluorobenzyl)-1*H*-indazole-3-carbonyl)morpholine-3-carboxamide,
*N*-cyclohexyl-4-(1-(4-fluorobenzyl)-1*H*-indazole-3-carbonyl)morpholine-3-carboxamide,
*N*-(*tert*-butyl)-4-(1-(cyclohexylmethyl)-1*H*-indazole-3-carbonyl)morpholine-3-carboxamide,
methyl 3-(tert-butylcarbamoyl)-4-(1-(4-fluorobenzyl)-1H-indazole-3-carbonyl)piperazine-1-carboxylate, and
methyl 3-(tert-butylcarbamoyl)-4-(1-(cyclohexylmethyl)-1H-indazole-3-carbonyl)piperazine-1-carboxylate,
or a stereoisomer or a pharmaceutically acceptable salt thereof.

In a preferred embodiment, the compound of formula (I) is selected from the group consisting of:
(S)-1-(1-pentyl-1H-indole-3-carbonyl)piperidine-2-carboxamide,
(R)-1-(1-pentyl-1H-indole-3-carbonyl)piperidine-2-carboxamide,
(S)-1-(1-(5-fluoropentyl)-1H-indole-3-carbonyl)piperidine-2-carboxamide,
(R)-1-(1-(5-fluoropentyl)-1H-indole-3-carbonyl)piperidine-2-carboxamide,
(S)-1-(1-(cyclohexylmethyl)-1H-indole-3-carbonyl)piperidine-2-carboxamide,
(R)-1-(1-(cyclohexylmethyl)-1H-indole-3-carbonyl)piperidine-2-carboxamide,
(S)-1-(1-(4-fluorobenzyl)-1H-indole-3-carbonyl)piperidine-2-carboxamide,
(R)-1-(1-(4-fluorobenzyl)-1H-indole-3-carbonyl)piperidine-2-carboxamide,
(S)-1-(1-pentyl-1H-indazole-3-carbonyl)piperidine-2-carboxamide,
(R)-1-(1-pentyl-1H-indazole-3-carbonyl)piperidine-2-carboxamide,
(S)-1-(1-(5-fluoropentyl)-1H-indazole-3-carbonyl)piperidine-2-carboxamide,
(R)-1-(1-(5-fluoropentyl)-1H-indazole-3-carbonyl)piperidine-2-carboxamide,
(S)-1-(1-(cyclohexylmethyl)-1H-indazole-3-carbonyl)piperidine-2-carboxamide,
(R)-1-(1-(cyclohexylmethyl)-1H-indazole-3-carbonyl)piperidine-2-carboxamide,
(S)-1-(1-(4-fluorobenzyl)-1H-indazole-3-carbonyl)piperidine-2-carboxamide,
(R)-1-(1-(4-fluorobenzyl)-1H-indazole-3-carbonyl)piperidine-2-carboxamide,
*(R)-N-*(*tert-*butyl)-1-(1-(4-fluorobenzyl)-1*H*-indole-3-carbonyl)piperidine-2-carboxamide,
*(S)*-*N*-(*tert*-butyl)-1-(1-(4-fluorobenzyl)-1*H*-indole-3-carbonyl)piperidine-2-carboxamide,
*(R)-N*-benzyl-1-(1-(4-fluorobenzyl)-1*H*-indole-3-carbonyl)piperidine-2-carboxamide,
*(S)*-*N*-benzyl-1-(1-(4-fluorobenzyl)-1*H*-indole-3-carbonyl)piperidine-2-carboxamide,
(R)-N-(2,3-dihydrobenzo[b][1,4]dioxin-6-yl)-1-(1-(4-fluorobenzyl)-1H-indole-3-carbonyl)piperidine-2-carboxamide,
*(S)*-*N*-(2,3-dihydrobenzo[*b*][1,4]dioxin-6-yl)-1-(1-(4-fluorobenzyl)-1*H*-indole-3-carbonyl)piperidine-2-carboxamide,
methyl (R)-(1-(1-(4-fluorobenzyl)-1*H*-indole-3-carbonyl)piperidine-2-carbonyl) glycinate,
methyl (S)-(1-(1-(4-fluorobenzyl)-1*H*-indole-3-carbonyl)piperidine-2-carbonyl) glycinate,
*(R)*-*N*-cyclohexyl-1-(1-(4-fluorobenzyl)-1*H*-indazole-3-carbonyl)piperidine-2-carboxamide,
*(S)*-*N*-cyclohexyl-1-(1-(4-fluorobenzyl)-1*H-*indazole-3-carbonyl)piperidine-2-carboxamide,
*(R)*-*N*-benzyl-1-(1-(4-fluorobenzyl)-1*H*-indazole-3-carbonyl)piperidine-2-carboxamide,
*(S)*-*N*-benzyl-1-(1-(4-fluorobenzyl)-1*H*-indazole-3-carbonyl)piperidine-2-carboxamide,
*(R)*-*N*-(2,3-dihydrobenzo[*b*][1,4]dioxin-6-yl)-1-(1-(4-fluorobenzyl)-1*H*-indazole-3-carbonyl)piperidine-2-carboxamide,
*(S)*-*N*-(2,3-dihydrobenzo[*b*][1,4]dioxin-6-yl)-1-(1-(4-fluorobenzyl)-1*H*-indazole-3-carbonyl)piperidine-2-carboxamide,
(R)-1-(1-(4-fluorobenzyl)-1H-indazole-3-carbonyl)-N-(2-(methylamino)-2-oxoethyl) piperidine-2-carboxamide,
(S)-1-(1-(4-fluorobenzyl)-1*H*-indazole-3-carbonyl)-*N*-(2-(methylamino)-2-oxoethyl) piperidine-2-carboxamide,
*(R)-N-*(*tert*-butyl)*-*1*-*(1*-*(cyclohexylmethyl)-1*H*-indole-3-carbonyl)piperidine-2-carboxamide,
*(S)-N-*(*tert*-butyl)-1-(1-(cyclohexylmethyl)-1*H-*indole-3-carbonyl)piperidine-2-carboxamide,
*(R)*-*N*-cyclohexyl-1-(1-(cyclohexylmethyl)-1*H*-indole-3-carbonyl)piperidine-2-carboxamide,
*(S)*-*N*-cyclohexyl-1-(1-(cyclohexylmethyl)-1*H*-indole-3-carbonyl)piperidine-2-carboxamide,
(R)-1-(1-(cyclohexylmethyl)-1*H*-indole-3-carbonyl)-*N*-(pyridin-3-ylmethyl)piperidine-2-carboxamide,
(S)-1-(1-(cyclohexylmethyl)-1*H*-indole-3-carbonyl)-*N*-(pyridin-3-ylmethyl)piperidine-2-carboxamide,
methyl (R)-(1-(1-(cyclohexylmethyl)-1*H*-indole-3-carbonyl)piperidine-2-carbonyl)glycinate,
methyl (S)-(1-(1-(cyclohexylmethyl)-1*H*-indole-3-carbonyl)piperidine-2-carbonyl)glycinate,
*(R)*-*N*-(*tert*-butyl)-1-(1-(cyclohexylmethyl)-1*H*-indazole-3-carbonyl)piperidine-2-carboxamide,
*(S)-N-*(*tert*-butyl)-1-(1-(cyclohexylmethyl)-1*H*-indazole-3-carbonyl)piperidine-2-carboxamide,
*(R)*-*N*-cyclohexyl-1-(1-(cyclohexylmethyl)-1*H*-indazole-3-carbonyl)piperidine-2-carboxamide,
*(S)*-*N*-cyclohexyl-1-(1-(cyclohexylmethyl)-1*H*-indazole-3-carbonyl)piperidine-2-carboxamide,
(R)-1-(1-(cyclohexylmethyl)-1*H*-indazole-3-carbonyl)-*N*-(pyridin-3-ylmethyl)pi-peridine-2-carboxamide,
(S)-1-(1-(cyclohexylmethyl)-1*H*-indazole-3-carbonyl)-*N*-(pyridin-3-ylmethyl)pi-peridine-2-carboxamide,
(R)-1-(1-(cyclohexylmethyl)-1*H*-indazole-3-carbonyl)-*N*-(2,3-dihydrobenzo[*b*] [1,4]dioxin-6-yl)piperidine-2-carboxamide,
(S)-1-(1-(cyclohexylmethyl)-1*H*-indazole-3-carbonyl)-*N*-(2,3-dihydrobenzo[*b*] [1,4]dioxin-6-yl)piperidine-2-carboxamide,
*(R)-N-(tert-butyl)-1-(1-(4-fluorobenzyl)-1H-indazole-3-carbonyl)pyrrolidine-2-*carboxamide,
*(S)-N-*(*tert*-butyl)-1-(1-(4-fluorobenzyl)-1*H*-indazole-3-carbonyl)pyrrolidine-2-carboxamide,
(*R*)-1-(1-(4-fluorobenzyl)-1*H*-indazole-3-carbonyl)-*N*-(pyridin-3-ylmethyl)pyrolidine-2-carboxamide,
(S)-1-(1-(4-fluorobenzyl)-1*H*-indazole-3-carbonyl)-*N*-(pyridin-3-ylmethyl)pyrolidine-2-carboxamide,
(R)-1-(1-(4-fluorobenzyl)-1*H*-indazole-3-carbonyl)-*N*-(2-(methylamino)-2-oxoethyl)pyrrolidine-2-carboxamide,
(S)-1-(1-(4-fluorobenzyl)-1*H*-indazole-3-carbonyl)-*N*-(2-(methylamino)-2-oxoethyl)pyrrolidine-2-carboxamide,
*(R)*-*N*-cyclohexyl-1-(1-(cyclohexylmethyl)-1*H*-indole-3-carbonyl)pyrrolidine-2-carboxamide,
*(S)*-*N*-cyclohexyl-1-(1-(cyclohexylmethyl)-1*H*-indole-3-carbonyl)pyrrolidine-2-carboxamide,
(R)-1-(1-(cyclohexylmethyl)-1H-indole-3-carbonyl)-N-(2,3-dihydrobenzo[b][1,4] dioxin-6-yl)pyrrolidine-2-carboxamide,
(S)-1-(1-(cyclohexylmethyl)-1H-indole-3-carbonyl)-N-(2,3-dihydrobenzo[b][1,4] dioxin-6-yl)pyrrolidine-2-carboxamide,
methyl (R)-(1-(cyclohexylmethyl)-1*H*-indole-3-carbonyl)prolylglycinate,
methyl (S)-(1-(cyclohexylmethyl)-1*H*-indole-3-carbonyl)prolylglycinate,
*(R)-N-*(*tert*-butyl)*-*1-(1-(cyclohexylmethyl)-1*H*-indazole-3-carbonyl)pyrrolidine-2-carboxamide,
*(S)-N-*(*tert*-butyl*)*-1-(1-(cyclohexylmethyl)-1*H*-indazole-3-carbonyl)pyrrolidine-2-carboxamide,
*(R)*-*N*-benzyl-1-(1-(cyclohexylmethyl)-1*H*-indazole-3-carbonyl)pyrrolidine-2-carboxamide,
*(S)*-*N*-benzyl-1-(1-(cyclohexylmethyl)-1*H*-indazole-3-carbonyl)pyrrolidine-2-carboxamide,
*(R)-N-*(*tert*-butyl)-4-(1-(4-fluorobenzyl)-1*H*-indazole-3-carbonyl)morpholine-3-carboxamide,
*(S)*-*N*-(*tert*-butyl)-4-(1-(4-fluorobenzyl)-1*H*-indazole-3-carbonyl)morpholine-3-carboxamide,
*(R)*-*N*-cyclohexyl-4-(1-(4-fluorobenzyl)-1*H*-indazole-3-carbonyl)morpholine-3-carboxamide,
*(S)*-N-cyclohexyl-4-(1-(4-fluorobenzyl)-1*H*-indazole-3-carbonyl)morpholine-3-carboxamide,
*(R)-N-(tert-butyl)-4-(1-(cyclohexylmethyl)-1H-indazole-3-carbonyl)morpholine-3-*carboxamide,
*(S)*-*N*-(*tert*-butyl)-4-(1-(cyclohexylmethyl)-1*H*-indazole-3-carbonyl)morpholine-3-carboxamide,
methyl (R)-3-(tert-butylcarbamoyl)-4-(1-(4-fluorobenzyl)-1H-indazole-3-carbonyl)piperazine-1-carboxylate,
methyl (S)-3-(tert-butylcarbamoyl)-4-(1-(4-fluorobenzyl)-1H-indazole-3-carbonyl)piperazine-1-carboxylate,
methyl (R)-3-(tert-butylcarbamoyl)-4-(1-(cyclohexylmethyl)-1H-indazole-3-carbonyl)piperazine-1-carboxylate, and
methyl (S)-3-(tert-butylcarbamoyl)-4-(1-(cyclohexylmethyl)-1H-indazole-3-carbonyl)piperazine-1-carboxylate,
or a pharmaceutically acceptable salt thereof.

In a more preferred embodiment, the compound of formula (I) is selected from the group consisting of:
(S)-1-(1-pentyl-1H-indole-3-carbonyl)piperidine-2-carboxamide,
(S)-1-(1-(5-fluoropentyl)-1H-indole-3-carbonyl)piperidine-2-carboxamide,
(S)-1-(1-(cyclohexylmethyl)-1H-indole-3-carbonyl)piperidine-2-carboxamide,
(S)-1-(1-(4-fluorobenzyl)-1H-indole-3-carbonyl)piperidine-2-carboxamide,
(S)-1-(1-pentyl-1H-indazole-3-carbonyl)piperidine-2-carboxamide,
(S)-1-(1-(5-fluoropentyl)-1H-indazole-3-carbonyl)piperidine-2-carboxamide,
(S)-1-(1-(cyclohexylmethyl)-1H-indazole-3-carbonyl)piperidine-2-carboxamide,
(S)-1-(1-(4-fluorobenzyl)-1H-indazole-3-carbonyl)piperidine-2-carboxamide,
*(S)*-*N*-(*tert*-butyl)-1-(1-(4-fluorobenzyl)-1*H*-indole-3-carbonyl)piperidine-2-carboxamide,
(S)-N-benzyl-1-(1-(4-fluorobenzyl)-1H-indole-3-carbonyl)piperidine-2-carboxamide,
*(S)*-*N*-(2,3-dihydrobenzo[*b*][1,4]dioxin-6-yl)-1-(1-(4-fluorobenzyl)-1*H*-indole-3-carbonyl)piperidine-2-carboxamide,
methyl (S)-(1-(1-(4-fluorobenzyl)-1H-indole-3-carbonyl)piperidine-2-carbonyl) glycinate,
*(S)*-*N*-cyclohexyl-1-(1-(4-fluorobenzyl)-1*H*-indazole-3-carbonyl)piperidine-2-carboxamide,
*(S)*-*N*-benzyl-1-(1-(4-fluorobenzyl)-1*H*-indazole-3-carbonyl)piperidine-2-carboxamide,
*(S)-N-*(2,3-dihydrobenzo[*b*][1,4]dioxin-6-yl)-1-(1-(4-fluorobenzyl)-1*H-*indazole-3-carbonyl)piperidine-2-carboxamide,
(S)-1-(1-(4-fluorobenzyl)-1*H*-indazole-3-carbonyl)-*N-*(2-(methylamino)-2-oxoethyl) piperidine-2-carboxamide,
*(S)-N-*(*tert*-butyl)*-*1-(1-(cyclohexylmethyl)-1*H-*indole-3-carbonyl)piperidine-2-carboxamide,
*(S)*-*N*-cyclohexyl-1-(1-(cyclohexylmethyl)-1*H*-indole-3-carbonyl)piperidine-2-carboxamide,
(S)-1-(1-(cyclohexylmethyl)-1*H*-indole-3-carbonyl)-*N*-(pyridin-3-ylmethyl)piperidine-2-carboxamide,
methyl (S)-(1-(1-(cyclohexylmethyl)-1*H-*indole-3-carbonyl)piperidine-2-carbonyl)glycinate,
*(S)-N-*(*tert*-butyl)*-*1-(1-(cyclohexylmethyl)-1*H-i*ndazole-3-carbonyl)piperidine-2-carboxamide,
*(S)*-*N*-cyclohexyl-1-(1-(cyclohexylmethyl)-1*H*-indazole-3-carbonyl)piperidine-2-carboxamide,
(S)-1-(1-(cyclohexylmethyl)-1*H*-indazole-3-carbonyl)-*N*-(pyridin-3-ylmethyl)piperidine-2-carboxamide,
(S)-1-(1-(cyclohexylmethyl)-1*H*-indazole-3-carbonyl)-*N*-(2,3-dihydrobenzo[*b*] [1,4]dioxin-6-yl)piperidine-2-carboxamide,
*(S)*-*N*-(*tert*-butyl)-1-(1-(4-fluorobenzyl)-1*H*-indazole-3-carbonyl)pyrrolidine-2-carboxamide,
(S)-1-(1-(4-fluorobenzyl)-1*H*-indazole-3-carbonyl)-*N*-(pyridin-3-ylmethyl)pyrolidine-2-carboxamide,
(S)-1-(1-(4-fluorobenzyl)-1*H*-indazole-3-carbonyl)-*N*-(2-(methylamino)-2-oxoethyl)pyrrolidine-2-carboxamide,
*(S)*-*N*-cyclohexyl-1-(1-(cyclohexylmethyl)-1*H*-indole-3-carbonyl)pyrrolidine-2-carboxamide,
(S)-1-(1-(cyclohexylmethyl)-1*H*-indole-3-carbonyl)-*N*-(2,3-dihydrobenzo[*b*][1,4] dioxin-6-yl)pyrrolidine-2-carboxamide,
methyl (S)-(1-(cyclohexylmethyl)-1*H*-indole-3-carbonyl)prolylglycinate,
*(S)-N-*(*tert*-butyl)*-*1-(1-(cyclohexylmethyl)-1*H*-indazole-3-carbonyl)pyrrolidine-2-carboxamide,
*(S)*-*N*-benzyl-1-(1-(cyclohexylmethyl)-1*H*-indazole-3-carbonyl)pyrrolidine-2-carboxamide,
(*S*)-*N*-butyl)-4-(1-(4-fluorobenzyl)-1H-indazole-3-carbonyl)morpholine-3-carboxamide,
*(S)*-*N*-cyclohexyl-4-(1-(4-fluorobenzyl)-1*H*-indazole-3-carbonyl)morpholine-3-carboxamide,
*(S)*-*N*-(tert-butyl)-4-(1-(cyclohexylmethyl)-1H-indazole-3-carbonyl)morpholine-3-carboxamide,
methyl (S)-3-(tert-butylcarbamoyl)-4-(1-(4-fluorobenzyl)-1H-indazole-3-carbonyl)piperazine-1-carboxylate, and
methyl (S)-3-(tert-butylcarbamoyl)-4-(1-(cyclohexylmethyl)-1H-indazole-3-carbonyl)piperazine-1-carboxylate,
or a pharmaceutically acceptable salt thereof.

The R and S configuration refers to the stereochemistry of the chiral carbon atom marked with an asterisk * in formula (I) below.

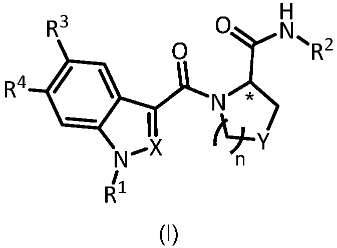

### Pharmaceutical compositions

**The** second aspect of the invention relates to a pharmaceutical composition comprising a compound of formula (I), or a stereoisomer or a pharmaceutically acceptable salt thereof, as defined in the first aspect, and a pharmaceutically acceptable excipient.

As used herein, the term "pharmaceutically acceptable excipient" refers to conventional pharmaceutically acceptable carrier materials suitable for drug administration, and include any such materials known in the art that are nontoxic and do not interact with other components of a pharmaceutical composition or drug delivery system in a deleterious manner.

**The** pharmaceutical compositions may be in oral form, either solid or liquid. Suitable dose forms for oral administration may be tablets, capsules, syrups or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the apropriate unit dosage form. Adequate excipients can be used, such as bulking agents, buffering agents or surfactants.

The pharmaceutical compositions may comprise from 0.1 to 90 wt% of the compound of formula (I), with respect to the total weight of the composition, preferably from 0.1 to 50 wt%, more preferably from 0.1 to 10 wt%. The pharmaceutical composition may comprise from 10% to 99.9 wt% of pharmaceutically acceptable excipients, with respect to the total weight of the composition, preferably from 50 to 99.9 wt%, more preferably from 90 to 99.9 wt%.

The pharmaceutical composition of the invention may be obtained by mixing the active ingredient(s), i.e. the compound of formula (I), with the suitable pharmaceutically acceptable excipients.

### Synthesis of the compound of formula (1)

The synthetic procedure to be used for the preparation of the compounds of formula (I) depends on the nature of substituent R². One of the synthetic pathways to be used in the present invention to obtain compounds of formula (I) is the Ugi-Joullié reaction, which is suitable for all compounds of formula (I) except those where R² is H.

Thus, the third aspect of the invention relates to a process for the preparation of a compound of formula (I) according to the first aspect, wherein R² is selected from the group consisting of C₁-C₆ alkyl, -(CH₂)ₚ-C₃-C₈ cycloalkyl, -(CH₂)ₚ-C₆-C₁₂ aryl, -(CH₂)ₚ-CH₂-heteroaryl wherein said heteroaryl is a 6- to 12-memebered monocyclic or bicyclic ring system containing one, two or three heteroatoms selected from the N and O, -(CH₂)ₚ- heterocyclyl wherein said heterocyclyl is 3- to 8-membered monocyclic ring containing one or two heteroatoms selected from N and O, -CH₂C(=O)OR^{a}, and -CH₂-C(=O)NR^{a}; wherein p is selected from 0, 1 and 2; and R^{a} is H or C₁-C₆ alkyl, and
wherein the process comprises the Ugi-Joullié reaction of a compound of formula (II) with a compound of formula (V) and a compound of formula (VI) to give the compound of formula (I), 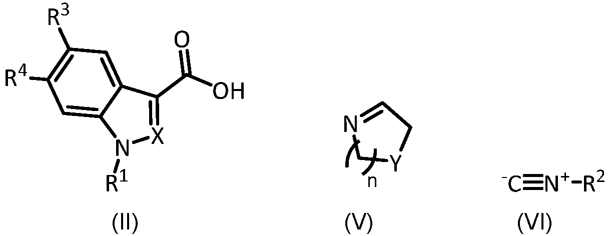 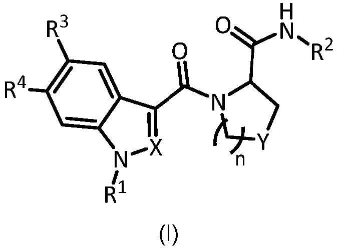
wherein R¹, R³, R⁴, X, Y and n are as defined for the compounds of formula (I) in the first aspect, and R² is as defined above.

Compounds of formula (II) may be prepared by processes known in the art, such as those described by Gioé-Gallo et al. [Biomedicine & Pharmacotherapy, 2023, 164, 114934]. In particular, compounds of formula (II) may be obtained by alkylation of methyl 3-indolecarboxylate (when X is CH₂) or 3-indazolecarboxylate (when X is N), compound of formula (VII), with alkyl bromides R¹-Br under basic conditions to yield compounds of formula (VIII), wherein R¹ is as defined above for the compounds of formula (I). In particular, this alkylation is carried out using NaH as the base. Preferably, the solvent used is dimethylformamide (DMF). Preferably, the alkylation is carried out at a temperature of 20 to 25 °C. Preferably, the reaction is carried out for 20 to 30 hours. Compounds of formula (VII) may be subjected to a saponification reaction, preferably using NaOH as the base and dioxane as the solvent, to yield compound (II). This saponification is preferably carried out at 100 to 130 °C. Preferably, the saponification reaction is carried out for 10 to 15 hours.

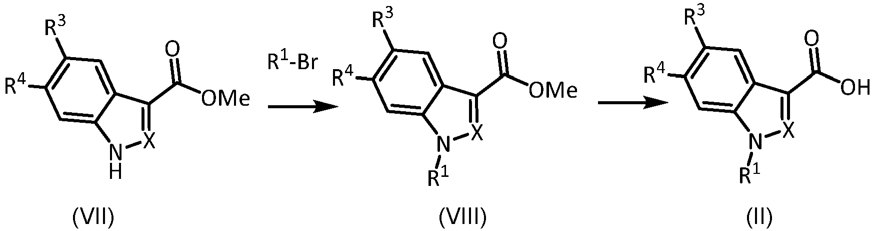

Compounds of formula (VI) are commercially available and compounds of formula (V) may be prepared by processes known in the art, such as those described in Khan and Saxena [Adv. Synth. Catal., 2013, 355, 2617-2626] and Kauffmann et al. [Angew. Chem. Int. Ed. Engl., 1967, 6(7), 633-634]. Compounds of formula (V) wherein Y is not NH may also be prepared starting from the corresponding heterocyclic compound (IX) by reaction with N-chlorosuccinimide in diethyl ether at room temperature, followed by reaction with MeONa in methanol, as described in the examples.

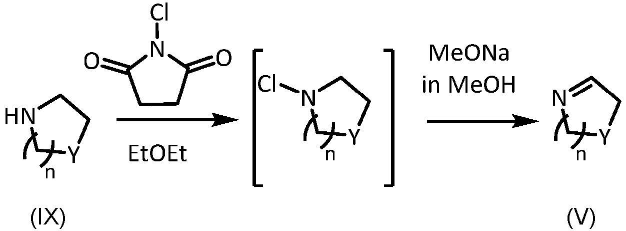

The Ugi-Joullié reaction is generally carried out in a suitable solvent, such as polar aprotic solvents (in particular dimethylformamide and tetrahydrofuran), C₁-C₄ alkanols optionally fluorinated, in particular methanol, ethanol, isopropanol, trifluoroethanol, and mixtures thereof, in particular methanol, ethanol, isopropanol, trifluoroethanol, a mixture of dimethylformamide (DMF) and methanol, or a mixture of tetrahydrofuran (THF) and methanol. In one embodiment, the Ugi-Jouillé reaction to prepare the compounds of formula (I) is carried out in a solvent selected from the group consisting of DMF, methanol, ethanol and mixtures thereof; preferably in DMF, methanol and mixtures thereof.

The term "polar aprotic solvent" refers to a solvent which has a dielectric constant greater than 5 and whcih does not have any acidic proton.

The term "alkanol" refers to an alkyl group, as previously defined, having one or more hydroxyl groups, preferably one hydroxyl group.

Preferably, the Ugi-Jouillé reaction to prepare the compounds of formula (I) is carried out at a temperature of from 10 to 35 °C, preferably from 20 to 25 °C.

The other synthetic pathways to be used in the present invention is to obtain compounds of formula (I) wherein R² is H.

Thus, in a fourth aspect, the present invention relates to a process for the preparation of a compound of formula (I) according to the first aspect, wherein R² is H and n is 2, and wherein the process comprises:
a) amidation of a compound of formula (II) with a compound of formula (III) to give a compound of formula (IV) 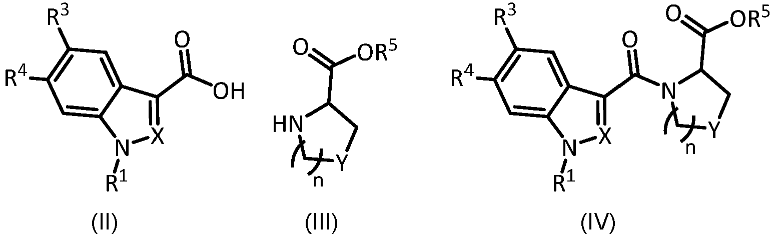 wherein R¹, R³, R⁴, X and Y are as defined for the compounds of formula (I) in the first aspect, n is 2 and R⁵ is C₁-C₃ alkyl;
b) ammonolysis of the compound of formula (IV) obtained in step a) to a compound of formula (I) 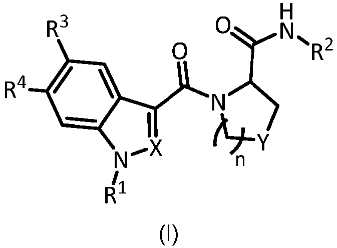 wherein R¹, R³, R⁴, X and Y are as defined for the compounds of formula (I) in the first aspect, n is 2 and R² is H.

The first step is the amidation of the compound of formula (II) with the compound of formula (III) to give the compound of formula (IV).

Compounds of formula (II) may be prepared by processes known in the art, such as those described by Gioé-Gallo et al. [Biomedicine & Pharmacotherapy, 2023, 164, 114934], as explained above.

Compounds of formula (III) are commercially available.

Preferably, in this amidation reaction of step a), R⁵ is methyl in the compounds of formula (III). Accordingly, in the compounds of formula (IV), R⁵ is also preferably methyl.

The amidation of step a) is preferably carried out in the presence of an amide coupling agent.

The term "amide coupling agent" refers to compounds which are promote the reaction of the carboxylic acid group of compound (II) and the amine group of compound (III) for the formation of an amide bond (compound (IV)).

Examples of amide coupling agents are O-(7-azabenzotriazol-1-yl)-N,N,N',N'-tetramethyluronium hexafluorophosphate (HATU), O-(7-azabenzotriazol-1-yl)-N,N,N',N'-tetramethyluronium tetrafluoroborate (TATU), O-(benzotriazol-1-yl)-N,N,N',N'-tetramethyluronium hexafluorophosphate (HBTU), O-(benzotriazol-1-yl)- N,N,N',N'-tetramethyluronium tetrafluoroborate (TBTU), or O-(6-chlorobenzotriazol-1-yl)-N,N,N',N'-tetramethyluronium hexafluorophosphate (HCTU). Preferably, the amidation of step a) is carried out in the presence of HATU.

The amidation of step a) is preferably carried out in the presence of a tertiary amine base.

The term "tertiary amine base" refers to a base which consists of a nitrogen atom bonded to three substituents, preferably three alkyl substituents, wherein alkyl is as previously defined. Said alkyl substituents may be the same or different from each other.

Examples of tertiary amine bases suitable for the amidation reaction of step a) are diisopropylethylamine (DIPEA) and/or, triethylamine. Preferably, the amidation of step a) is carried out in the presence of DIPEA.

Preferably, the amidation of step a) is carried out in the presence of an aprotic organic solvent.

The term "aprotic organic solvent" refers to an organic solvent which does not have any acidic proton.

Examples of aprotic organic solvents suitable for the amidation reaction of step a) are dichloromethane (DCM), acetonitrile, dimethylformamide, dimethylacetamide, dimethylsulfoxide, and N-methylpyrrolidone. Preferably, the amidation of step a) is carried out in the presence of DCM as the aprotic organic solvent.

More preferably, the amidation of step a) is carried out in the presence of HATU, as the amide coupling agent, DIPEA, as the tertiary amine base, and DCM as the aprotic organic solvent.

Preferably, the amidation reaction of step a) is carried out at a temperature of from 10 to 35 °C, preferably from 20 to 25 °C.

Preferably, the amidation reaction of step a) is carried out for 20 to 30 hours.

Once the amide of formula (I) has been obtained, step b) is carried out.

Step b) is the ammonolysis of the compound of formula (IV) obtained in step a) to a compound of formula (I) 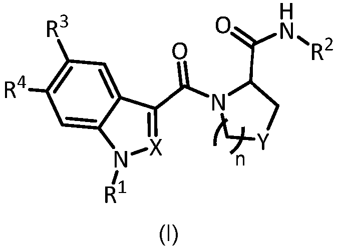 wherein R¹, R³, R⁴, X and Y are as defined for the compounds of formula (I) in the first aspect, n is 2 and R² is H.

The ammonolysis is preferably carried out with NH₃ in methanol.

Preferably, the ammonolysis of step b) is carried out at a temperature of from 10 to 35 °C, preferably from 20 to 25 °C.

Preferably, the ammonolysis of step b) is carried out for 3 to 6 hours.

### Intermediate compound of formula (IV)

In the synthesis of compounds of formula (I) R² is H a new intermediate ester compound has been obtained, which is the compound of formula (IV).

Thus, in a fifth aspect, the invention relates to a compound of formula (IV) 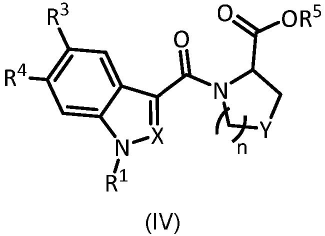 wherein R¹, R³, R⁴, X and Y are as defined above for the compounds of formula (I) of the first aspect, n is 2 and R⁵ is C₁-C₃ alkyl.

In a preferred embodiment, in the compound of formula (IV) R⁵ is methyl.

In a particular embodiment, the compound of the fifth aspect has formula (IV'): 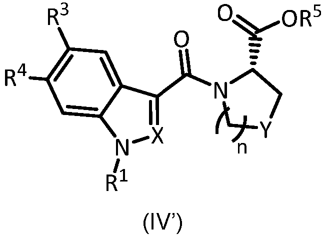 wherein R¹, R³, R⁴, R⁵, **X, Y,** n are as defined above, or a pharmaceutically acceptable salt thereof.

In a particular embodiment, the compound of formula (IV) is selected from the group consisting of:
methyl 1-(1-pentyl-1H-indole-3-carbonyl)piperidine-2-carboxylate,
methyl 1-(1-(5-fluoropentyl)-1H-indole-3-carbonyl)piperidine-2-carboxylate,
methyl 1-(1-(cyclohexylmethyl)-1H-indole-3-carbonyl)piperidine-2-carboxylate,
methyl 1-(1-(4-fluorobenzyl)-1H-indole-3-carbonyl)piperidine-2-carboxylate,
methyl 1-(1-pentyl-1H-indazole-3-carbonyl)piperidine-2-carboxylate,
methyl 1-(1-(5-fluoropentyl)-1H-indazole-3-carbonyl)piperidine-2-carboxylate,
methyl 1-(1-(cyclohexylmethyl)-1H-indazole-3-carbonyl)piperidine-2-carboxylate, and
methyl 1-(1-(4-fluorobenzyl)-1H-indazole-3-carbonyl)piperidine-2-carboxylate,
or a stereoisomer or a pharmaceutically acceptable salt thereof.

In another particular embodiment, the compound of formula (IV) is selected from the group consisting of:
methyl (S)-1-(1-pentyl-1H-indole-3-carbonyl)piperidine-2-carboxylate,
methyl (R)-1-(1-pentyl-1H-indole-3-carbonyl)piperidine-2-carboxylate,
methyl (S)-1-(1-(5-fluoropentyl)-1H-indole-3-carbonyl)piperidine-2-carboxylate,
methyl (R)-1-(1-(5-fluoropentyl)-1H-indole-3-carbonyl)piperidine-2-carboxylate,
methyl (S)-1-(1-(cyclohexylmethyl)-1H-indole-3-carbonyl)piperidine-2-carboxylate,
methyl (R)-1-(1-(cyclohexylmethyl)-1H-indole-3-carbonyl)piperidine-2-carboxylate,
methyl (S)-1-(1-(4-fluorobenzyl)-1H-indole-3-carbonyl)piperidine-2-carboxylate,
methyl (R)-1-(1-(4-fluorobenzyl)-1H-indole-3-carbonyl)piperidine-2-carboxylate,
methyl (S)-1-(1-pentyl-1H-indazole-3-carbonyl)piperidine-2-carboxylate,
methyl (R)-1-(1-pentyl-1H-indazole-3-carbonyl)piperidine-2-carboxylate,
methyl (S)-1-(1-(5-fluoropentyl)-1H-indazole-3-carbonyl)piperidine-2-carboxylate,
methyl (R)-1-(1-(5-fluoropentyl)-1H-indazole-3-carbonyl)piperidine-2-carboxylate,
methyl (S)-1-(1-(cyclohexylmethyl)-1H-indazole-3-carbonyl)piperidine-2-carboxylate,
methyl (R)-1-(1-(cyclohexylmethyl)-1H-indazole-3-carbonyl)piperidine-2-carboxylate,
methyl (S)-1-(1-(4-fluorobenzyl)-1H-indazole-3-carbonyl)piperidine-2-carboxylate, and
methyl (R)-1-(1-(4-fluorobenzyl)-1H-indazole-3-carbonyl)piperidine-2-carboxylate,
or a pharmaceutically acceptable salt thereof.

In another particular embodiment, the compound of formula (IV) is selected from the group consisting of:
methyl (S)-1-(1-pentyl-1H-indole-3-carbonyl)piperidine-2-carboxylate,
methyl (S)-1-(1-(5-fluoropentyl)-1H-indole-3-carbonyl)piperidine-2-carboxylate,
methyl (S)-1-(1-(cyclohexylmethyl)-1H-indole-3-carbonyl)piperidine-2-carboxylate,
methyl (S)-1-(1-(4-fluorobenzyl)-1H-indole-3-carbonyl)piperidine-2-carboxylate,
methyl (S)-1-(1-pentyl-1H-indazole-3-carbonyl)piperidine-2-carboxylate,
methyl (S)-1-(1-(5-fluoropentyl)-1H-indazole-3-carbonyl)piperidine-2-carboxylate,
methyl (S)-1-(1-(cyclohexylmethyl)-1H-indazole-3-carbonyl)piperidine-2-carboxylate, and
methyl (S)-1-(1-(4-fluorobenzyl)-1H-indazole-3-carbonyl)piperidine-2-carboxylate,
or a pharmaceutically acceptable salt thereof

### Medicinal use of the compounds of formula (I)

As shown in the examples of the present application, the compounds of formula (I) are selective agonists of the CB₂ receptor.

Thus, in one aspect, the invention relates to a compound of formula (I), or a stereoisomer or a pharmaceutically acceptable salt thereof, as defined in the first aspect, for use in the treatment and/or prevention of diseases characterized by moderate to chronic inflammation, wherein therapeutic efficacy is mediated through CB₂ receptor agonism.

This aspect may also be formulated as the use of a compound of formula (I), or a stereoisomer or a pharmaceutically acceptable salt thereof, as defined in the first aspect, in the manufacture of a medicament for the treatment and/or prevention of diseases characterized by moderate to chronic inflammation, wherein therapeutic efficacy is mediated through CB₂ receptor agonism.

This aspect may also be formulated as a method of treatment and/or prevention of diseases characterized by moderate to chronic inflammation, wherein therapeutic efficacy is mediated through CB₂ receptor agonism, wherein said method comprises administering to a subject in need thereof a compound of formula (I), or a stereoisomer or a pharmaceutically acceptable salt thereof.

As explained in the background section of the present document, diseases characterized by moderate to chronic inflammation, wherein therapeutic efficacy is mediated through CB₂ receptor agonism are, for example, pathologies neuroinflammatory and neurodegenerative diseases, such as Alzheimer's disease, Parkinson's disease, multiple sclerosis, amyotrophic lateral sclerosis (ALS), Huntington's disease, and neuropathic pain; peripheral inflammatory diseases, such as rheumatoid arthritis, inflammatory bowel disease (such as Crohn's disease and ulcerative colitis), systemic lupus erythematosus, and psoriasis; cancer-related conditions associated with chronic inflammation and immune dysregulation, such as glioblastoma, pancreatic cancer, breast cancer, and colorectal cancer; chronic inflammatory diseases, such as diabetic retinopathy, chronic obstructive pulmonary disease (COPD), and atherosclerosis.

In a further aspect, the invention relates to a compound of formula (I), or a stereoisomer or a pharmaceutically acceptable salt thereof, as defined in the first aspect, for use in medicine.

This aspect may also be formulated as the use of a compound of formula (I), or a stereoisomer or a pharmaceutically acceptable salt thereof, as defined in the first aspect, in the manufacture of a medicament.

In a further aspect, the invention relates to a compound of formula (I), or a stereoisomer or a pharmaceutically acceptable salt thereof, as defined in the first aspect, for use in the treatment and/or prevention of a disease selected from the group consisting of pathologies neuroinflammatory and neurodegenerative diseases, such as Alzheimer's disease, Parkinson's disease, multiple sclerosis, amyotrophic lateral sclerosis (ALS), Huntington's disease, and neuropathic pain; peripheral inflammatory diseases, such as rheumatoid arthritis, inflammatory bowel disease (such as Crohn's disease and ulcerative colitis), systemic lupus erythematosus, and psoriasis; cancer-related conditions associated with chronic inflammation and immune dysregulation, such as glioblastoma, pancreatic cancer, breast cancer, and colorectal cancer; chronic inflammatory diseases, such as diabetic retinopathy, chronic obstructive pulmonary disease (COPD), and atherosclerosis.

This aspect may also be formulates as the use of a compound of formula (I), or a stereoisomer or a pharmaceutically acceptable salt thereof, as defined in the first aspect, in the manufacture of a medicament for the treatment and/or prevention of a disease selected from the group consisting of pathologies neuroinflammatory and neurodegenerative diseases, such as Alzheimer's disease, Parkinson's disease, multiple sclerosis, amyotrophic lateral sclerosis (ALS), Huntington's disease, and neuropathic pain; peripheral inflammatory diseases, such as rheumatoid arthritis, inflammatory bowel disease (such as Crohn's disease and ulcerative colitis), systemic lupus erythematosus, and psoriasis; cancer-related conditions associated with chronic inflammation and immune dysregulation, such as glioblastoma, pancreatic cancer, breast cancer, and colorectal cancer; chronic inflammatory diseases, such as diabetic retinopathy, chronic obstructive pulmonary disease (COPD), and atherosclerosis.

This aspect may also be formulated as a method of treatment and/or prevention of a disease selected from the group consisting of pathologies neuroinflammatory and neurodegenerative diseases, such as Alzheimer's disease, Parkinson's disease, multiple sclerosis, amyotrophic lateral sclerosis (ALS), Huntington's disease, and neuropathic pain; peripheral inflammatory diseases, such as rheumatoid arthritis, inflammatory bowel disease (such as Crohn's disease and ulcerative colitis), systemic lupus erythematosus, and psoriasis; cancer-related conditions associated with chronic inflammation and immune dysregulation, such as glioblastoma, pancreatic cancer, breast cancer, and colorectal cancer; chronic inflammatory diseases, such as diabetic retinopathy, chronic obstructive pulmonary disease (COPD), and atherosclerosis; wherein the method comprises administering to a subject in need thereof a compound of formula (I), or a stereoisomer or a pharmaceutically acceptable salt thereof, as defined in the first aspect.

The terms "treat", "treatment", or "treatment of" as used herein refer to reducing the potential for a certain disease or disorder, reducing the occurrence of a certain disease or disorder, and/or a reduction in the severity of a certain disease or disorder, preferably, to an extent that the subject no longer suffers discomfort and/or altered function due to it. It also refers to mitigating or decreasing at least one clinical symptom and/or inhibition or delay in the progression of the condition and/or prevention or delay of the onset of a disease or illness.

The terms "prevention", "preventing" or "prevent" as used herein refer to avoiding the appearance of a certain disease or disorder. The prevention can be complete (e.g., the total absence of a disease). The prevention can also be partial, such that for example the occurrence of a disease in a subject is less than that which would have occurred without the administration of the compounds of the present invention. Prevention also refers to reduced susceptibility to a clinical condition. The prevention also includes reducing the risk of suffering the disease.

The subject to which the compounds and pharmaceutical compositions described herein are administered is a human or animal; preferably subjects are mammals, more preferably humans.

The compound of formula (I), or a stereoisomer or a pharmaceutically acceptable salt thereof, as defined in the first aspect, is typically administered in a therapeutically effective amount. The term "therapeutically effective amount" as used herein means a nontoxic but sufficient amount to provide the desired effect. The therapeutically effective amount will vary from subject to subject, depending on the age and general condition of the individual, the particular active agent, and the like. However, an appropriate "effective" amount in any individual case may be determined by one of ordinary skill in the art using routine experimentation.

The following examples represent specific embodiments of the present invention. They do not intend to limit in any way the scope of the invention defined in the present description.

### Examples

### Materials and methods

### Chemistry

All starting materials, reagents and solvents were purchased (Sigma Aldrich) and used without further purification. After extraction from aqueous phases, the organic solvents were dried over anhydrous magnesium sulphate. The reactions were monitored by thin-layer chromatography (TLC) on 2.5 mm Merck silica gel GF 254 strips, and the purified compounds each showed a single spot. Unless stated otherwise, UV light, phosphomolybdic acid or iodine vapor were used to detect compounds. The amide coupling reactions were performed in coated Kimble vials on a PLS (6×4) Organic Synthesizer with orbital stirring. The purity and identity of all tested compounds were established by a combination of high-performance liquid chromatography (HPLC), mass spectrometry and nuclear magnetic resonance (NMR) spectroscopy. Purification of isolated products was carried out by column chromatography (Kieselgel 0.040-0.063 mm, E. Merck) or preparative chromatography (UniplateTM Techware 20x20 cm Silica G 1000mm Thick 15µm).

The NMR spectra were recorded on Bruker AM300 and XM500 spectrometers. Chemical shifts are given as δ values against tetramethylsilane as internal standard and J values are given in Hz. The signal multiplicity shown with the abbreviatures s, singlet; d, doublet; t, triplet; dd, doublet of doublets; dt, doublet of triplets; td, triplet of doublets; and m, multiplet, followed by number of protons (deduced by integration). The coupling constants (J) are deduced in Hertz (Hz).

Mass spectra were obtained on a Varian MAT-711 instrument. High-resolution mass spectra were obtained on an Autospec Micromass spectrometer. Analytical HPLC and Low-resolution mass spectra were performed on a Waters^{™} Acquity^{™} Arc^{™} UHPLC System using a XBridge^{®} C18, 4.6 mm × 50 mm, 3.5 µm column with gradient elution using the mobile phases (A) H2O containing 0.1% HCOOH and (B) MeCN containing 0.1% HCOOH and a flow rate of 1 mL/min. The purity of all tested compounds was determined to be >95%.

The chiral analysis was performed using an Agilent 1100 Series HPLC Value System (G1322A Degasser; G1311A Quaternary Pump; G1314A VWD Detector; G1313A ALS Autosampler). Enantiomers were evaluated with 250 mm x 20 mm Chiralpak^{®} 5µm IE-3 (DAICEL) using an isocratic mobile phase n-hexane/i-propanol 80:20.

The purity of all tested compounds was determined to be >95% using analytical HPLC [Water Breeze^{™} 2 system (binary pump 1525, detector UV/Visible 2489, 7725i Manual Injector Kit 1500 Series)] using a Luna 5 µm Silica (2) 100 Å, LC Column 150 x 4.6 mm column with gradient elution using the mobile phases dichloromethane, isopropanol, and a flow rate of 1 mL/min. The stereochemical purity of described compounds was analyzed and confirmed (e.e. >97%) by chiral HPLC.

### General procedure for the synthesis of esters of formula (IV) wherein R⁵ is methyl:

A mixture of the 1-substituted indole (or indazole)-3-carboxylic acid (1 mmol) (compound of formula (II), the corresponding cyclic amino ester (compound of formula (III) wherein R³ is methyl) (1.5 mmol), HATU (1.5 mmol) and DIPEA (4 mmol) in CH₂Cl₂ (2 mL) was stirred with orbital stirring at room temperature for 24h. After completion of the reaction, water was added, and the mixture was extracted with CH₂Cl₂. The organic phase was dried over MgSO₄, filtered, and concentrated. The resulting product was purified by column chromatography on silica gel. The purity of all prepared compounds of formula (IV) wherein R⁵ is methyl was established by high-performance liquid chromatography (HPLC) and showed to be >95%.

### General procedures for the synthesis of amides of formula (I) wherein R² is H:

A mixture of the corresponding ester of formula (IV) wherein R⁵ is methyl, and a solution 4M of ammonia (4 mmol) in MeOH (2 mL) was stirred at room temperature for 24h. After completion of the reaction, solvent was evaporated. The resulting product was purified by column chromatography on silica gel using MeOH/CH₂Cl₂. The purity of all prepared compounds of formula (I) wherein R² is H was established by high-performance liquid chromatography (HPLC) and showed to be >95%.

### General procedure for the synthesis of heterocyclic imines of formula (V):

Piperidine, morpholine or pyrrolidine (1.0 eq.) was slowly added to a cold (0 °C) solution of N-chlorosuccinimide (1.1 eq.) in diethyl ether (50 mL). After removing the ice bath, the reaction was stirred at room temperature for 3.5 h. The mixture was filtered and the solid was washed with diethyl ether (x 3). The combined organic layers were washed with water (x 2), dried over Na₂SO₄ and concentrated under reduced pressure to 1/3 of the volume. MeONa solution in MeOH (1.4 eq.) was added slowly using a water bath to control the reaction temperature. After refluxing the mixture at 40 °C for 1 h, water was added until all precipitated sodium chloride was dissolved. The mixture was extracted with diethyl ether (x 3) and the combined layers were dried over Na₂SO₄. Removing the solvent under reduced pressure afforded the compounds of formula (V) as thick light-yellow oil.

Other heterocyclic imines were obtained following published procedures [Khan and Saxena, Adv. Synth. Catal., 2013, 355, 2617-2626; and Kauffmann et al., Angew. Chem. Int. Ed. Engl., 1967, 6(7), 633-634]

### General procedures for the synthesis of amides of formula (I) wherein R² is not H by the Ugi-Jouillé reaction:

A mixture of the 1-substituted indole (or indazole)-3-carboxylic acid of formula (II) (1.0 eq.), the heterocyclic imine of formula (V) (1.0 eq.), and the corresponding isocyanide of formula (VI) (1.0 eq.) in MeOH (1 mL) was stirred with orbital stirring at room temperature for 24h. The solvent was removed in vacuo and the obtained residue was purified by column chromatography on silica gel to yield the expected product. The purity of all prepared compounds was established by high-performance liquid chromatography (HPLC) and showed to be >95%.

### Preparative and analytical chiral HPLC conditions

Preparative and analytical HPLC analyses were performed using a Waters Alliance e2695 system (Waters, USA), equipped with an e2695 separation module, a photodiode array (PDA) detector model 2998, and a thermostatted column oven. System control and data acquisition were conducted using Empower 3 software. Chromatographic separations were achieved using a Chiralpak IE column (20 mm i.d. × 250 mm length, 5 µm particle size; DAICEL Corp.), maintained at 25 °C. The mobile phase was a binary solvent system: (A) dichloromethane (DCM, HPLC grade) and (B) isopropanol (IPA, HPLC grade). Isocratic elution was employed with three different compositions: 2%, 4%, and 10% IPA in DCM, at constant flow rates of 3 or 6 mL/min. The injection volume was 200 µL. Detection was performed using the PDA detector, with spectra recorded over the 200-600 nm range, and quantification carried out at 254 nm, corresponding to the analyte's maximum absorbance. All sample and standard solutions were filtered through 0.45 µm PTFE membranes and degassed by ultrasonication prior to injection.

Preparative separations were carried out for at least 45 minutes per run. Under these conditions, enantiomers were typically resolved with retention time differences of 3-5 minutes. The enantiomeric excess (ee) of the isolated compounds was routinely assessed using the same HPLC method and confirmed to be >98% for each enantiomer.

### Circular Dichroism (CD)

CD spectra of ligands (>98%) were recorded on a Jasco-815 system equipped with a Peltier-type thermostatic accessory (CDF-426S, Jasco). Measurements were carried out at 20 °C using a 1-mm quartz cell in a volume of 300-350 µL. Compounds (0.1 mg) were dissolved in MeOH (1.0 mL). The instrument settings were bandwidth, 1.0 nm; data pitch, 1.0 nm; speed, 500 nm/min; accumulation, 10; wavelengths, 400-190 nm.

### Binding assays

Radioligand binding competition assays of CB₁ receptors were carried out in polypropylene 96-well plates by incubating 5 µg of membranes from Chinese hamster ovary (CHO)-CB₁ C3 cell line (PerkinElmer) with 1 nM [³H]-CP55940 (Perkin Elmer) and test compounds in binding buffer (50 mM Tris-HCl, 5 mM MgCl₂, 1mM EDTA, 0.5% BSA. pH: 7.4). Non-specific binding was determined in the presence of 10 µM Surinabant. The reaction mixture was incubated at 30°C for 60 min, then 200 µL were transferred to GF/C 96-well plate (Millipore, Madrid, Spain) and washed four times with 250 µL wash buffer (50 mM Tris-HCl, 5 mM MgCl₂, 1 mM EDTA, 0.5% BSA. pH: 7.4). Radioactivity was detected in a microplate beta scintillation counter (Microbeta Trilux, PerkinElmer, Madrid, Spain). Radioligand binding competition assays of CB₂ receptors were carried out in polypropylene 96-well plates by incubating 5 µg of membranes from Human embryonic kidney 293 cells (HEK)-CB₂ cell line with 0.2 nM [³H]-CP55940 (Perkin Elmer) and test compounds in binding buffer (50 mM Tris-HCl, 5 mM MgCl₂, 2.5 mM EGTA, 0.1% BSA. pH: 7,4). Non-specific binding was determined in the presence of 10 µM GW405833. The reaction mixture was incubated at 30°C for 90 min, then 200 µL were transferred to GF/C 96-well plate (Millipore, Madrid, Spain) and washed four times with 250 µL wash buffer (50mM Tris-HCl, 5 mM MgCl₂, 2.5 mM EGTA, 1% BSA. pH: 7,4). Data was fitted to 4-parameter logistic equation by employing GraphPad Prism software (V7.0) and Kᵢ data was calculated with the equation: *Kᵢ=IC₅₀*/*(1*+*(F*/*K_{D}))*; where IC₅₀ was the concentration of the ligand that displaced the specific binding of the radioligand in a 50%; K_{D} is the dissociation constant of the radioligand, and F is the concentration of the radioligand employed in the assay.

### Functional experiments. Cell Culture and Transient Transfection.

HEK-293T cells were grown in Dulbecco's Modified Eagle's Medium (DMEM) medium (Gibco, Paisley, Scotland, United Kingdom) supplemented with 2 mM L-glutamine, 100 U/mL penicillin/streptomycin, MEM Non-Essential Amino Acids Solution (1/100) and 5% (v/v) heat inactivated Fetal Bovine Serum (FBS) (Invitrogen, Paisley, Scotland, United Kingdom). Cells were maintained in a humid atmosphere of 5% CO₂ at 37 °C. Cells were transiently transfected with the PEI (Polyethyleneimine, Sigma, St. Louis, MO, United States) method as previously described [Navarro et al., BMC Biol. 2018, 16, 1-15] and used for functional assays 48 h later.

### Neuronal primary cultures

To prepare primary neurons, brains from fetuses of pregnant CD1 mice were removed (gestational age: 19 days). Neurons were isolated as described in Hradsky et al. [J. Biol. Chem. 2011, 286, 36762-36776]. Briefly, after removal of the meninges, samples were dissected and digested with 0.25% trypsin (20 min at 37 °C). The effect of trypsin was stopped by adding an equal volume of culture medium (supplemented DMEM). A single-cell suspension was obtained by repeated pipetting followed by passage through a 100 µm-pore mesh. Pelleted (7 min, 200×g) cells were resuspended in 2 mL of supplemented DMEM and seeded at a density of 3.5×10⁵cells/mL in 6-well plates. After 24 h, the medium was replaced by neurobasal medium supplemented with 2 mM L-glutamine, 100 U/mL penicillin/streptomycin and 2 % (v/v) B27 medium (GIBCO, Waltham, MA, USA). Primary neurons were assayed after 14 days in culture. Using NeuN as a marker, the percentage of neurons in the culture was >90%.

### cAMP Determination

Signaling experiments have been performed as previously described [Navarro et al., J. Biol. Chem. 2010, 285, 27346-27359]. Two hours before initiating the experiment, HEK-293T cell-culture medium was replaced by serum-starved DMEM medium. Then, cells were detached, resuspended in growing medium containing 50 mM zardaverine (Tocris, Bristol, UK) and placed in 384-well microplates (2,500 cells/well). Cells were pretreated (15 min) with cannabinoid compounds (1 nM to 100 µM) -or vehicle- before adding 0.5 mM forskolin (Tocris, Bristol, UK) to induce cAMP accumulation. Readings were performed after 15 min incubation at 25 °C. Homogeneous Time Resolved Fluorescence (HTRF) energy transfer measures were performed using the Lance Ultra cAMP kit (PerkinElmer, Waltham, MA, United States). Fluorescence at 665 nm was analyzed in a PHERA star Flagship microplate reader equipped with an HTRF optical module (BMG Lab Technologies, Offenburg, Germany).

### Viability assay

Primary cultures of **striatal** neurons were treated for 24 h with selected test compounds (100 nM). Afterwards, cells were scrapped from the plate and resuspended in neurobasal medium supplemented with 2 mM L-glutamine, 100 U/mL penicillin/streptomycin and 2 % (v/v) B27 (GIBCO). Trypan blue staining was performed mixing 1 part of 0.4% trypan blue and 1 part of cell suspension in a plastic tube. After ~3 min of incubation at room temperature, 10 µl of the mixture were sampled in a Neubauer chamber and counted with a Countess II FL (Life Technologies, California, CA, USA). The unstained (viable) and stained (nonviable) cells were counted separately, and the percentage of viability was calculated as: total number of viable cells/total number of cells x 100.

### Human Microsomal Stability

The human microsomal stability of selected compounds was evaluated by following a previously described method [Prieto-Diaz et al., J. Med. Chem. 2023, 66, 890-912]. The human microsomes employed were purchased from Tebu-Xenotech. The compound was incubated with the microsomes at 37 °C in a 50 mM phosphate buffer (pH=7.4) containing 3 mM MgCl₂, 1 mM NADP, 10 mM glucose-6-phosphate and 1 U/mL glucose-6-phosphate-dehydrogenase. Samples (75 µL) were taken from each well at 0, 10, 20, 40 and 60 minutes and transferred to a plate containing 4 °C 75 µL acetonitrile and 30 µL of 0.5% formic acid in water were added for improving the chromatographic conditions. The plate was centrifuged (46000 g, 30 min) and supernatants were taken and analyzed in an UPLC-MS/MS (Xevo-TQD, Waters) by employing a BEH C18 column and an isocratic gradient of 0.1% formic acid in water: 0.1% formic acid acetonitrile (60:40). The metabolic stability of the compounds was calculated from the logarithm of the remaining compounds at each of the time point studied.

### Drug Transport Experiments

Caco-2 cells were grown in Dulbecco's high-glucose modified eagle medium, composed of 10% fetal bovine serum, 2 mM of glutamine, 100 U/mL of penicillin, and 0.1 mg/mL of streptomycin (all components purchased from Corning^{®}, Milan, Italy) in a humidified incubator at 37 °C with a 5% CO₂ atmosphere. The experiment started with the preparation of the Caco-2 monolayer, which occurred by seeding the cells (20,000/well) in Millicell plates (Millipore, Milan, Italy). Its growth was followed for 21 days by changing the medium occasionally and measuring its transepithelial electrical resistance (TEER) daily using an epithelial voltohmmeter (Millicell-ERS) until a TEER value > 240 ohms x cm² was reached. After 21 days, the plate was washed twice with Hank's balanced salt solution (HBSS) (Invitrogen). After the second wash, the wells were filled with buffer, and the plate was kept at 37°C for 30 min. After the incubation time, the HBSS buffer was replaced with the solutions of the compounds to be tested at a concentration of 1 × 10⁻⁴ M. The plates were placed in an incubator at 37°C for 120 min. The apparent permeability (Papp), in units of nm s⁻¹, was calculated as previously reported [Miciaccia et al., Pharmaceuticals 2024, 17, 135; Braconi et al., Eur. J. Med. Chem. 2023, 259, 115716].

### Calcein-AM experiment

This experiment was carried out as described by Contino et al. with minor modifications [Contino et al., Eur. J. Med. Chem. 2020, 208, 112843-112843]. Each cell line (30,000 cells per well) was seeded into black CulturePlate 96/wells plate with 100 µL medium and allowed to become confluent overnight. 100 µL of test compounds were solubilized in culture medium and added to monolayers, with final concentrations ranging from 0.1 to 100 µM. 96/Wells plate was incubated at 37 °C for 30 min. Calcein-AM was added in 100 µL of Phosphate Buffered Saline (PBS) to yield a final concentration of 2.5 µM and plate was incubated for 30 min. Each well was washed 3 times with ice cold PBS. Saline buffer was added to each well and the plate was read with Victor3 (PerkinElmer) at excitation and emission wavelengths of 485 nm and 535 nm, respectively. In these experimental conditions Calcein cell accumulation in the absence and in the presence of tested compounds was evaluated and fluorescence basal level was estimated with untreated cells. In treated wells the increase of fluorescence with respect to basal level was measured. EC₅₀ values were determined by fitting the fluorescence increase percentage versus log[dose].

*Anti-Inflammatory and Pro-Inflammatory Cytokine Detection* The amount of cytokines was measured in 5 µL of supernatants, derived from 1 × 10⁴ cells, using the ProQuantum immunoassays kits for TNF-α, IFN-γ, IL-1β, IL-6, IL-10 and IL-4 [all from ThermoFisher Scientific (Waltham, MA)] according to the manufacturer's instructions. The results were expressed as pg/mL based on the titration curved of each kits.

### Data and statistical analysis

Kᵢ and EC₅₀ values were obtained by fitting the data with nonlinear regression using Prism 9 software (GraphPad, San Diego, CA). Results are the mean of four experiments (n = 4), each performed in duplicate. Data are represented as mean ± standard error of mean (SEM) with statistical significance set at P < 0.05. The number of samples (n) in each experimental condition is indicated in the corresponding tables. Outliers were assessed by the ROUT method [Hanuš et al., Proc. Natl. Acad. Sci. 1999, 96, 14228-14233], thus any sample was excluded assuming a Q value of 1% in GraphPad Prism 9. Comparisons among experimental groups were performed by Student's t test or one-way analysis of variance (ANOVA) followed by Tukey's multiple comparisons post-hoc test using GraphPad Prism 9, as indicated.

### Synthesis results

### Intermediate esters of formula (IV) wherein R⁵ is methyl:

**(S)-Methyl 1-(1-pentyl-1*H*-indole-3-carbonyl)piperidine-2-carboxylate (IVa).** Yield: 79%. ¹H NMR (300 MHz, CDCl₃) δ (ppm): 7.77 (d, *J =* 7.0 Hz, 1H, Ar-H), 7.47 (s, 1H, Ar-H), 7.36 (d, *J =* 7.4 Hz, 1H, Ar-H), 7.30 - 7.16 (m, 2H, Ar-H), 5.50 - 5.33 (m, 1H, CH), 4.13 (t, *J =* 7.2 Hz, 2H, C*H*₂CH₂CH₂CH₂CH₃), 3.80 (s, 3H, OCH₃), 3.38 - 3.22 (m, 1H, Pip), 2.33 (d, *J =* 13.5 Hz, 1H, Pip), 1.97 - 1.73 (m, 4H, Pip, CH₂C*H*₂CH₂CH₂CH₃), 1.71 - 1.43 (m, 3H, Pip), 1.43 - 1.25 (m, 5H, CH₂CH₂C*H*₂CH₂CH₃, CH₂CH₂CH₂C*H*₂CH₃, Pip), 0.89 (t, J= 6.8 Hz, 3H, CH₂CH₂CH₂CH₂C*H*₃). ¹³C NMR (75 MHz, CDCl₃) δ (ppm): 172.36, 168.01, 135.93, 130.53, 126.44, 122.38, 120.92, 120.84, 110.55, 110.04, 55.02, 52.32, 46.88, 44.96, 29.87, 29.23, 27.33, 25.81, 22.41, 21.53, 14.04. LRMS (ESI) m/z calcd. for C₂₁H₂₉N₂O₃ [M+H]⁺: 357.2; found: 357.2.

(R)-Methyl 1-(1-pentyl-1*H*-indole-3-carbonyl)piperidine-2-carboxylate (IVb). Yield: 75%. ¹H NMR (300 MHz, CDCl₃) δ (ppm): 7.77 (d, *J =* 7.8 Hz, 1H, Ar-H), 7.46 (s, 1H, Ar-H), 7.36 (d, *J =* 7.4 Hz, 1H, Ar-H), 7.31 - 7.15 (m, 2H, Ar-H), 5.48 - 5.29 (m, 1H, CH), 4.12 (t, *J =* 7.2 Hz, 2H, C*H*₂CH₂CH₂CH₂CH₃), 3.79 (s, 3H, OCH₃), 3.41 - 3.20 (m, 1H, Pip), 2.32 (d, *J* = 13.6 Hz, 1H, Pip), 1.95 - 1.72 (m, 4H, CH₂C*H*₂CH₂CH₂CH₃), 1.72 - 1.41 (m, 3H, Pip), 1.38 - 1.29 (m, 5H, CH₂CH₂C*H*₂CH₂CH₃, CH₂CH₂CH₂C*H*₂CH₃, Pip), 0.89 (t, *J* = 6.8 Hz, 3H, CH₂CH₂CH₂CH₂C*H*₃). LRMS (ESI) *m*/*z* calcd. for C₂₁H₂₉N₂O₃ [M+H]⁺: 357.2; found: 357.2.

(S)-Methyl 1-(1-(5-fluoropentyl)-1*H*-indole-3-carbonyl)piperidine-2-carboxylate (IVc). Yield: 83%. ¹H NMR (300 MHz, CDCl₃) δ (ppm): 7.76 (d, *J* = 7.6 Hz, 1H, Ar-H), 7.46 (s, 1H, Ar-H), 7.35 (d, *J =* 7.9 Hz, 1H, Ar-H), 7.29 - 7.17 (m, 2H, Ar-H), 5.50 - 5.26 (m, 1H, CH), 4.50 (t, *J* = 5.9 Hz, 1H, CH₂CH₂CH₂CH₂C*H*₂F), 4.34 (t, *J* = 5.9 Hz, 1H, CH₂CH₂CH₂CH₂C*H*₂F), 4.15 (t, *J* = 7.1 Hz, 2H, C*H*₂CH₂CH₂CH₂CH₂F), 3.78 (s, 3H, OCH₃), 3.39 - 3.18 (m, 1H, Pip), 2.32 (d, *J* = 13.4 Hz, 1H, Pip), 1.99 - 1.84 (m, 4H, CH₂CH₂CH₂C*H*₂CH₂F, CH₂C*H*₂CH₂CH₂CH₂F, Pip), 1.84 - 1.63 (m, 6H, CH₂CH₂CH₂ C*H*₂CH₂F, CH₂C*H*₂CH₂CH₂CH₂F, Pip) 1.55 - 1.41 (m, 2H, CH₂CH₂C*H*₂CH₂CH₂F). ¹³C NMR (75 MHz, CDCl₃) δ (ppm): 172.34, 164.72, 135.88, 130.39, 126.46, 122.51, 121.03, 120.90, 110.80, 109.95, 83.80 (d, *J* = 165.0 Hz), 54.23, 52.34, 46.72, 44.85, 30.12 (d, *J* = 19.8 Hz), 29.86, 27.33, 25.81, 23.04 (d, *J =* 4.9 Hz), 21.52. LRMS (ESI) *m*/*z* calcd. for C₂₁H₂₈FN₂O₃ [M+H]⁺: 375.2; found: 375.2.

(R)-Methyl 1-(1-(5-fluoropentyl)-1H-indole-3-carbonyl)piperidine-2-carboxylate (IVd). Yield: 74%. ¹H NMR (300 MHz, CDCl₃) δ (ppm): 7.77 (d, *J* = 7.5 Hz, 1H, Ar-H), 7.46 (s, 1H, Ar-H), 7.35 (d, *J =* 7.7 Hz, 1H, Ar-H), 7.30 - 7.17 (m, 2H, Ar-H), 5.48 - 5.29 (m, 1H, CH), 4.50 (t, *J* = 5.9 Hz, 1H, CH₂CH₂CH₂CH₂C*H*₂F), 4.35 (t, *J* = 5.9 Hz, 1H, CH₂CH₂CH₂CH₂C*H*₂F), 4.15 (t, *J* = 7.1 Hz, 2H, C*H*₂CH₂CH₂CH₂CH₂F), 3.79 (s, 3H, OCH₃), 3.38 - 3.20 (m, 1H, Pip), 2.32 (d, *J* = 13.4 Hz, 1H, Pip), 1.99 - 1.85 (m, 4H, CH₂CH₂CH₂C*H*₂CH₂F, CH₂C*H*₂CH₂CH₂CH₂F, Pip), 1.85 - 1.59 (m, 6H, CH₂CH₂CH₂C*H*₂ CH₂F, CH₂C*H*₂CH₂CH₂CH₂F, Pip), 1.52 - 1.41 (m, 2H, CH₂CH₂C*H*₂CH₂CH₂F). LRMS (ESI) *m*/*z* calcd. for C₂₁H₂₈FN₂O₃ [M+H]⁺: 375.2; found: 375.2.

(S)-Methyl 1-(1-(cyclohexylmethyl)-1H-indole-3-carbonyl)piperidine-2-carboxylate (IVe). Yield: 86%. ¹H NMR (300 MHz, CDCl₃) δ (ppm): 7.78 (d, *J* = 7.6 Hz, 1H, Ar-H), 7.44 (s, 1H, Ar-H), 7.36 (d, *J* = 8.4 Hz, 1H, Ar-H), 7.32 - 7.13 (m, 2H, Ar-H), 5.49 - 5.27 (m, 1H, CH), 4.31 - 4.07 (m, 1H, Pip), 3.96 (d, *J* = 7.3 Hz, 2H, *CH*₂-Cy)*,* 3.80 (s, 3H, OCH₃), 3.42 - 3.20 (m, 1H, Pip), 2.33 (d, *J =* 13.7 Hz, 1H, *CH-Cy),* 1.97 - 1.59 (m, 8H, Pip, Cy), 1.59 - 1.35 (m, 1H, Pip, Cy), 1.32 - 1.10 (m, 4H, Pip, Cy), 1.10 - 0.89 (m, 3H, Pip, Cy). LRMS (ESI) *m*/*z* calcd. for C₂₃H₃₁N₂O₃ [M+H]⁺: 383.2; found: 383.2.

***(*R)-Methyl** 1-(1-(cyclohexylmethyl)-1H-indole-3-carbonyl)piperidine-2-carboxylate (IVf). Yield: 74%. ¹H NMR (300 MHz, CDCl₃) δ (ppm): 7.75 (d, J= 7.8 Hz, 1H, Ar-H), 7.41 (s, 1H, Ar-H), 7.34 (d, *J* = 7.8 Hz, 1H, Ar-H), 7.28 - 7.13 (m, 2H, Ar-H), 5.52 - 5.21 (m, 1H, CH), 4.26 - 4.02 (m, 1H, Pip), 3.93 (d, *J* = 6.9 Hz, 2H, *CH*₂-Cy)*,* 3.77 (s, 3H, OCH₃), 3.38 - 3.17 (m, 1H, Pip), 2.30 (d, *J =* 13.6 Hz, 1H, *CH-Cy),* 1.93 - 1.55 (m, 9H, Pip, Cy), 1.55 - 1.32 (m, 1H, Pip, Cy), 1.28 - 1.10 (m, 4H, Pip, Cy), 1.08 - 0.88 (m, 2H, Pip, Cy). ¹³C NMR (75 MHz, CDCl₃) δ (ppm): 172.33, 167.99, 136.24, 131.18, 126.35, 122.30, 120.84, 120.76, 110.32, 110.26, 64.75, 53.36, 52.28, 38.72, 32.41, 31.16, 27.30, 26.34, 25.77, 24.04, 21.49. HRMS (ESI) *m*/*z* calcd. for C₂₃H₃₁N₂O₃ [M+H]⁺: 383.2329; found: 383.2323.

(S)-Methyl **1-(1-(4-fluorobenzyl)-1*H*-indole-3-carbonyl)piperidine-2-carboxylate** (IVg). Yield: 72%. ¹H NMR (300 MHz, CDCl₃) δ (ppm): 7.80 (dd, J = 6.3, 2.8 Hz, 1H, Ar-H), 7.48 (s, 1H, Ar-H), 7.32 - 7.20 (m, 3H, Ar-H), 7.19 - 7.10 (m, 2H, Ar-H), 7.06 - 6.97 (m, 2H, Ar-H), 5.30 (s, 2H, C*H*₂-Ph), 4.24 - 4.07 (m, 1H, CH), 3.79 (s, 3H, OCH₃), 2.34 (d, *J =* 13.4 Hz, 1H, Pip), 1.91 - 1.73 (m, 2H, Pip), 1.72 - 1.32 (m, 5H, Pip). LRMS (ESI) *m*/*z* calcd. for C₂₃H₂₄FN₂O₃ [M+H]⁺: 395.2; found: 395.2.

(R)-Methyl 1-(1-(4-fluorobenzyl)-1*H*-indole-3-carbonyl)piperidine-2-carboxylate (IVh). Yield: 78%. ¹H NMR (300 MHz, CDCl₃) δ **(ppm):** 7.78 (dd, *J* = 6.2, 3.1 Hz, 1H, Ar-H), 7.45 (s, 1H, Ar-H), 7.30 - 7.16 (m, 3H, Ar-H), 7.16 - 7.06 (m, 2H, Ar-H), 7.04 - 6.90 (m, 2H, Ar-H), 5.26 (s, 2H, C*H*₂-Ph), 4.31 - 3.92 (m, 1H, CH), 3.77 (s, 3H, OCH₃), 2.31 (d, *J =* 13.5 Hz, 1H, Pip), 1.87 - 1.70 (m, 2H, Pip), 1.69 - 1.31 (m, 5H, Pip). ¹³C NMR (75 MHz, CDCl₃) δ (ppm): 172.23, 167.70, 162.56 (d, *J =* 246.8 Hz), 136.02, 132.20 (d, *J = 3.1* Hz), 130.55, 128.95 (d, *J =* 8.3 Hz), 126.62, 122.82, 121.31, 120.92, 115.98 (d, *J* = 21.8 Hz), 111.41, 110.26, 54.43, 52.32, 49.94, 45.17, 27.28, 25.76, 21.46. HRMS (ESI) *m*/*z* calcd. for C₂₃H₂₄FN₂O₃ [M+H]⁺: 395.1765; found: 395.1773.

(S)-Methyl 1-(1-pentyl-1*H*-indazole-3-carbonyl)piperidine-2-carboxylate (IVi). Yield: 81%. ¹H NMR (300 MHz, CDCl₃) δ (ppm): 8.20 (d, *J* = 7.9 Hz, 1H, Ar-H), 7.46 - 7.33 (m, 2H, Ar-H), 7.29 - 7.17 (m, 1H, Ar-H), 5.66 - 5.55 (m, 1H, CH), 4.39 (t, *J =* 7.2 Hz, 2H, C*H*₂CH₂CH₂CH₂CH₃), 3.78 (s, 3H, OCH₃), 3.41 - 3.23 (m, 1H, Pip), 2.34 (t, *J* = 16.5 Hz, 1H, Pip), 2.00 - 1.85 (m, 3H, Pip, CH₂C*H*₂CH₂CH₂CH₃), 1.83 - 1.73 (m, 3H, Pip), 1.70 - 1.42 (m, 2H, Pip), 1.39 - 1.20 (m, 4H, CH₂CH₂C*H*₂CH₂CH₃, CH₂CH₂CH₂C*H*₂CH₃), 0.87 (t, *J* = 6.8 Hz, 3H, CH₂CH₂CH₂CH₂C*H*₃). ¹³C NMR (75 MHz, CDCl₃) δ (ppm): 172.00, 164.37, 138.99, 126.64, 123.10, 122.60, 122.23, 122.07, 109.15, 57.58, 52.59, 49.32, 42.73, 29.52, 29.33, 29.03, 27.34, 25.32, 21.89, 14.01. HRMS (ESI) *m*/*z* calcd. for C₂₀H₂₈N₃O₃ [M+H]⁺: 358.2125; found: 358.2128.

(R)-Methyl 1-(1-pentyl-1*H*-indazole-3-carbonyl)piperidine-2-carboxylate (IVj). Yield: 77%. ¹H NMR (300 MHz, CDCl₃) δ (ppm): 8.20 (d, *J =* 8.2 Hz, 1H, Ar-H), 7.47 - 7.34 (m, 2H, Ar-H), 7.33 - 7.15 (m, 1H, Ar-H), 5.68 - 5.54 (m, 1H, CH), 4.39 (t, *J =* 7.1 Hz, 2H, C*H*₂CH₂CH₂CH₂CH₃), 3.78 (s, 3H, OCH₃), 3.41 - 3.22 (m, 1H, Pip), 2.33 (t, *J* = 16.6 Hz, 1H, Pip), 2.02 - 1.85 (m, 3H, Pip, CH₂C*H*₂CH₂CH₂CH₃), 1.85 - 1.72 (m, 3H, Pip), 1.71 - 1.53 (m, 2H, Pip), 1.52 - 1.17 (m, 4H, CH₂CH₂C*H*₂CH₂CH₃, CH₂CH₂CH₂C*H*₂CH₃), 0.86 (t, *J* = 6.8 Hz, 3H, CH₂CH₂CH₂CH₂C*H*₃). ¹³C NMR (75 MHz, CDCl₃) δ (ppm): 172.30, 164.35, 138.96, 126.65, 123.06, 122.56, 122.20, 122.04, 109.09, 57.55, 52.56, 49.28, 42.69, 29.48, 29.30, 29.00, 27.30, 25.28, 21.85, 13.98. HRMS (ESI) *m*/*z* calcd. for C₂₀H₂₈N₃O₃ [M+H]⁺: 358.2125; found: 358.2124.

(S)-Methyl **1-(1-(5-fluoropentyl)-1*H*-indazole-3-carbonyl)piperidine-2-carboxylate (IVk).** Yield: 79%. ¹H NMR (300 MHz, CDCl₃) δ (ppm): 8.18 (d, *J* = 8.2 Hz, 1H, Ar-H), 7.43 - 7.31 (m, 2H, Ar-H), 7.26 - 7.15 (m, 1H, Ar-H), 5.64 - 5.51 (m, 1H, CH), 4.45 (t, *J* = 5.9 Hz, 1H, CH₂CH₂CH₂CH₂C*H*₂F), 4.29 (t, *J =* 5.9 Hz, 1H, CH₂CH₂CH₂CH₂C*H*₂F), 4.07 (t, *J =* 6.9 Hz, 2H, C*H*₂CH₂CH₂CH₂CH₂F), 3.76 (s, 3H, OCH₃), 3.38 - 3.23 (m, 1H, Pip), 2.41 - 2.20 (m, 1H, Pip), 1.99 - 1.87 (m, 2H, CH₂CH₂CH₂C*H*₂CH₂F, CH₂C*H*₂CH₂CH₂CH₂F, Pip), 1.87 - 1.53 (m, 6H, CH₂CH₂CH₂C*H*₂CH₂F, CH₂C*H*₂CH₂CH₂CH₂F, Pip), 1.51 - 1.33 (m, 4H, CH₂CH₂C*H*₂CH₂CH₂F, Pip). LRMS (ESI) *m*/*z* calcd. for C₂₀H₂₇FN₃O₃ [M+H]⁺: 376.2; found: 376.2.

**(R)-Methyl 1-(1-(5-fluoropentyl)-1*H*-indazole-3-carbonyl)piperidine-2-carboxylate (IVI).** Yield: 80%. ¹H NMR (300 MHz, CDCl₃) δ (ppm): 8.19 (d, *J* = 8.1 Hz, 1H, Ar-H), 7.46 - 7.33 (m, 2H, Ar-H), 7.29 - 7.15 (m, 1H, Ar-H), 5.64 - 5.53 (m, 1H, CH), 4.46 (t, *J* = 6.0 Hz, 1H, CH₂CH₂CH₂CH₂C*H*₂F), 4.31 (t, *J* = 6.0 Hz, 1H, CH₂CH₂CH₂CH₂C*H*₂F), 4.11 (t, *J* = 7.0 Hz, 2H, C*H*₂CH₂CH₂CH₂CH₂F) 3.77 (s, 3H, OCH₃), 3.40 - 3.23 (m, 1H, Pip), 2.44 - 2.23 (m, 1H, Pip), 2.05 - 1.89 (m, 2H, CH₂CH₂CH₂C*H*₂CH₂F, CH₂C*H*₂CH₂CH₂CH₂F, Pip), 1.89 - 1.57 (m, 6H, CH₂CH₂CH₂C*H*₂CH₂F, CH₂C*H*₂CH₂CH₂CH₂F, Pip), 1.56 - 1.31 (m, 4H, CH₂CH₂C*H*₂CH₂CH₂F, Pip). ¹³C NMR (75 MHz, CDCl₃) δ (ppm): 172.29, 164.18, 139.07, 126.81, 124.33, 123.12, 122.60, 122.22, 109.00, 83.79 (d, *J =* 165.1 Hz), 57.57, 52.58, 49.04, 42.71, 30.00 (d, *J* = 19.7 Hz), 29.30, 27.32, 25.28, 22.76 (d, *J* = 5.1 Hz), 21.40. HRMS (ESI) *m*/*z* calcd. for C₂₀H₂₇FN₃O₃ [M+H]⁺: 376.2031; found: 376.2029.

(S)-Methyl **1-(1-(cyclohexylmethyl)-1H-indazole-3-carbonyl)piperidine-2-carboxylate (IVm).** Yield: 85%. ¹H NMR (300 MHz, CDCl₃) δ (ppm): 8.20 (d, *J =* 8.2 Hz, 1H, Ar-H), 7.43 - 7.33 (m, 2H, Ar-H), 7.28 - 7.16 (m, 1H, Ar-H), 5.64 - 5.54 (m, 1H, CH), 4.21 (d, *J* = 7.1 Hz, 2H, *CH*₂-Cy)*,* 3.78 (s, 3H, OCH₃), 3.39 - 3.25 (m, 2H, Pip), 2.43 - 2.24 (m, 1H, CH-Cy), 2.11 - 1.34 (m, 12H, Pip, Cy), 1.30 - 0.89 (m, 4H, Pip, Cy). LRMS (ESI) *m*/*z* calcd. for C₂₂H₃₀N₃O₃ [M+H]⁺: 384.2; found: 384.2.

(R)-Methyl 1-(1-(cyclohexylmethyl)-1H-indazole-3-carbonyl)piperidine-2-carboxylate (IVn). Yield: 80%. ¹H NMR (500 MHz, DMSO-*d₆*) δ (ppm): 7.99 (d, *J* = 8.2 Hz, 1H, Ar-H), 7.68 (dd, *J* = 8.5, 1.0 Hz, 1H, Ar-H), 7.43 (ddd, *J* = 8.3, 6.8, 1.1 Hz, 1H, Ar-H), 7.23 (ddd, *J* = 7.9, 6.8, 0.8 Hz, 1H, Ar-H), 4.60 - 4.53 (m, 1H, CH), 4.30 (d, *J =* 6.9 Hz, 2H, *CH*₂-Cy)*,* 3.71 (s, 3H, OCH₃), 2.24 - 2.17 (m, 1H, Pip), 2.03 - 1.93 (m, 1H, *CH-Cy*)*,* 1.85 - 1.76 (m, 1H, Pip), 1.76 1.36 (m, 12H, Pip, Cy), 1.24 - 1.01 (m, 4H, Pip, Cy). ¹³C NMR (75 MHz, CDCl₃) δ (ppm): 172.35, 164.39, 139.22, 126.60, 124.49, 122.80, 122.10, 109.34, 56.56, 52.85, 52.35, 42.73, 38.76, 31.02, 29.82, 27.33, 26.35, 25.73, 24.92, 21.47. HRMS (ESI) *m*/*z* calcd. for C₂₂H₃₀N₃O₃ [M+H]⁺: 384.2282; found: 384.2288.

(S)-Methyl 1-(1-(4-fluorobenzyl)-1*H*-indazole-3-carbonyl)piperidine-2-carboxylate (IVo). Yield: 92%. ¹H NMR (300 MHz, CDCl₃) δ (ppm): 8.22 (d, *J* = 8.2 Hz, 1H, Ar-H), 7.41 - 7.30 (m, 2H, Ar-H), 7.29 - 7.11 (m, 3H, Ar-H), 6.98 (t, *J* = 8.3 Hz, 2H, Ar-H), 5.58 (s, 2H, C*H*₂-Ph), 4.84 - 4.63 (m, 1H, CH), 3.79 (s, 3H, OCH₃), 3.42 - 3.25 (m, 2H, Pip), 2.45 - 2.24 (m, 1H, Pip), 1.93 - 1.35 (m, 5H, Pip). LRMS (ESI) *m*/*z* calcd. for C₂₂H₂₃FN₃O₃ [M+H]⁺: 396.2; found: 396.2.

(R)-Methyl 1-(1-(4-fluorobenzyl)-1*H*-indazole-3-carbonyl)piperidine-2-carboxylate (IVp). Yield: 87%. ¹H NMR (300 MHz, CDCl₃) δ (ppm): 8.22 (d, *J* = 8.1 Hz, 1H, Ar-H), 7.41 - 7.31 (m, 2H, Ar-H), 7.29 - 7.12 (m, 3H, Ar-H), 6.98 (t, *J =* 8.4 Hz, 2H, Ar-H), 5.58 (s, 2H, C*H*₂-Ph), 4.85 - 4.63 (m, 1H, CH), 3.79 (s, 3H, OCH₃), 3.43 - 3.23 (m, 2H, Pip), 2.46 - 2.26 (m, 1H, Pip), 1.93 - 1.34 (m, 5H, Pip). ¹³C NMR (75 MHz, CDCl₃) δ (ppm): 172.24, 164.21, 162.57 (d, *J =* 246.5 Hz), 139.38, 132.00 (d, *J =* 3.2 Hz), 129.26, 129.15 (d, *J = 8.2* Hz), 127.16, 124.73, 122.96, 122.48, 115.83 (d, *J =* 21.6 Hz), 109.27, 57.59, 52.83, 52.39, 42.77, 27.33, 25.28, 21.39. HRMS (ESI) *m*/*z* calcd. for C₂₂H₂₃FN₃O₃ [M+H]⁺: 396.1718; found: 396.1719.

### Final compounds of formula (I) wherein R² is H

(S)-1-(1-pentyl-1H-indole-3-carbonyl)piperidine-2-carboxamide (Ia). Compound of formula I, wherein R¹ is pentyl, R², R³ and R⁴ are H, X is CH, Y is CH₂, and n is 2. Yield: 81%. ¹H NMR (300 MHz, CDCl₃) δ (ppm): 7.77 (d, *J =* 7.7 Hz, 1H, Ar-H), 7.43 (s, 1H, Ar-H), 7.36 (d, *J* = 8.0 Hz, 1H, Ar-H), 7.30 - 7.16 (m, 2H, Ar-H), 6.75 (s, 1H, NH₂), 5.56 (s, 1H, NH₂), 5.26 - 5.13 (m, 1H, CH), 4.28 - 4.15 (m, 1H, Pip), 4.10 (t, *J =* 7.2 Hz, 2H, C*H*₂CH₂CH₂CH₂CH₃), 3.17 - 3.00 (m, 1H, Pip), 2.35 (d, *J =* 13.0 Hz, 1H, Pip), 1.94 - 1.55 (m, 7H, Pip, CH₂C*H*₂CH₂CH₂CH₃), 1.43 - 1.22 (m, 4H, CH₂CH₂C*H*₂CH₂CH₃, CH₂CH₂ CH₂C*H*₂CH₃), 0.88 (t, *J* = 6.5 Hz, 3H, CH₂CH₂CH₂CH₂C*H*₃). ¹³C NMR (75 MHz, CDCl₃) δ (ppm): 173.96, 168.48, 136.11, 130.74, 126.66, 122.69, 121.25, 120.76, 110.19, 109.67, 54.18, 46.96, 45.52, 29.84, 29.18, 26.04, 25.77, 22.38, 20.98, 14.02. LRMS (ESI) *m*/*z* calcd. for C₂₀H₂₈N₃O₂ [M+H]⁺: 342.2; found: 342.2.

(R)-1-(1-pentyl-1H-indole-3-carbonyl)piperidine-2-carboxamide (Ib). Compound of formula I, wherein R¹ is pentyl, R², R³ and R⁴ are H, X is CH, Y is CH₂, and n is 2. Yield: 83%. ¹H NMR (300 MHz, CDCl₃) δ (ppm): 7.78 (d, *J =* 7.7 Hz, 1H, Ar-H), 7.44 (s, 1H, Ar-H), 7.37 (d, *J =* 7.8 Hz, 1H, Ar-H), 7.31 - 7.17 (m, 2H, Ar-H), 6.78 (s, 1H, NH₂), 5.64 (s, 1H, NH₂), 5.28 - 5.16 (m, 1H, CH), 4.27 - 4.16 (m, 1H, Pip), 4.11 (t, *J =* 7.2 Hz, 2H, C*H*₂CH₂CH₂ CH₂CH₃), 3.17 - 3.03 (m, 1H, Pip), 2.36 (d, *J* = 13.1 Hz, 1H, Pip), 1.94 - 1.54 (m, 7H, Pip, CH₂C*H*₂CH₂CH₂CH₃), 1.42 - 1.20 (m, 4H, CH₂CH₂C*H*₂CH₂CH₃, CH₂CH₂CH₂C*H*₂CH₃), 0.89 (t, *J =* 6.6 Hz, 3H, CH₂CH₂CH₂CH₂C*H*₃). ¹³C NMR (75 MHz, CDCl₃) δ (ppm): 173.96, 168.46, 136.10, 130.71, 126.66, 122.68, 121.23, 120.77, 110.17, 109.67, 54.24, 46.95, 45.49, 29.84, 29.18, 26.03, 25.79, 22.37, 20.98, 14.01. LRMS (ESI) *m*/*z* calcd. for C₂₀H₂₈N₃O₂ [M+H]⁺: 342.2; found: 342.2.

**(*S*)-1-(1-(5-fluoropentyl)-1H-indole-3-carbonyl)piperidine-2-carboxamide** (Ic). Compound of formula I, wherein R¹ is 5-fluoropentyl, R², R³ and R⁴ are H, X is CH, Y is CH₂, and n is 2. Yield: 89%. ¹H NMR (300 MHz, CDCl₃) δ (ppm): 7.79 (d, *J =* 7.7 Hz, 1H, Ar-H), 7.46 (s, 1H, Ar-H), 7.37 (d, *J* = 7.9 Hz, 1H, Ar-H), 7.32 - 7.19 (m, 2H, Ar-H), 6.71 (s, 1H, NH₂), 5.42 (s, 1H, NH₂), 5.28 - 5.18 (m, 1H, CH), 4.51 (t, *J* = 5.9 Hz, 1H, CH₂CH₂CH₂CH₂C*H*₂F), 4.35 (t, *J* = 5.9 Hz, 1H, CH₂CH₂CH₂CH₂C*H*₂F), 4.17 (t, *J* = 7.1 Hz, 2H, C*H*₂CH₂CH₂CH₂CH₂F), 3.18 - 3.02 (m, 1H, Pip), 2.37 (d, *J =* 13.7 Hz, 1H, Pip), 1.99 - 1.85 (m, 2H, CH₂CH₂CH₂ C*H*₂CH₂F), 1.83 - 1.56 (m, 8H, CH₂C*H*₂CH₂CH₂CH₂F, Pip), 1.54 - 1.42 (m, 2H, CH₂CH₂ C*H*₂CH₂CH₂F). LRMS (ESI) *m*/*z* calcd. for C₂₀H₂₇FN₃O₂ [M+H]⁺: 360.2; found: 360.2.

(R)-1-(1-(5-fluoropentyl)-1H-indole-3-carbonyl)piperidine-2-carboxamide (Id). Compound of formula I, wherein R¹ is 5-fluoropentyl, R², R³ and R⁴ are H, X is CH, Y is CH₂, and n is 2. Yield: 72%. ¹H NMR (300 MHz, CDCl₃) δ (ppm): 7.79 (d, *J =* 7.7 Hz, 1H, Ar-H), 7.45 (s, 1H, Ar-H), 7.37 (d, *J* = 8.0 Hz, 1H, Ar-H), 7.33 - 7.19 (m, 2H, Ar-H), 6.74 (s, 1H, NH₂), 5.56 (s, 1H, NH₂), 5.27 - 5.17 (m, 1H, CH), 4.50 (t, *J* = 5.9 Hz, 1H, CH₂CH₂CH₂CH₂C*H*₂F), 4.35 (t, *J* = 5.9 Hz, 1H, CH₂CH₂CH₂CH₂C*H*₂F), 4.15 (t, *J* = 7.1 Hz, 2H, C*H*₂CH₂CH₂CH₂CH₂F), 3.19 - 3.03 (m, 1H, Pip), 2.36 (d, *J =* 13.0 Hz, 1H, Pip), 1.99 - 1.85 (m, 2H, CH₂CH₂ CH₂C*H*₂CH₂F), 1.83 - 1.60 (m, 8H, CH₂C*H*₂CH₂CH₂CH₂F, Pip), 1.54 - 1.41 (m, 2H, CH₂CH₂C*H*₂CH₂CH₂F). ¹³C NMR (75 MHz, CDCl₃) δ (ppm): 173.91, 168.37, 136.04, 130.61, 126.68, 122.80, 121.32, 120.82, 110.10, 109.87, 83.76 (d, *J=* 165.0 Hz), 54.04, 46.78, 45.47, 30.06 (d, *J =* 19.9 Hz), 29.81, 26.01, 25.81, 23.02 (d, *J* = 4.8 Hz), 20.96. LRMS (ESI) *m*/*z* calcd. for C₂₀H₂₇FN₃O₂ [M+H]⁺: 360.2; found: 360.2.

**(S)-1-(1-(cyclohexylmethyl)-1H-indole-3-carbonyl)piperidine-2-carboxamide** (Ie). Compound of formula I, wherein R¹ is cyclohexylmethyl, R², R³ and R⁴ are H, X is CH, Y is CH₂, and n is 2. Yield: 84%. ¹H NMR (300 MHz, CDCl₃) δ (ppm): 7.81 (dd, *J* = 8.0, 1.2 Hz, 1H, Ar-H), 7.42 (s, 1H, Ar-H), 7.40 - 7.34 (m, 1H, Ar-H), 7.32 - 7.19 (m, 2H, Ar-H), 6.80 (s, 1H, NH₂), 5.70 (s, 1H, NH₂), 5.28 - 5.18 (m, 1H, CH), 4.31 - 4.15 (m, 1H, Pip), 3.96 (d, *J = 7.2* Hz, 2H, *CH*₂-Cy)*,* 3.20 - 3.05 (m, 1H, Pip), 2.43 - 2.32 (m, 1H, CH-Cy), 1.96 - 1.53 (m, 11H, Pip, Cy), 1.30 - 1.13 (m, 3H, Pip, Cy), 1.11 - 0.92 (m, 2H, Pip, Cy). ¹³C NMR (75 MHz, CDCl₃) δ (ppm): 173.99, 168.41, 136.42, 131.37, 126.59, 124.60, 122.61, 121.15, 120.72, 110.41, 109.46, 54.37, 53.43, 45.43, 38.68, 31.13, 26.30, 26.00, 25.74, 20.97. HRMS (ESI) *m*/*z* calcd. for C₂₂H₃₀N₃O₂ [M+H]⁺: 368.2333; found: 368.2333.

**(R)-1-(1-(cyclohexylmethyl)-1H-indole-3-carbonyl)piperidine-2-carboxamide** (If). Compound of formula I, wherein R¹ is cyclohexylmethyl, R², R³ and R⁴ are H, X is CH, Y is CH₂, and n is 2. Yield: 90%. ¹H NMR (300 MHz, CDCl₃) δ (ppm): 7.81 (dd, J= 7.5, 1.6 Hz, 1H, Ar-H), 7.42 (s, 1H, Ar-H), 7.40 - 7.35 (m, 1H, Ar-H), 7.33 - 7.19 (m, 2H, Ar-H), 6.83 (s, 1H, NH₂), 5.81 (s, 1H, NH₂), 5.28 - 5.19 (m, 1H, CH), 4.31 - 4.17 (m, 1H, Pip), 3.96 (d, *J =* 7*.*1 Hz, 2H, *CH*₂-Cy)*,* 3.21 - 3.05 (m, 1H, Pip), 2.43 - 2.31 (m, 1H, CH-Cy), 1.83 - 1.55 (m, 11H, Pip, Cy), 1.36 - 1.11 (m, 3H, Pip, Cy), 1.10 - 0.95 (m, 2H, Pip, Cy). ¹³C NMR (75 MHz, CDCl₃) δ (ppm): 173.89, 168.30, 136.30, 131.26, 126.47, 122.50, 121.04, 120.61, 110.30, 109.34, 54.01, 53.32, 45.38, 38.57, 31.02, 26.19, 25.89, 25.68, 25.63, 20.86. HRMS (ESI) *m*/*z* calcd. for C₂₂H₃₀N₃O₂ [M+H]⁺: 368.2330; found: 368.2333.

**(S)-1-(1-(4-fluorobenzyl)-1H-indole-3-carbonyl)piperidine-2-carboxamide (Ig)**. Compound of formula I, wherein R¹ is 4-fluorobenzyl, R², R³ and R⁴ are H, X is CH, Y is CH₂, and n is 2. Yield: 73%. ¹H NMR (300 MHz, CDCl₃) δ (ppm): 7.86 - 7.75 (m, 1H, Ar-H), 7.44 (s, 1H, Ar-H), 7.32 - 7.18 (m, 3H, Ar-H), 7.17 - 7.06 (m, 2H, Ar-H), 7.06 - 6.93 (m, 2H, Ar-H), 6.73 (s, 1H, NH₂), 5.70 (s, 1H, NH₂), 5.27 (s, 2H, C*H*₂-Ph), 5.24 - 5.15 (m, 1H, CH), 4.28 - 4.09 (m, 1H, Pip), 3.21 - 3.00 (m, 1H, Pip), 2.33 (d, *J =* 13.0 Hz, 1H, Pip), 1.94 - 1.51 (m, 5H, Pip). ¹³C NMR (75 MHz, CDCl₃) δ (ppm): 173.87, 168.13, 162.57 (d, *J =* 247.1 Hz), 136.18, 132.04 (d, *J =* 3.3 Hz), 130.76, 128.88 (d, *J =* 8.1 Hz), 126.79, 123.09, 121.58, 120.84, 116.04 (d, *J* = 21.8 Hz), 110.49, 54.22, 49.95, 45.44, 40.07, 25.96, 25.87, 20.90. LRMS (ESI) *m*/*z* calcd. for C₂₂H₂₃FN₃O₂ [M+H]⁺: 380.2; found: 380.2.

***(R)-1-(1-(4-fluorobenzyl)-1H-indole-3-carbonyl)piperidine-2-carboxamide*** (Ih). Compound of formula I, wherein R' is 4-fluorobenzyl, R², R³ and R⁴ are H, X is CH, Y is CH₂, and n is 2. Yield: 79%. ¹H NMR (300 MHz, DMSO-*d₆*) δ (ppm): 7.84 (s, 1H, Ar-H), 7.72 (d, *J = 7.7* Hz, 1H, Ar-H), 7.51 (d, *J =* 7.9 Hz, 1H, Ar-H), 7.31 (dd, *J* = 9.2, 6.3 Hz, 3H, Ar-H), 7.22 - 7.08 (m, 5H, Ar-H, NH₂), 5.46 (s, 2H, C*H*₂-Ph), 5.07 - 4.90 (m, 1H, CH), 2.17 (d, *J =* 12.8 Hz, 1H, Pip), 1.72 - 1.54 (m, 3H, Pip), 1.53 - 1.32 (m, 2H, Pip), 1.32 - 1.19 (m, 2H, Pip). LRMS (ESI) *m*/*z* calcd. for C₂₂H₂₃FN₃O₂ [M+H]⁺: 380.2; found: 380.2.

**(*S*)-1-(1-pentyl-1*H*-indazole-3-carbonyl)piperidine-2-carboxamide (li).** Compound of formula I, wherein R¹ is pentyl, R², R³ and R⁴ are H, X is N, Y is CH₂, and n is 2. Yield: 79%. ¹H NMR (300 MHz, CDCl₃) δ (ppm): 8.21 - 8.05 (m, 1H, Ar-H), 7.51 - 7.37 (m, 2H, Ar-H), 7.35 - 7.21 (m, 1H, Ar-H), 7.17 (s, 1H, NH₂), 6.57 (s, 1H, NH₂), 5.90 - 5.38 (m, 1H, CH), 4.76 (d, *J* = 13.6 Hz, 1H, Pip), 4.38 (t, *J* = 7.2 Hz, 2H, C*H*₂CH₂CH₂CH₂CH₃), 3.28 - 3.08 (m, 1H, Pip), 2.45 - 2.24 (m, 1H, Pip), 2.01 - 1.50 (m, 7H, Pip, CH₂C*H*₂CH₂CH₂CH₃), 1.44 - 1.20 (m, 4H, CH₂CH₂C*H*₂CH₂CH₃, CH₂CH₂CH₂C*H*₂CH₃), 0.86 (t, *J* = 6.8 Hz, 3H, CH₂CH₂CH₂CH₂C*H*₃). ¹³C NMR (75 MHz, CDCl₃) δ (ppm): 173.65, 164.25, 138.81, 127.00, 124.51, 122.51, 109.32, 58.32, 53.15, 49.40, 45.75, 40.83, 29.44, 29.00, 26.56, 25.59, 22.29, 21.06, 14.00. HRMS (ESI) *m*/*z* calcd. for C₁₉H₂₇N₄O₂ [M+H]⁺: 343.2129; found: 343.2123.

**(R)-1-(1-pentyl-1H-indazole-3-carbonyl)piperidine-2-carboxamide (Ij).** Compound of formula I, wherein R¹ is pentyl, R², R³ and R⁴ are H, X is N, Y is CH₂, and n is 2. Yield: 88%. ¹H NMR (300 MHz, CDCl₃) δ (ppm): 8.27 - 8.03 (m, 1H, Ar-H), 7.53 - 7.35 (m, 2H, Ar-H), 7.34 - 7.21 (m, 1H, Ar-H), 7.17 (s, 1H, NH₂), 6.57 (s, 1H, NH₂), 6.09 - 5.36 (m, 1H, CH), 4.75 (d, *J* = 13.5 Hz, 1H, Pip), 4.36 (t, *J* = 7.2 Hz, 2H, C*H*₂CH₂CH₂CH₂CH₃), 3.31 - 3.06 (m, 1H, Pip), 2.45 - 2.24 (m, 1H, Pip), 2.06 - 1.47 (m, 7H, Pip, C*H*₂CH₂CH₂CH₂CH₃), 1.44 - 1.15 (m, 4H, CH₂CH₂C*H*₂CH₂CH₃, CH₂CH₂CH₂C*H*₂CH₃), 0.85 (t, J= 6.7 Hz, 3H, CH₂CH₂CH₂CH₂C*H*₃). ¹³C NMR (75 MHz, CDCl₃) δ (ppm): 173.65, 163.52, 138.82, 127.01, 124.52, 122.50, 109.32, 58.32, 53.13, 49.39, 45.73, 40.82, 29.44, 28.99, 26.54, 25.41, 22.28, 21.06, 14.00. HRMS (APCI) *m*/*z* calcd. for C₁₉H₂₇N₄O₂ [M+H]⁺: 343.2129; found: 343.2127.

**(*S*)-1-(1-(5-fluoropentyl)-1*H*-indazole-3-carbonyl)piperidine-2-carboxamide (Ik).** Compound of formula I, wherein R¹ is 5-fluoropentyl, R², R³ and R⁴ are H, X is N, Y is CH₂, and n is 2. Yield: 77%. ¹H NMR (300 MHz, CDCl₃) δ (ppm): 8.27 - 8.04 (m, 1H, Ar-H), 7.52 - 7.37 (m, 2H, Ar-H), 7.35 - 7.21 (m, 1H, Ar-H), 7.08 (s, 1H, NH₂), 6.55 (s, 1H, NH₂), 5.82 - 5.37 (m, 1H, CH), 4.76 (d, *J* = 13.4 Hz, 1H, Pip), 4.48 (t, *J* = 5.9 Hz, 1H, CH₂CH₂CH₂CH₂C*H*₂F), 4.40 (t, *J* = 7.9 Hz, 2H, C*H*₂CH₂CH₂CH₂CH₂F), 4.33 (t, *J =* 5.9 Hz, 1H, CH₂CH₂CH₂CH₂C*H*₂F), 3.27 - 3.06 (m, 1H, Pip), 2.48 - 2.24 (m, 1H, Pip), 2.08 - 1.92 (m, 2H, CH₂CH₂CH₂C*H*₂CH₂F), 1.91 - 1.58 (m, 7H, CH₂C*H*₂CH₂CH₂CH₂F, Pip), 1.56 - 1.37 (m, 2H, CH₂CH₂C*H*₂CH₂CH₂F). LRMS (ESI) *m*/*z* calcd. for C₁₉H₂₆FN₄O₂ [M+H]⁺: 361.2; found: 361.2.

***(R)-1-(1-(5-fluoropentyl)-1H-indazole-3-carbonyl)piperidine-2-carboxamide* (II).** Compound of formula I, wherein R¹ is 5-fluoropentyl, R², R³ and R⁴ are H, X is N, Y is CH₂, and n is 2. Yield: 75%. ¹H NMR (300 MHz, CDCl₃) δ (ppm): 8.23 - 8.04 (m, 1H, Ar-H), 7.50 - 7.37 (m, 2H, Ar-H), 7.34 - 7.20 (m, 1H, Ar-H), 7.08 (s, 1H, NH₂), 6.56 (s, 1H, NH₂), 5.90 - 5.38 (m, 1H, CH), 4.75 (d, *J* = 13.5 Hz, 1H, Pip), 4.48 (t, *J* = 5.9 Hz, 1H, CH₂CH₂CH₂CH₂C*H*₂F), 4.40 (t, *J* = 7.8 Hz, 2H, C*H*₂CH₂CH₂CH₂CH₂F), 4.32 (t, *J =* 5.9 Hz, 1H, CH₂CH₂CH₂CH₂C*H*₂F), 3.28 - 3.08 (m, 1H, Pip), 2.46 - 2.24 (m, 1H, Pip), 2.06 - 1.89 (m, 2H, CH₂CH₂CH₂CH₂C*H*₂F), 1.85 - 1.57 (m, 7H, CH₂C*H*₂CH₂CH₂CH₂F, Pip), 1.53 - 1.34 (m, 2H, CH₂CH₂C*H*₂CH₂CH₂F). ¹³C NMR (75 MHz, CDCl₃) δ (ppm): 173.65, 164.14, 138.88, 127.11, 124.49, 122.77, 122.55, 109.20, 83.77 (d, *J =* 164.9 Hz), 55.75, 49.10, 43.24, 29.95 (d, *J* = 19.9 Hz), 29.30, 26.59, 25.80, 25.24, 22.80 (d, *J* = 5.0 Hz), 21.02. LRMS (ESI) *m*/*z* calcd. for C₁₉H₂₆FN₄O₂ [M+H]⁺: 361.2; found: 361.2.

**(*S*)-1-(1-(cyclohexylmethyl)-1*H*-indazole-3-carbonyl)piperidine-2-carboxamide (Im).** Compound of formula I, wherein R¹ is cyclohexylmethyl, R², R³ and R⁴ are H, X is N, Y is CH₂, and n is 2. Yield: 84%. ¹H NMR (300 MHz, CDCl₃) δ (ppm): 8.23 - 8.00 (m, 1H, Ar-H), 7.49 - 7.33 (m, 2H, Ar-H), 7.32 - 7.23 (m, 1H, Ar-H), 7.18 (s, 1H, NH₂), 6.61 (s, 1H, NH₂), 5.93 - 5.35 (m, 1H, CH), 4.74 (d, *J =* 13.5 Hz, 1H, Pip), 4.20 (d, *J* = 7.5 Hz, 2H, C*H₂-*Cy)*,* 3.25 - 3.10 (m, 1H, Pip), 2.50 - 2.21 (m, 1H, C*H*-Cy), 2.18 - 1.90 (m, 1H, Pip), 1.88 - 1.42 (m, 10H, Pip, Cy), 1.36 - 0.92 (m, 5H, Pip, Cy). ¹³C NMR (75 MHz, CDCl₃) δ (ppm): 173.68, 163.52, 140.68, 137.34, 126.96, 124.36, 122.43, 109.55, 58.29, 55.66, 53.15, 45.73, 40.82, 38.73, 30.97, 29.79, 26.27, 25.71, 21.04. HRMS (APCI) *m*/*z* calc. for C₂₁H₂₀N₄O₂ [M+H]⁺: 369.2285; found: 369.2288.

**(*R*)-1-(1-(cyclohexylmethyl)-1*H*-indazole-3-carbonyl)piperidine-2-carboxamide (In).** Compound of formula I, wherein R¹ is cyclohexylmethyl, R², R³ and R⁴ are H, X is N, Y is CH₂, and n is 2. Yield: 94%. ¹H NMR (300 MHz, CDCl₃) δ (ppm): 8.22 - 8.04 (m, 1H, Ar-H), 7.48 - 7.36 (m, 2H, Ar-H), 7.31 - 7.22 (m, 1H, Ar-H), 7.19 (s, 1H, NH₂), 6.58 (s, 1H, NH₂), 5.79 - 5.37 (m, 1H, CH), 4.76 (d, *J =* 13.6 Hz, 1H, Pip), 4.22 (d, *J =* 7.6 Hz, 2H, C*H₂-*Cy), 3.26 - 3.06 (m, 1H, Pip), 2.48 - 2.21 (m, 1H, C*H*-Cy), 2.09 - 1.85 (m, 1H, Pip), 1.84 - 1.39 (m, 10H, Pip, Cy), 1.31 - 0.93 (m, 5H, Pip, Cy). ¹³C NMR (75 MHz, CDCl₃) δ (ppm): 173.67, 164.29, 140.71, 137.35, 127.00, 124.38, 122.46, 109.57, 58.30, 55.69, 53.18, 45.75, 40.82, 38.75, 30.99, 29.81, 26.28, 25.72, 21.05. LRMS (ESI) *m*/*z* calc. for C₂₁H₂₉N₄O₂ [M+H]⁺: 369.2; found: 369.2.

**(*S*)-1-(1-(4-fluorobenzyl)-1*H*-indazole-3-carbonyl)piperidine-2-carboxamide (lo).** Compound of formula I, wherein R¹ is 4-fluorobenzyl, R², R³ and R⁴ are H, X is N, Y is CH₂, and n is 2. Yield: 85%. ¹H NMR (300 MHz, CDCl₃) δ (ppm): 8.25 - 8.01 (m, 1H, Ar-H), 7.47 - 7.32 (m, 2H, Ar-H), 7.31 - 7.10 (m, 3H, Ar-H), 7.06 - 6.96 (m, 2H, Ar-H), 6.95 (s, 1H, NH₂), 6.54 (s, 1H, NH₂) 5.98 - 5.68 (m, 1H, CH), 5.57 (s, 2H, C*H*₂-Ph), 5.54 - 5.37 (m, 1H, Pip), 4.73 (d, *J* = 13.5 Hz, 1H, Pip), 3.25 - 3.11 (m, 1H, Pip), 2.46 - 2.19 (m, 1H, Pip), 1.86 - 1.38 (m, 4H, Pip). ¹³C NMR (75 MHz, CDCl₃) δ (ppm): 173.52, 167.57, 162.61 (d, *J =* 247.1 Hz), 139.21, 131.79 (d, *J =* 3.2 Hz), 129.33 (d, *J =* 8.1 Hz), 127.44, 124.80, 122.79, 115.93 (d, *J* = 21.7 Hz), 109.45, 58.35, 53.01, 45.75, 42.19, 40.83, 26.66, 25.42, 20.98. HRMS (ESI) *m*/*z* calc. for C₂₁H₂₂FN₄O₂ [M+H]⁺: 381.1721; found: 381.1723.

**(*R*)-1-(1-(4-fluorobenzyl)-1*H*-indazole-3-carbonyl)piperidine-2-carboxamide (Ip).** Compound of formula I, wherein R¹ is 4-fluorobenzyl, R², R³ and R⁴ are H, X is N, Y is CH₂, and n is 2. Yield: 80%. ¹H NMR (300 MHz, CDCl₃) δ (ppm): 8.22 - 8.05 (m, 1H, Ar-H), 7.47 - 7.33 (m, 2H, Ar-H), 7.31 - 7.13 (m, 3H, Ar-H), 7.05 - 6.97 (m, 2H, Ar-H), 6.95 (s, 1H, NH₂), 6.53 (s, 1H, NH₂), 5.82 - 5.63 (m, 1H, CH), 5.57 (s, 2H, C*H*₂-Ph), 5.55 - 5.38 (m, 1H, Pip), 4.73 (d, *J* = 13.4 Hz, 1H, Pip), 3.27 - 3.09 (m, 1H, Pip), 2.46 - 2.24 (m, 1H, Pip), 1.96 - 1.45 (m, 4H, Pip). ¹³C NMR (75 MHz, CDCl₃) δ (ppm): 173.46, 166.61, 162.67 (d, *J =* 246.8 Hz), 139.25, 131.77 (d, *J =* 3.2 Hz), 129.35 (d, *J =* 8.1 Hz), 127.52, 124.85, 122.85, 115.98 (d, *J* = 21.9 Hz), 109.47, 58.38, 52.91, 45.76, 40.85, 38.76, 26.62, 25.39, 21.02. HRMS (ESI) *m*/*z* calc. for C₂₁H₂₂FN₄O₂ [M+H]⁺: 381.1721; found: 381.1721.

### Final compounds of formula (I) wherein R² is not H

**N-(*tert*-butyl)-1-(1-(4-fluorobenzyl)-1*H*-indole-3-carbonyl)piperidine-2-carboxamide (Iq).** Compound of formula I, wherein R¹ is 4-fluorobenzyl, R² is *tert-*butyl, R³ and R⁴ are H, X is CH, Y is CH₂, and n is 2. ¹H NMR (300 MHz, CDCl₃) δ (ppm): 8.33 (s, 0.2H), 7.82 - 7.77 (m, 1H), 7.42 (s, 1H), 7.33 - 7.27 (m, 1H), 7.27 - 7.22 (m, 2H), 7.16 - 7.10 (m, 2H), 7.04 - 6.97 (m, 2H), 6.56 (s, 0.8H), 5.31 (s, 2H), 5.11 (br s, 1H), 4.18 (br s, 1H), 3.07 (br s, 1H), 2.38 - 2.26 (m, 1H), 1.88 - 1.53 (m, 5H),1.37 (s, 9H). HRMS (ESI) *m*/*z* calcd. for C₂₆H₃₁FN₃O₂ [M+H]⁺: 436,5514; found: 436.5522.

***N*-benzyl-1-(1-(4-fluorobenzyl)-1*H*-indole-3-carbonyl)piperidine-2-carboxamide (Ir).** Compound of formula I, wherein R¹ is 4-fluorobenzyl, R² is benzyl, R³ and R⁴ are H, X is CH, Y is CH₂, and n is 2. ¹H NMR (300 MHz, CDCl₃) δ (ppm): 8.19 - 8.13 (m, 1H), 7.57 - 7.53 (m, 1H), 7.49 - 7.43 (m, 2H), 7.41 - 7.36 (m, 3H), 7.32 - 7.29 (m, 3H), 7.22 (s, 1H), 7.15 - 7.12 (m, 1H), 7.09 - 7.06 (m, 2H), 6.57 (t, *J* = 6.1 Hz, 1H), 5.39 (s, 2H), 4.59 - 4.55 (m, 1H), 4.47 - 4.39 (m, 2H), 3.91 - 3.88 (m, 1H), 3.72 - 3.69 (m, 1H), 2.10 - 2.08 (m, 1H), 1.91 - 1.78 (m, 3H), 1.75 - 1.67 (m, 2H). HRMS (ESI) *m*/*z* calcd. for C₂₉H₂₉FN₃O₂ [M+H]⁺: 470.5684; found: 470.5682.

***N*-(2,3-dihydrobenzo[*b*][1,4]dioxin-6-yl)-1-(1-(4-fluorobenzyl)-1*H*-indole-3-carbonyl)piperidine-2-carboxamide (Is).** Compound of formula I, wherein R¹ is 4-fluorobenzyl, R² is 2,3-dihydrobenzo[b][1,4]dioxin-6-yl, R³ and R⁴ are H, X is CH, Y is CH₂, and n is 2. ¹H NMR (300 MHz, CDCl₃) δ (ppm): 9.17 (s, 1H), 8.12 - 8.09 (m, 1H), 7.55 - 7.50 (m, 1H), 7.48 - 7.41 (m, 2H), 7.38 - 7.35 (m, 1H), 7.24 (s, 1H), 7.21 (d, *J =* 8.8 Hz, 1H), 7.16 - 7.13 (m, 1H), 7.09 (s, 1H), 7.07 - 7.04 (m, 2H), 6.81 (d, *J* = 8.9 Hz, 1H), 5.41 (s, 2H), 4.57 - 4.54 (m, 1H), 4.32 - 4.29 (m, 2H), 4.27 - 4.22 (m, 2H), 3.95 - 3.91 (m, 1H), 3.79 - 3.72 (m, 1H), 2.17 - 2.13 (m, 1H), 1.98 - 1.91 (m, 1H), 1.88 - 1.65 (m, 4H). HRMS (ESI) *m*/*z* calcd. for C₃₀H₂₉FN₃O₄ [M+H]⁺: 514.5774; found: 514.5778.

**Methyl (1-(1-(4-fluorobenzyl)-1*H*-indole-3-carbonyl)piperidine-2-carbonyl) glycinate (It).** Compound of formula I, wherein R¹ is 4-fluorobenzyl, R² is -CH₂-COOMe, R³ and R⁴ are H, X is CH, Y is CH₂, and n is 2. ¹H NMR (300 MHz, CDCl₃) δ (ppm): 8.13 - 8.12 (m, 1H), 7.57 - 7.53 (m, 1H), 7.49 - 7.42 (m, 2H), 7.39 - 7.30 (m, 2H), 7.24 (s, 1H), 7.17 - 7.13 (m, 1H), 7.11 - 7.02 (m, 2H), 5.42 (s, 2H), 4.59 - 4.55 (m, 1H), 3.93 (d, *J* = 6.4 Hz, 2H), 3.90 - 3.85 (m, 1H), 3.73 (s, 3H), 3.72 - 3.68 (m, 1H), 2.11 - 2.06 (m, 1H), 1.89 - 1.73 (m, 3H), 1.72 - 1.61 (m, 2H). HRMS (ESI) *m*/*z* calcd. for C₂₅H₂₇FN₃O₄ [M+H]⁺: 452.5064; found: 452.5072.

***N*-cyclohexyl-1-(1-(4-fluorobenzyl)-1*H*-indazole-3-carbonyl)piperidine-2-carboxamide (Iu).** Compound of formula I, wherein R¹ is 4-fluorobenzyl, R² is cyclohexyl, R³ and R⁴ are H, X is N, Y is CH₂, and n is 2. ¹H NMR (300 MHz, CDCl₃) δ (ppm): 8.49 (d, *J* = 7.8 Hz, 1H), 7.83 (d, *J =* 7.5 Hz, 1H), 7.49 - 7.45 (m, 1H), 7.40 - 7.34 (m, 2H), 7.29 - 7.24 (m, 1H), 7.19 - 7.13 (m, 2H), 6.01 (brs, 1H), 5.72 (s, 2H), 4.71 - 4.67 (m, 1H), 3.98 - 3.87 (m, 2H), 3.78 - 3.72 (m, 1H), 2.11 - 2.05 (m, 1H), 1.90 - 1.84 (m, 1H), 1.81 - 1.73 (m, 4H), 1.72 - 1.59 (m, 5H), 1.52 - 1.43 (m, 5H). HRMS (ESI) *m*/*z* calcd. for C₂₇H₃₂FN₄O₂ [M+H]⁺: 463.5774; found: 463.5780.

***N*-benzyl-1-(1-(4-fluorobenzyl)-1*H*-indazole-3-carbonyl)piperidine-2-carboxamide (Iv).** Compound of formula I, wherein R¹ is 4-fluorobenzyl, R² is benzyl, R³ and R⁴ are H, X is N, Y is CH₂, and n is 2. ¹H NMR (300 MHz, CDCl₃) δ (ppm): 8.47 (d, *J* = 8.0 Hz, 1H), 7.77 (d, *J* = 7.8 Hz, 1H), 7.49 - 7.46 (m, 1H), 7.41 - 7.31 (m, 4H), 7.30 - 7.29 (m, 5H), 7.18 - 7.10 (m, 2H), 6.89 (t, *J* = 5.9 Hz, 1H), 5.75 (s, 2H), 4.75 - 4.71 (m, 1H), 4.51 - 4.39 (m, 2H), 3.99 - 3.89 (m, 1H), 3.81 - 3.72 (m, 1H), 2.15 - 2.06 (m, 1H), 1.89 - 1.71 (m, 3H), 1.70 - 1.59 (m, 2H). HRMS (ESI) *m*/*z* calcd. for C₂₈H₂₈FN₄O₂ [M+H]⁺: 471.5564; found: 471.5566.

***N*-(2,3-dihydrobenzo[*b*][1,4]dioxin-6-yl)-1-(1-(4-fluorobenzyl)-1*H*-indazole-3-carbonyl)piperidine-2-carboxamide (Iw).** Compound of formula I, wherein R¹ is 4-fluorobenzyl, R² is 2,3-dihydrobenzo[b][1,4]dioxin-6-yl, R³ and R⁴ are H, X is N, Y is CH₂, and n is 2. ¹H NMR (300 MHz, CDCl₃) δ (ppm): 9.25 (s, 1H), 8.49 - 8.45 (m, 1H), 7.81 - 7.76 (m, 1H), 7.51 - 7.47 (m, 1H), 7.41 - 7.34 (m, 2H), 7.29 - 7.22 (m, 1H), 7.21 - 7.10 (m, 3H), 7.09 (s, 1H), 6.83 (d, *J =* 9.1 Hz, 1H), 5.73 (s, 2H), 4.82 - 4.76 (m, 1H), 4.34 - 4.23 (m, 4H), 3.97 - 3.88 (m, 1H), 3.79 - 3.71 (m, 1H), 2.13 - 2.08 (m, 1H), 1.98 - 1.61 (m, 5H). HRMS (ESI) *m*/*z* calcd. for C₂₉H₂₈FN₄O₄ [M+H]⁺: 515.5654; found: 515.5648.

**1-(1-(4-fluorobenzyl)-1*H*-indazole-3-carbonyl)-N-(2-(methylamino)-2-oxoethyl)piperidine-2-carboxamide (Ix).** Compound of formula I, wherein R¹ is 4-fluorobenzyl, R² is -CH₂-CONHMe, R³ and R⁴ are H, X is N, Y is CH₂, and n is 2. ¹H NMR (300 MHz, CDCl₃) δ (ppm): 8.49 (d, *J* = 7.9 Hz, 1H), 7.79 - 7.75 (m, 1H), 7.50 (t, *J* = 6.4 Hz, 1H), 7.47 - 7.41 (m, 1H), 7.39 - 7.30 (m, 2H), 7.29 - 7.22 (m, 1H), 7.19 - 7.10 (m, 2H), 6.41 (brs, 1H), 5.73 (s, 2H), 4.79 - 4.72 (m, 1H), 3.99 - 3.83 (m, 3H), 3.77 - 3.70 (m, 1H), 3.25 (d, *J* = 5.1 Hz, 3H), 2.12 - 2.03 (m, 1H), 1.90 - 1.76 (m, 3H), 1.74 - 1.62 (m, 2H). HRMS (ESI) *m*/*z* calcd. for C₂₄H₂₇FN₅O₃ [M+H]⁺: 452.5104; found: 452.5112.

***N*-(*tert*-Butyl)-1-(1-(cyclohexylmethyl)-1*H*-indole-3-carbonyl)piperidine-2-carboxamide (ly).** Compound of formula I, wherein R¹ is cyclohexylmethyl, R² is *tert-*butyl, R³ and R⁴ are H, X is CH, Y is CH₂, and n is 2. ¹H NMR (300 MHz, CDCl₃) δ (ppm): 7.75 (s, 1H), 7.70 - 7.64 (m, 1H), 7.46 - 7.41 (m, 1H), 7.34 - 7.23 (m, 2H), 6.67 (s, 1H), 4.67 - 4.61 (m, 1H), 3.97 (d, *J* = 7.1 Hz, 2H), 3.91 - 3.83 (m, 1H), 3.73 - 3.64 (m, 1H), 2.11 - 2.02 (m, 1H), 1.89 - 1.74 (m, 4H), 1.74 - 1.47 (m, 6H), 1.47 - 1.29 (m, 6H), 1.26 (s, 9H). HRMS (ESI) *m*/*z* calcd. for C₂₆H₃₈N₃O₂ [M+H]⁺: 424.6090; found: 424.6094.

***N*-cyclohexyl-1-(1-(cyclohexylmethyl)-1H-indole-3-carbonyl)piperidine-2-carboxamide (Iz).** Compound of formula I, wherein R¹ is cyclohexylmethyl, R² is cyclohexyl, R³ and R⁴ are H, X is CH, Y is CH₂, and n is 2. ¹H NMR (300 MHz, CDCl₃) δ (ppm): 7.71 (s, 1H), 7.62 (m, 1H), 7.43 (d, *J* = 6.8 Hz, 1H), 7.34 - 7.23 (m, 2H), 5.75 (brs, 1H), 4.56 - 4.49 (m, 1H), 4.09 - 3.90 (m, 3H), 3.88 - 3.82 (m, 1H), 3.75 - 3.67 (m, 1H), 2.06 - 2.03 (m, 1H), 1.85 -1.17 (m, 26H). HRMS (ESI) *m*/*z* calcd. for C₂₈H₄₀N₃O₂ [M+H]⁺: 450.6470; found: 450.6476.

**1-(1-(cyclohexylmethyl)-1*H*-indole-3-carbonyl)-*N*-(pyridin-3-ylmethyl)piperidine-2-carboxamide (laa).** Compound of formula I, wherein R¹ is cyclohexylmethyl, R² is pyridin-3-ylmethyl, R³ and R⁴ are H, X is CH, Y is CH₂, and n is 2. ¹H NMR (300 MHz, CDCl₃) δ (ppm): 8.54 (s, 1H), 8.49 - 8.44 (m, 1H), 7.81 - 7.75 (m, 2H), 7.65 (d, *J =* 6.9 Hz, 1H), 7.41 (d, *J* = 6.7 Hz, 1H), 7.32 - 7.24 (m, 3H), 6.35 (t, *J* = 5.8 Hz, 1H), 4.62 - 4.58 (m, 1H), 4.57 - 4.48 (m, 2H), 3.94 (d, *J =* 7.1 Hz, 2H), 3.89 - 3.83 (m, 1H), 3.75 - 3.67 (m, 1H), 2.10 - 2.02 (m, 1H), 1.91 - 1.77 (m, 4H), 1.75 - 1.48 (m, 6H), 1.47 - 1.13 (m, 6H). HRMS (ESI) *m*/*z* calcd. for C₂₈H₃₅N₄O₂ [M+H]⁺: 459.6140; found: 459.6138.

**Methyl (1-(1-(cyclohexylmethyl)-1*H*-indole-3-carbonyl)piperidine-2-carbonyl)glycinate (Ibb).** Compound of formula I, wherein R¹ is cyclohexylmethyl, R² is -CH₂-COOMe, R³ and R⁴ are H, X is CH, Y is CH₂, and n is 2. ¹H NMR (300 MHz, CDCl₃) δ (ppm): 7.71 (s, 1H), 7.69 - 7.62 (m, 1H), 7.45 (d, *J =* 6.3 Hz, 1H), 7.35 - 7.28 (m, 2H), 5.92 (brs, 1H), 4.59 - 4.52 (m, 1H), 3.96 (d, *J* = 5.1 Hz, 2H), 3.94 - 3.90 (m, 2H), 3.89 - 3.83 (m, 1H), 3.78 - 3.62 (m, 4H), 2.11 - 2.00 (m, 1H), 1.91 - 1.73 (m, 4H), 1.73 - 1.56 (m, 4H), 1.55 - 1.40 (m, 4H), 1.30 - 1.19 (m, 4H). HRMS (ESI) *m*/*z* calcd. for C₂₅H₃₄N₃O₄ [M+H]⁺: 440,5640; found: 440.5648.

***N*-(*tert*-butyl)-1-(1-(cyclohexylmethyl)-1*H*-indazole-3-carbonyl)piperidine-2-carboxamide (Icc).** Compound of formula I, wherein R¹ is cyclohexylmethyl, R² is *tert-*butyl, R³ and R⁴ are H, X is N, Y is CH₂, and n is 2. ¹H NMR (300 MHz, CDCl₃) δ (ppm): 8.47 (d, *J* = 7.6 Hz, 1H), 8.31 (d, *J* = 7.4 Hz, 1H), 7.32 - 7.29 (m, 1H), 7.27 - 7.20 (m, 1H), 5.67 (s, 1H), 4.69 - 4.60 (m, 1H), 4.25 (d, *J* = 4.6 Hz, 2H), 3.98 - 3.89 (m, 1H), 3.77 - 3.67 (m, 1H), 2.11 - 1.91 (m, 2H), 1.89 - 1.73 (m, 3H), 1.70 - 1.58 (m, 4H), 1.57 - 1.31 (m, 8H), 1.23 (s, 9H). HRMS (ESI) *m*/*z* calcd. for C₂₆H₃₈N₃O₂ [M+H]⁺: 424.6090; found: 424.6086.

***N*-cyclohexyl-1-(1-(cyclohexylmethyl)-1*H*-indazole-3-carbonyl)piperidine-2-carboxamide (Idd).** Compound of formula I, wherein R¹ is cyclohexylmethyl, R² is cyclohexyl, R³ and R⁴ are H, X is N, Y is CH₂, and n is 2. ¹H NMR (300 MHz, CDCl₃) δ (ppm): 8.37 (d, *J* = 7.3 Hz, 1H), 8.28 (d, *J* = 7.4 Hz, 1H), 7.37 - 7.29 (m, 1H), 7.27 - 7.19 (m, 1H), 5.94 (brs, 1H), 4.69 - 4.62 (m, 1H), 4.25 (d, *J* = 4.6 Hz, 2H), 4.01 - 3.86 (m, 2H), 3.78 - 3.70 (m, 1H), 2.12 - 1.96 (m, 2H), 1.89 - 1.72 (m, 5H), 1.71 - 1.45 (m, 8H), 1.44 - 1.18 (m, 12H). HRMS (ESI) *m*/*z* calcd. for C₂₈H₄₀N₃O₂ [M+H]⁺: 450.6470; found: 450.6472.

**1-(1-(cyclohexylmethyl)-1*H*-indazole-3-carbonyl)-*N*-(pyridin-3-ylmethyl)piperidine-2-carboxamide (lee).** Compound of formula I, wherein R¹ is cyclohexylmethyl, R² is pyridin-3-ylmethyl, R³ and R⁴ are H, X is N, Y is CH₂, and n is 2. ¹H NMR (300 MHz, CDCl₃) δ (ppm): 8.60 (s, 1H), 8.53 (d, *J* = 7.8 Hz, 1H), 8.50 - 8.47 (m, 1H), 8.39 - 8.33 (m, 1H), 7.82 - 7.75 (m, 1H), 7.42 - 7.24 (m, 4H), 4.71 - 4.64 (m, 1H), 4.59 - 4.47 (m, 2H), 4.22 (d, *J* = 4.6 Hz, 2H), 3.99 - 3.84 (m, 1H), 3.81 - 3.72 (m, 1H), 2.13 - 1.96 (m, 2H), 1.89 - 1.74 (m, 3H), 1.73 - 1.55 (m, 4H), 1.56 - 1.47 (m, 2H), 1.47 - 1.25 (m, 6H). HRMS (ESI) *m*/*z* calcd. for C₂₈H₃₅N₄O₂ [M+H]⁺: 459.6140; found: 459.6138.

**1-(1-(cyclohexylmethyl)-1*H*-indazole-3-carbonyl)-N-(2,3-dihydrobenzo[b] [1,4]dioxin-6-yl)piperidine-2-carboxamide (Iff).** Compound of formula I, wherein R¹ is cyclohexylmethyl, R² is 2,3-dihydrobenzo[b] [1,4]dioxin-6-yl, R³ and R⁴ are H, X is N, Y is CH₂, and n is 2. ¹H NMR (300 MHz, CDCl₃) δ (ppm): 9.21 (s, 1H), 8.51 (d, *J* = 7.7 Hz, 1H), 8.41 (d, *J =* 7.3 Hz, 1H), 7.37 - 7.31 (m, 1H), 7.29 - 7.24 (m, 1H), 7.19 (d, *J =* 8.7 Hz, 1H), 7.11 (s, 1H), 6.78 (d, *J* = 8.6 Hz, 1H), 4.79 - 4.71 (m, 1H), 4.33 - 4.21 (m, 4H), 4.19 (d, *J* = 4.6 Hz, 2H), 3.98 - 3.89 (m, 1H), 3.78 - 3.70 (m, 1H), 2.13 - 2.06 (m, 1H), 2.04 - 1.72 (m, 4H), 1.71 - 1.45 (m, 6H), 1.44 - 1.21 (m, 6H). HRMS (ESI) *m*/*z* calcd. for C₃₀H₃₆N₃O₄ [M+H]⁺: 502.6350; found: 502.6354.

***N*-(*tert*-butyl)-1-(1-(4-fluorobenzyl)-1*H*-indazole-3-carbonyl)pyrrolidine-2-carboxamide (Igg).** Compound of formula I, wherein R¹ is 4-fluorobenzyl, R² is *tert-*butyl*,* R³ and R⁴ are H, X is N, Y is CH₂, and n is 1. ¹H NMR (300 MHz, CDCl₃) δ (ppm): 8.49 (d, *J* = 7.9 Hz, 1H), 7.83 (d, *J =* 7.3 Hz, 1H), 7.46 - 7.41 (m, 1H), 7.39 - 7.32 (m, 2H), 7.29 - 7.23 (m, 1H), 7.19 - 7.09 (m, 2H), 5.87 (s, 1H), 5.69 (s, 2H), 4.53 - 4.48 (m, 1H), 3.89 - 3.81 (m, 1H), 3.76 - 3.67 (m, 1H), 2.24 - 2.14 (m, 1H), 2.09 - 1.76 (m, 3H), 1.23 (s, 9H). HRMS (ESI) *m*/*z* calcd. for C₃₀H₃₆FN₃O₂ [M+H]⁺: 423.5124; found: 423.5118.

**1-(1-(4-fluorobenzyl)-1*H*-indazole-3-carbonyl)-*N*-(pyridin-3-ylmethyl)pyrolidine-2-carboxamide (Ihh).** Compound of formula I, wherein R¹ is 4-fluorobenzyl, R² is pyridine-3-ylmethyl, R³ and R⁴ are H, X is N, Y is CH₂, and n is 1. ¹H NMR (300 MHz, CDCl₃) δ (ppm): 8.62 (s, 1H), 8.54 (d, *J* = 7.8 Hz, 1H), 8.52 - 8.47 (m, 1H), 7.84 - 7.72 (m, 2H), 7.51 - 7.47 (m, 1H), 7.39 - 7.29 (m, 3H), 7.28 - 7.20 (m, 2H), 7.18 - 7.09 (m, 2H), 5.73 (s, 2H), 4.59 - 4.42 (m, 3H), 3.89 - 3.81 (m, 1H), 3.76 - 3.67 (m, 1H), 2.21 - 2.17 (m, 1H), 2.06 - 1.79 (m, 3H). HRMS (ESI) *m*/*z* calcd. for C₂₆H₂₅FN₅O₂ [M+H]⁺: 458.5174; found: 458.5170.

**1-(1-(4-fluorobenzyl)-1*H*-indazole-3-carbonyl)-N-(2-(methylamino)-2-oxoethyl)pyrrolidine-2-carboxamide (lii).** Compound of formula I, wherein R¹ is 4-fluorobenzyl, R² is -CH₂-CONHMe, R³ and R⁴ are H, X is N, Y is CH₂, and n is 1. ¹H NMR (300 MHz, CDCl₃) δ (ppm): 8.49 (d, *J* = 8.0 Hz, 1H), 7.79 (d, *J =* 7.7 Hz, 1H), 7.51 (t, *J =* 6.4 Hz, 1H), 7.49 - 7.43 (m, 1H), 7.39 - 7.30 (m, 2H), 7.29 - 7.21 (m, 1H), 7.19 - 7.09 (m, 2H), 6.38 (brs, 1H), 5.74 (s, 2H), 4.49 - 4.42 (m, 1H), 3.96 - 3.73 (m, 3H), 3.69 - 3.62 (m, 1H), 3.19 (d, *J =* 4.8 Hz, 3H), 2.21 - 2.17 (m, 1H), 2.09 - 1.76 (m, 3H). HRMS (ESI) *m*/*z* calcd. for C₂₃H₂₄FN₄O₄ [M+H]⁺: 439.4674; found: 439.4682.

***N*-cyclohexyl-1-(1-(cyclohexylmethyl)-1*H*-indole-3-carbonyl)pyrrolidine-2-carboxamide (Ijj).** Compound of formula I, wherein R¹ is cyclohexylmethyl, R² is cyclohexyl, R³ and R⁴ are H, X is CH, Y is CH₂, and n is 1. ¹H NMR (300 MHz, CDCl₃) δ (ppm): 7.73 (s, 1H), 7.64 (d, *J* = 7.1 Hz, 1H), 7.45 (d, *J* = 6.5 Hz, 1H), 7.36 - 7.25 (m, 2H), 5.92 (brs, 1H), 4.42 - 4.38 (m, 1H), 4.02 - 3.88 (m, 3H), 3.78 - 3.70 (m, 1H), 3.65 - 3.58 (m, 1H), 2.21 - 2.11 (m, 1H), 2.08 - 1.96 (m, 1H), 1.92 - 1.80 (m, 3H), 1.77 - 1.65 (m, 2H), 1.63 - 1.45 (m, 6H), 1.44 - 1.19 (m, 12H). HRMS (ESI) *m*/*z* calcd. for C₂₇H₃₈N₃O₂ [M+H]⁺: 436.6200; found: 436.6198.

**1-(1-(cyclohexylmethyl)-1*H*-indole-3-carbonyl)-*N*-(2,3-dihydrobenzo[*b*][1,4]** dioxin-**6-yl)pyrrolidine-2-carboxamide (Ikk).** Compound of formula I, wherein R¹ is cyclohexylmethyl, R² is 2,3-dihydrobenzo[b][1,4]dioxin-6-yl, R³ and R⁴ are H, X is CH, Y is CH₂, and n is 1. ¹H NMR (300 MHz, CDCl₃) δ (ppm): 9.52 (s, 1H), 7.74 (s, 1H), 7.71 - 7.66 (m, 1H), 7.44 - 7.38 (m, 1H), 7.32 - 7.21 (m, 2H), 7.17 (d, *J =* 8.1 Hz, 1H), 7.09 (s, 1H), 6.81 (d, *J =* 7.9 Hz, 1H), 4.52 - 4.47 (m, 1H), 4.32 - 4.23 (m, 4H), 4.02 (d, *J* = 4.7 Hz, 2H), 3.86 - 3.79 (m, 1H), 3.70 - 3.72 (m, 1H), 2.31 - 2.24 (m, 1H), 2.10 - 1.85 (m, 4H), 1.69 - 1.26 (m, 10H). HRMS (ESI) *m*/*z* calcd. for C₂₉H₃₄N₃O₄ [M+H]⁺: 488.6080; found: 488.6084.

**Methyl (1-(cyclohexylmethyl)-1*H*-indole-3-carbonyl)prolylglycinate (III).** Compound of formula I, wherein R¹ is cyclohexylmethyl, R² is -CH₂-COOMe, R³ and R⁴ are H, X is CH, Y is CH₂, and n is 1. ¹H NMR (300 MHz, CDCl₃) δ (ppm): 7.72 (s, 1H), 7.69 - 7.62 (m, 1H), 7.48 - 7.43 (m, 1H), 7.37 - 7.25 (m, 2H), 6.15 (t, *J* = 6.3 Hz, 1H), 4.48 - 4.42 (m, 1H), 4.01 (d, *J =* 4.8 Hz, 2H), 3.98 - 3.92 (m, 2H), 3.78 - 3.69 (m, 4H), 3.64 - 3.57 (m, 1H), 2.22 - 2.18 (m, 1H), 2.09 - 1.98 (m, 1H), 1.96 - 1.81 (m, 3H), 1.71 - 1.24 (m, 10H). HRMS (ESI) *m*/*z* calcd. for C₂₄H₃₂N₃O₄ [M+H]⁺: 426.5370; found: 426.5372.

***N*-(*tert*-butyl)-1-(1-(cyclohexylmethyl)-1*H*-indazole-3-carbonyl)pyrrolidine-2-carboxamide (Imm).** Compound of formula I, wherein R¹ is cyclohexylmethyl, R² is *tert-*butyl, R³ and R⁴ are H, X is N, Y is CH₂, and n is 1. ¹H NMR (300 MHz, CDCl₃) δ (ppm): 8.42 (d, *J* = 8.0 Hz, 1H), 8.36 (d, *J* = 7.4 Hz, 1H), 7.35 - 7.29 (m, 1H), 7.27 - 7.21 (m, 1H), 6.21 (s, 1H), 4.48 - 4.42 (m, 1H), 4.21 (d, *J =* 4.6 Hz, 2H), 3.89 - 3.80 (m, 1H), 3.76 - 3.68 (m, 1H), 2.22 - 2.15 (m, 1H), 2.06 - 1.92 (m, 2H), 1.91 - 1.81 (m, 2H), 1.68 - 1.48 (m, 4H), 1.46 - 1.25 (m, 6H), 1.23 (s, 9H). HRMS (ESI) *m*/*z* calcd. for C₂₄H₃₅N₄O₂ [M+H]⁺: 411.5700; found: 411.5704.

***N*-benzyl-1-(1-(cyclohexylmethyl)-1H-indazole-3-carbonyl)pyrrolidine-2-carboxamide (Inn).** Compound of formula I, wherein R¹ is cyclohexylmethyl, R² is benzyl, R³ and R⁴ are H, X is N, Y is CH₂, and n is 1. ¹H NMR (300 MHz, CDCl₃) δ (ppm): 8.43 (d, *J =* 7.9 Hz, 1H), 8.35 (d, *J* = 7.3 Hz, 1H), 7.38 - 7.21 (m, 7H), 6.72 (t, *J =* 5.8 Hz, 1H), 4.52 - 4.48 (m, 1H), 4.44 - 4.39 (m, 2H), 4.19 (d, *J* = 4.6 Hz, 2H), 3.86 - 3.79 (m, 1H), 3.74 - 3.66 (m, 1H), 2.21 - 2.16 (m, 1H), 2.07 - 1.95 (m, 2H), 1.92 - 1.79 (m, 2H), 1.68 - 1.26 (m, 10H). HRMS (ESI) *m*/*z* calcd. for C₂₇H₃₃N₄O₂ [M+H]⁺: 445.5870; found: 445.5866.

***N*-(*tert*-butyl)-4-(1-(4-fluorobenzyl)-1*H*-indazole-3-carbonyl)morpholine-3-carboxamide (loo).** Compound of formula I, wherein R¹ is 4-fluorobenzyl, R² is *tert*-butyl, R³ and R⁴ are H, X is N, Y is O, and n is 2. ¹H NMR (300 MHz, CDCl₃) δ (ppm): 8.36 - 8.29 (m, 1H), 7.69 - 7.65 (m, 1H), 7.48 - 7.39 (m, 1H), 7.38 - 7.25 (m, 3H), 7.19 - 7.07 (m, 2H), 6.35 (brs, 1H), 5.77 (d, *J =* 12.4 Hz, 1H), 5.59 (d, *J =* 12.3 Hz, 1H), 4.58 - 4.49 (m, 1H), 3.98 - 3.91 (m, 2H), 3.89 - 3.72 (m, 4H), 1.28 (s, 9H). HRMS (ESI) *m*/*z* calcd. for C₂₄H₂₈FN₄O₃ [M+H]⁺: 439,5114; found: 439.5118.

***N*-cyclohexyl-4-(1-(4-fluorobenzyl)-1*H*-indazole-3-carbonyl)morpholine-3-carboxamide (Ipp).** Compound of formula I, wherein R¹ is 4-fluorobenzyl, R² is cyclohexyl, R³ and R⁴ are H, X is N, Y is O, and n is 2. ¹H NMR (300 MHz, CDCl₃) δ (ppm): 8.35 - 8.27 (m, 1H), 7.68 - 7.61 (m, 1H), 7.46 - 7.38 (m, 1H), 7.36 - 7.24 (m, 3H), 7.15 - 7.07 (m, 2H), 6.59 (d, *J =* 9.8 Hz, 1H), 5.75 (d, *J =* 12.4 Hz, 1H), 5.56 (d, *J =* 12.4 Hz, 1H), 4.57 - 4.47 (m, 1H), 4.03 - 3.95 (m, 2H), 3.93 - 3.60 (m, 5H), 1.78 - 1.21 (m, 10H). HRMS (ESI) *m*/*z* calcd. for C₂₆H₃₀FN₄O₃ [M+H]⁺: 465,5494; found: 465.5490.

***N*-(*tert*-butyl)-4-(1-(cyclohexylmethyl)-1*H*-indazole-3-carbonyl)morpholine-3-carboxamide (Iqq).** Compound of formula I, wherein R¹ is cyclohexylmehtyl, R² is *tert-*butyl, R³ and R⁴ are H, X is N, Y is O, and n is 2. ¹H NMR (300 MHz, CDCl₃) δ (ppm): 8.30 - 8.21 (m, 1H), 7.44 - 7.22 (m, 3H), 6.40 (brs, 1H), 4.54 - 4.45 (m, 1H), 4.20 (d, *J* = 7.1 Hz, 2H), 4.01 - 3.92 (m, 2H), 3.89 - 3.75 (m, 4H), 1.98 - 1.82 (m, 1H), 1.68 - 1.55 (m, 4H), 1.54 - 1.32 (m, 6H), 1.26 (s, 9H). HRMS (ESI) m/z calcd. for C₂₄H₃₅N₄O₃ [M+H]⁺: 427,5690; found: 427.5692.

Methyl 3-(tert-butylcarbamoyl)-4-(1-(4-fluorobenzyl)-1H-indazole-3-carbonyl)piperazine-1-carboxylate (Irr). Compound of formula I, wherein R¹ is 4-fluorobenzyl, R² is *tert*-butyl, R³ and R⁴ are H, X is N, Y is -NCOOMe, and n is 2. ¹H NMR (300 MHz, CDCl₃) δ (ppm): 8.29 - 8.19 (m, 1H), 7.71 - 7.68 (m, 1H), 7.49 - 7.37 (m, 2H), 7.34 - 7.23 (m, 2H), 7.12 - 7.04 (m, 2H), 6. 37 (brs, 1H), 5.77 (d, *J* = 12.3 Hz, 1H), 5.56 (d, *J* = 12.4 Hz, 1H), 4.97 - 4.85 (m, 1H), 3.91 - 3.71 (m, 4H), 3.69 (s, 3H), 3.57 - 3.48 (m, 2H), 1.28 (s, 9H). HRMS (ESI) *m*/*z* calcd. for C₂₆H₃₁FN₅O₄ [M+H]⁺: 496,5634; found: 496.5636.

**Methyl 3-(*tert*-butylcarbamoyl)-4-(1-(cyclohexylmethyl)-1*H*-indazole-3-carbonyl)piperazine-1-carboxylate (Iss).** Compound of formula I, wherein R¹ is cyclohexylmethyl, R² is *tert-*butyl*,* R³ and R⁴ are H, X is N, Y is -NCOOMe, and n is 2.¹H NMR (300 MHz, CDCl₃) δ (ppm): 8.29 - 8.16 (m, 1H), 7.70 - 7.26 (m, 3H), 6.39 (brs, 1H), 4.96 - 4.82 (m, 1H), 4.38 - 4.17 (m, 2H), 3.92 - 3.72 (m, 4H), 3.70 (s, 3H), 3.65 - 3.53 (m, 2H), 1.98 - 1.86 (m, 1H), 1.70 - 1.54 (m, 4H), 1.53 - 1.30 (m, 6H), 1.28 (s, 9H). HRMS (ESI) *m*/*z* calcd. for C₂₆H₃₈N₅O₄ [M+H]⁺: 484,6210; found: 484,6216.

### Circular Dichroism (CD)

Each stereoisomer of the enantiomeric pairs of compounds of formula (IV) and of formula (I) exhibited symmetrical CD spectra, mirrored across the x-axis, with opposite signs (thus providing valuable stereochemical information), irrespective of the functional group (ester or amide) within the amino acid residue. The distinctive CD signal at ~240 nm allowed for an unambiguous assignment of the absolute configuration of each enantiomer: the (S) enantiomer displayed a negative Cotton effect, whereas the (R) enantiomer produced a positive Cotton effect. These data provide valuable references for the unambiguous stereo-configuration assignment.

### Biological evaluation

**Cannabimimetic binding profile.** The cannabimimetic binding profile of the synthesized compounds of formula (I) was firstly studied *in vitro* by evaluating their affinity (Kᵢ) for human CB₁R and CB₂R following established experimental protocols previously described [Gioé-Gallo et al., Biomed. Pharmacother. 2023, 164, 114934]. Competitive radioligand binding assays were conducted using membrane preparations from human CB₁R transfected in CHO C3 cells and from human CB₂R transfected in HEK-293T cells. A well-characterized tritiated ligand ([³H]CP55,940) was used for these assays. The binding affinity data obtained are reported in Table 1. For comparative purposes, binding data for representative cannabinoid receptor ligands (Surinabant, CP55,940, and GW405833) were also determined using the reported experimental protocols. These data are also included in the table.

**Table 1. Structure and binding data obtained for the compounds of formula (I). ^{a)}Displacement of specific [³H]CP 55,940 binding in membrane preparations of human CB₁R transfected in CHO-CB₁ C3 cell line expressed as Kᵢ in nM (n = 3) or percentage displacement of specific binding at a concentration of 1 µM (n = 2). ^{b)}Displacement of specific [³H]CP 55,940 binding in membrane preparations of human CB₂R transfected in HEK-293T cells expressed as Kᵢ in nM (n = 3) or percentage displacement of specific binding at a concentration of 1 µM (n = 2).**

| **Compound** | **CB₁R** | **CB₂R** | **SI *Kᵢ*CB₁R/ *Kᵢ* CB₂R** |
|---|---|---|---|
| | ***Kᵢ* (nM) or % at 1 µM^{a)}** | ***Kᵢ* (nM) or % at 1 µM^{b)}** | |
| **Ia** | 3% | 111 ± 6 | >100 |
| **Ib** | 10% | 136 ± 5 | >100 |
| **Ic** | 7% | 132 ± 4 | >100 |
| **Id** | 15% | 169 ± 6 | >100 |
| **Ie** | 19% | 30.2 ± 1.2 | >100 |
| **If** | 11% | 96.8 ± 2.6 | >100 |
| **Ig** | 21% | 115 ± 5 | >100 |
| **Ih** | 3% | 207 ± 9 | >100 |
| **Ii** | 2% | 91.8 ± 4.9 | >100 |
| **Ij** | 2% | 145 ± 6 | >100 |
| **Ik** | 3% | 151 ± 6 | >100 |
| **Il** | 3% | 143 ± 5 | >100 |
| **Im** | 21% | 59.5 ± 2.8 | >100 |
| **In** | 26% | 112 ± 4 | >100 |
| **Io** | 3% | 54.6 ± 1.1 | >100 |
| **Ip** | 2% | 120 ± 5 | >100 |
| **Iq** | 21% | 35.1 | >100 |
| **Ir** | 33% | 77.3 | >100 |
| **Iv** | 8% | 33.1 | >100 |
| **Ix** | 26% | 10.4 | >100 |
| **Iy** | 5% | 42.1 | >100 |
| **Ijj** | 11% | 53.7 | >100 |
| **Inn** | 25% | 119 | >100 |
| **Iqq** | 34% | 135 | >100 |
| **Ipp** | 30% | 127 | >100 |
| **Surinabant** | 3.00 ± 0.2 | 400 ± 6 | 0.0075 |
| **GW405833** | 4772 ± 110 | 5.80 ± 0.4 | 822 |
| **CP55,940** | 1.01 ± 0.08 | 0.13 ± 0.08 | 7.76 |

As observed, compounds of formula I combine attractive CB₂R affinity (Kᵢ < 250 nM) and high subtype selectivity (> 100-fold).

**Intrinsic Activity Assays.** To gain deeper insight into the functional effects of the identified CB₂R ligands, a subset of compounds (I**e, If, Io,** and **Ip**) was selected. Initially we tested their ability to modify CB₂R-mediated cAMP accumulation, providing insights into their functional behavior as either agonists or antagonists (Table 2). Moreover, the signaling profiles of selected ligands were investigated through their impact on β-arrestin recruitment and Mitogen-Activated Protein Kinase (MAPK) phosphorylation pathway, enabling a more comprehensive evaluation of their intracellular signaling profiles (Table 2). A detailed description of the protocols employed is reported in the experimental part. An initial observation from the data (Table 2) reveals the identification of some compounds with EC₅₀ values in the low picomolar range across various signaling pathways, accompanied by Emax values indicative of either partial or full agonist profiles. The cAMP assay data presented in Table 2, expressed as the maximal effect achieved by JWH-133, provide unequivocal evidence that tested ligands behave as CB₂R agonists. A dose-dependent reduction in cAMP levels was observed for all evaluated compounds.

**Table 2. Structure, affinity, and functional data of selected derivatives. ^{a)}Displacement of specific [³H]CP 55,940 binding in membrane preparations of human CB₁R transfected in CHO-CB₁R C3 cell line expressed as Kᵢ in nM (n = 3) or percentage displacement of specific binding at a concentration of 1 µM (n = 2). ^{b)}Displacement of specific [³H]CP 55,940 binding in membrane preparations of human CB₂R transfected in HEK-293T cells expressed as Kᵢ in nM (n = 3) or percentage displacement of specific binding at a concentration of 1 µM (n = 2). ^{c)}Functional activity assessed in transfected HEK-293T cells by determining cAMP levels after forskolin stimulation. ^{d)}Emax values were normalized to a maximal efficacious concentration of CP 55,940. ^{e)}Bias factors were quantified by the relative activity model using JWH-133 as a reference ligand (see the Experimental Section). The preferred (biased) pathway is indicated in parentheses.**

| **Cpds** | **CB₁R** | **CB₂R** | | | | **Bias factor β-arrestin - cAMp^{e)}** | | **Bias factor MAPK - cAMP^{e)}** | | **Bias factor β-arrestin - MAPK^{e)}** | |
|---|---|---|---|---|---|---|---|---|---|---|---|
| | ***Kᵢ* or % at 1mM^{a)}** | ***Kᵢ* (nM) b)** | **EC₅₀ nM^{c)} (Emax)^{d)}** | | | | | | | | |
| | | | **AMPc** | **β-arrestin** | **MAPK** | $ΔΔ log \left(\frac{{E}_{max}}{{EC}_{50}}\right.$ | ${10}^{\left\|ΔΔ log\left(\frac{{E}_{max}}{{EC}_{50}}\right.\right.}$ | $ΔΔ log \left(\frac{{E}_{max}}{{EC}_{50}}\right.$ | ${10}^{\left\|ΔΔ log\left(\frac{{E}_{max}}{{EC}_{50}}\right.\right.}$ | $ΔΔ log \left(\frac{{E}_{max}}{{EC}_{50}}\right.$ | ${10}^{\left\|ΔΔ log\left(\frac{{E}_{max}}{{EC}_{50}}\right.\right.}$ |
| **Ie** | 22% | 30.2 | 1701 ± 12 (82%) | 0.40 ± 0.08 (120%) | 0.09 ± 0.002 (74%) | 3.12 (β-arrestin) | 1306 (β-arrestin) | 3.84 (MAPK) | 6843 (MAPK) | -0.72 (MAPK) | 5 (MAPK) |
| **If** | 11% | 96.8 | 118 ± 4 (55%) | 0.08 ± 0.01 (88%) | 0.07 ± 0.007 (94%) | 2.69 (β-arrestin) | 495 (β-arrestin) | 3.06 (MAPK) | 1156 (MAPK) | -0.37 (MAPK) | 2 (MAPK) |
| **Io** | 3% | 54.6 | 0.60 ± 0.09 (60%) | 60.9 ± 2.5 (148%) | 0.03 ± 0.003 (68%) | -2.29 (G protein) | 196 (G protein) | 0.96 (MAPK) | 9 (MAPK) | -3.25 (MAPK) | 1783 (MAPK) |
| **Ip** | 2% | 120 | 6.80 ± 0.3 (58%) | 326 ± 4 (77%) | 0.40 ± 0.02 (79%) | -2.24 (G protein) | 172 (G protein) | 0.97 (MAPK) | 9 (MAPK) | -3.20 (MAPK) | 1599 (MAPK) |
| **JWH-133** | - | - | 6.73 ± 0.5 (98%) | 1.34 ± 0.05 (93%) | 2.70 ± 0.19 (98%) | | 1 | | 1 | | 1 |

**BBB Permeability and P-glycoprotein Interaction Investigation.** The therapeutic potential of CB2R agonists in treating neurodegenerative diseases, hinges critically on their ability to cross the blood-brain barrier (BBB) and evade active efflux mechanisms, such as that mediated by P-glycoprotein (P-gP). The BBB is a highly selective barrier that regulates the passage of substances into the central nervous system (CNS), making it essential to evaluate drug candidates' permeability across this barrier [Pardridge, J. Cereb. Blood Flow Metab. 2012, 32, 1959-1972]. Furthermore, P-gP, a transporter protein expressed in the BBB, often actively pumps foreign substances out of the brain, limiting drug efficacy in CNS applications [Feng et al., J. Pharm. Sci. 2018, 107, 2225-2235]. Therefore, understanding both BBB permeability and P-gp interaction is crucial for developing CNS-acting agents, as effective compounds must exhibit both good brain penetration and minimal efflux to ensure sustained therapeutic concentrations within the CNS [Zhou et al., CNS Neurosci. Ther. 2019, 25, 665-673].

We experimentally assessed the BBB permeability and P-gp interaction of the selected CB₂R agonists. For these studies, two pairs of amide enantiomers (**Ip** and **Io**, **Ie**, and **If**) were selected, and the evaluations were conducted using established protocols previously published by our group [Graziano et al., Eur. J. Med. Chem. 2023, 248, 115109]. Key parameters measured included the permeability coefficients (*P*ₐₚₚBA and *P*ₐₚₚAB), the bidirectional permeability ratio (BA/AB), and the degree of interaction with P-gP, as detailed in Table 3.

**Table 6. Evaluation of the BBB permeability and P-gp interaction of selected CB₂R**

| **Ligand** | ***P*ₐₚₚBA (nm/sec)** | ***P*ₐₚₚAB (nm/sec)** | **Pₐₚₚ (BA/AB)** | **P-gp Interaction EC₅₀ (µM) or % at 100 µM** |
|---|---|---|---|---|
| **Io** | 290 | 727 | 0.39 | 49% |
| **Ip** | 219 | 632 | 0.35 | 56.9 µM |
| **Ie** | 506 | 995 | 0.51 | 30% |
| **If** | 393 | 792 | 0.49 | 61.8 µM |

The overall analysis of the experimental data (Table 3) reveals that the four tested compounds demonstrate good potential as CNS-targeted therapeutics based on their BBB permeability and P-glycoprotein (P-gp) interactions. All four compounds showed a noticeable ability to cross membranes, with particularly rapid transport from the apical compartment, where efflux pumps are expressed following polarization, to the basolateral compartment (*P*ₐₚₚAB). Because these derivatives exhibit low (EC₅₀ ranging from 56.9 to 61.8 µM) or negligible (30% and 49% inhibition at 100 µM) interaction with P-gp, they are expected to readily cross the BBB and reach their CNS target, avoiding P-gp-mediated efflux. In the permeability assay, le exhibited the most promising profile, with high PₐₚₚBA (506 nm/sec) and PₐₚₚAB (995 nm/sec) values and minimal P-gp interaction (30% inhibition at 1 µM), indicating strong BBB penetration and minimal efflux from the brain. This is further supported by its BA/AB ratio (0.51), indicative of balanced bidirectional permeability, thereby positioning it as a promising candidate for CNS applications, such as in neuroinflammation and neurodegenerative diseases. If also performed well, exhibiting a slightly lower PₐₚₚBA value of 393 nm/sec and a high PₐₚₚAB value of 792 nm/sec, along with low P-gp interaction (61.8 µM). Conversely, Ip and lo exhibited lower permeability and slightly higher P-gp interactions, although they are still predicted to cross the BBB.

**Anti-inflammatory profile of CB₂R agonists: Insights into Cytokine Production.** The CB₂R plays a crucial role in regulating immune and inflammatory responses [Rom et al., J. Neuroimmune Pharmacol. 2013, 8, 608-620]. Unlike CB₁R, which is predominantly expressed in the CNS system and associated with psychoactive effects, CB₂R is primarily found in peripheral tissues, including immune cells (e.g., monocytes, macrophages, and microglia). Its activation has been linked to anti-inflammatory and immunomodulatory effects, making it a promising target in conditions where chronic inflammation plays a central role in disease progression, including cancer, neurodegenerative diseases, and autoimmune diseases [Navarro et al., Front. Neurosci. 2016, 10, 406; Han et al., J. Med. Chem. 2013, 56, 8224-8256] Some selective CB₂R agonists have shown promise as therapeutic agents due to their ability to modulate cytokine profiles and reduce inflammation [Intranuovo et al., J. Med. Chem. 2023, 66, 235-250; Graziano et al., Eur. J. Med. Chem. 2023, 248, 115109].

Representative CB₂R agonists (**Ie**, **If**, **Io** and **Ip**) were evaluated for their ability to modulate the production of key pro- (e.g., TNF-α, IFN-γ, IL-1β, IL-6) and anti-inflammatory (e.g., IL-4, IL-10) cytokines in human monocytes and macrophages, where CB₂R is abundantly expressed [Turcotte et al., Cell. Mol. Life Sci. 2016, 73, 4449-4470]. The experiments were performed both in basal and LPS-activated states and would allow to gain insight into how these compounds affect circulating leukocytes (monocytes) and tissue-resident cells (macrophages). To mimic an inflammatory environment, both cell types were also treated with LPS, enabling an assessment of the anti-inflammatory potential of the compounds in both resting and activated states. Alongside the selected ligands, the study included HU308 [Hanuš et al., Proc. Natl. Acad. Sci. 1999, 96, 14228-14233], a well-established CB₂R-selective agonist known for its anti-inflammatory properties, and AM630 [Ross et al., Br. J. Pharmacol. 1999, 126, 665-672], a CB₂R-selective antagonist. AM630 was used alone and in combination with the test compounds to confirm that the observed effects were specifically mediated through CB₂R activation.

All tested compounds significantly reduced the production of pro-inflammatory cytokines (TNF-α, IFN-γ, IL-1β, and IL-6) in unstimulated monocytes and macrophages, demonstrating comparable or even superior efficacy to the reference CB₂R agonist HU308. Consistently, selected ligands also enhanced the production of anti-inflammatory cytokines (IL-4 and IL-10) to levels like those observed with HU308. The effects were even more pronounced in LPS-activated monocytes and macrophages, where a greater modulation of both pro- and anti-inflammatory cytokine levels was detected. As expected, the CB₂R antagonist AM630 reversed these effects across most tested conditions, confirming CB₂R-mediated activity.

These findings collectively highlight the anti-inflammatory properties of the selected CB₂R agonists. The maximum decrease in pro-inflammatory cytokines and the enhanced release of anti-inflammatory cytokines in LPS-stimulated cells indicate that these compounds are more effective in managing strong inflammatory responses, both in circulating immune cells (monocytes) and tissue-resident cells (macrophages). The cytokine modulation profiles observed support the hypothesis that these ligands promote a shift towards an anti-inflammatory immune response, reducing the detrimental effects of chronic inflammation. This makes them promising candidates for the development of new therapies addressing severe inflammatory conditions, such as systemic infections, sepsis, neuroinflammation, and certain types of cancer or scenarios where conventional anti-inflammatory treatments are insufficient. Moreover, the selective targeting of CB₂R without activating CB₁R ensures a favorable safety profile, minimizing the risk of psychoactive side effects, and positioning these ligands as promising candidates for neurodegenerative and oncological therapies.

**Evaluation of the neuroprotective effect of selected CB₂R agonists:** Activation of the CB₂R by selective small molecules offers significant neuroprotective potential, particularly for neurodegenerative diseases [Cabral and Griffin-Thomas, Expert Rev. Mol. Med. 2009, 11, e3]. Primarily expressed in immune cells and microglia in the CNS, CB₂R activation reduces neuroinflammation by lowering pro-inflammatory cytokines and promoting an anti-inflammatory state [Bala et al., Psychopharmacology (Berl.) 2024, 241, 1939-1954]. This effect is critical in pathologies such as Alzheimer's, Parkinson's, and Multiple Sclerosis, where inflammation plays a key role [Vuic et al., Biomedicines 2022, 10, 3000; Benito et al., J. Neurosci. 2003, 23, 11136-11141]. Additionally, CB₂R activation has been shown to decrease oxidative stress and apoptosis in neurons, supporting cell survival. Unlike CB₁R, CB₂R modulation avoids psychoactive effects, making it an attractive target for neuroprotective therapies.

A mouse primary cell experiment was performed to examine the neuroprotective effects of CB₂R agonists **Ie**, **If**, **Io**, and **Ip**. First, to exclude any intrinsic toxicity of the selected candidates, their potential effects on primary cell cultures were assessed. The results unequivocally confirmed that the selected CB₂R agonists did not affect primary cell survival.

Following the examination of the neuroprotective effects of herein developed CB₂R agonists, the selected derivatives (**Ie**, **If**, **Io**, and **Ip**) were evaluated in primary cultures exposed to LPS + IFN-γ, which caused a significant reduction (approximately 50%) in cell viability due to the induced inflammatory conditions. The addition of the studied compounds produced a marked recovery in cell viability (ranging from 77% to 93%), indicating their strong neuroprotective properties. These findings demonstrate the potential of these novel CB₂R agonists as promising therapeutic candidates for neuroinflammatory conditions.

Finally, to unequivocally confirm that the observed neuroprotective effect was indeed mediated by CB₂R activation, an additional experiment was conducted. In this experiment, primary cultures treated with LPS + IFN-γ, which exhibited reduced cell viability, were treated with compound **Io** in one set and with **Io** alongside the selective CB₂R antagonist SR144528 in another. The addition of ligand **Io** almost fully restored cell viability to near 100%. However, the presence of the antagonist effectively blocked the neuroprotective action of **Io**, preserving the reduced viability (~60%) observed with LPS + IFN-γ treatment alone. These results unequivocally demonstrate the significant neuroprotective effects of the CB₂R agonists are mediated through CB₂R activation.

## Claims

1. Compound of formula (I): 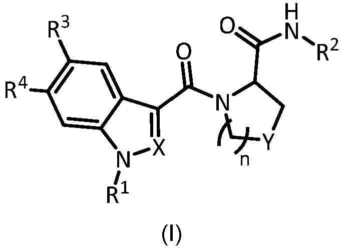 wherein
R¹ is selected from the group consisting of C₁-C₆ alkyl, -CH₂-C₃-C₈ cycloalkyl, and -CH₂-C₆-C₁₂ aryl, wherein each of said groups is optionally substituted by one or more halogen atoms;
R² is selected from the group consisting of H, C₁-C₆ alkyl, -(CH₂)ₚ-C₃-C₈ cycloalkyl, -(CH₂)ₚ-C₆-C₁₂ aryl, -(CH₂)ₚ-CH₂-heteroaryl wherein said heteroaryl is a 6- to 12-memebered monocyclic or bicyclic ring system containing one, two or three heteroatoms selected from N, O and S, -(CH₂)ₚ- heterocyclyl wherein said heterocyclyl is 3- to 8-membered monocyclic ring containing one or two heteroatoms selected from N, O and S, -CH₂C(=O)OR^{a}, and -CH₂-C(=O)NR^{a}; wherein p is selected from 0, 1 and 2; and R^{a} is H or C₁-C₆ alkyl;
R³ and R⁴ are independently selected from H and halogen atom, with the proviso that at least one of R³ and R⁴ is H;
X is CH or N;
Y is selected from the group consisting of CH₂, O, -NR^{b}, and -NC(=O)R^{b}, and - NC(=O)OR^{b}; wherein R^{b} is selected from C₁-C₆ alkyl and H; and
n is 1 or 2;
provided that when R² is H, n is 2;
or a stereoisomer or a pharmaceutically acceptable salt thereof.

2. Compound according to claim 1, wherein n is 2.

3. Compound according to any claim 1 or 2, wherein Y is CH₂ or O.

4. Compound according to any one of the preceding claims, wherein R¹ is selected from the group consisting of C₃-C₆ alkyl, -CH₂-C₅-C₇ cycloalkyl, and -CH₂-phenyl, wherein each of said groups is optionally substituted by one fluorine atom; preferably wherein R¹ is selected from the group consisting of C₄-C₅ alkyl optionally substituted with one fluorine atom, -CH₂-cyclohexyl, and -CH₂-phenyl substituted with one fluorine atom.

5. Compound according to any one of the preceding claims, wherein R² is selected from the group consisting of H, C₄-C₆ alkyl, -C₅-C₇ cycloalkyl, -CH₂-phenyl, -CH₂-heteroaryl wherein said heteroaryl is a 6- to 12-membered monocyclic or bicyclic ring system containing one or two heteroatoms selected from N and O, -(CH₂)₂-heterocyclyl wherein said heterocyclyl is a 5- to 7-memebered monocyclic ring containing one or two heteroatoms selected from N and O, and -CH₂-C(=O)N-C₁-C₃alkyl.

6. Compound according to claim 5, wherein R² is selected from the group consisting of H, *tert*-butyl, cyclohexyl, -CH₂-phenyl, -CH₂-pyridyl, -CH₂-C(=O)NCH₃, -CH₂-benzodioxanyl, and -(CH₂)₂-morpholinyl.

7. Compound according to any one of the preceding claims, wherein R³ and R⁴ are both H.

8. Compound of formula (I) according to claim 1, which is selected from the group consisting of:
1-(1-pentyl-1H-indole-3-carbonyl)piperidine-2-carboxamide,
1-(1-(5-fluoropentyl)-1H-indole-3-carbonyl)piperidine-2-carboxamide,
1-(1-(cyclohexylmethyl)-1H-indole-3-carbonyl)piperidine-2-carboxamide,
1-(1-(4-fluorobenzyl)-1H-indole-3-carbonyl)piperidine-2-carboxamide,
1-(1-pentyl-1H-indazole-3-carbonyl)piperidine-2-carboxamide,
1-(1-(5-fluoropentyl)-1H-indazole-3-carbonyl)piperidine-2-carboxamide,
1-(1-(cyclohexylmethyl)-1H-indazole-3-carbonyl)piperidine-2-carboxamide,
1-(1-(4-fluorobenzyl)-1H-indazole-3-carbonyl)piperidine-2-carboxamide,
*N*-(*tert*-butyl)-1-(1-(4-fluorobenzyl)-1*H*-indole-3-carbonyl)piperidine-2-carboxamide,
*N*-benzyl-1-(1-(4-fluorobenzyl)-1*H*-indole-3-carbonyl)piperidine-2-carboxamide,
*N*-(2,3-dihydrobenzo[*b*][1,4]dioxin-6-yl)-1-(1-(4-fluorobenzyl)-1*H*-indole-3-carbonyl)piperidine-2-carboxamide,
methyl (1-(1-(4-fluorobenzyl)-1*H*-indole-3-carbonyl)piperidine-2-carbonyl) glycinate,
*N*-cyclohexyl-1-(1-(4-fluorobenzyl)-1*H*-indazole-3-carbonyl)piperidine-2-carboxamide,
*N*-benzyl-1-(1-(4-fluorobenzyl)-1*H*-indazole-3-carbonyl)piperidine-2-carboxamide,
*N*-(2,3-dihydrobenzo[*b*][1,4]dioxin-6-yl)-1-(1-(4-fluorobenzyl)-1*H*-indazole-3-carbonyl)piperidine-2-carboxamide,
1-(1-(4-fluorobenzyl)-1*H*-indazole-3-carbonyl)-*N*-(2-(methylamino)-2-oxoethyl) piperidine-2-carboxamide,
*N*-(*tert*-butyl)-1-(1-(cyclohexylmethyl)-1*H*-indole-3-carbonyl)piperidine-2-carboxamide,
*N*-cyclohexyl-1-(1-(cyclohexylmethyl)-1*H*-indole-3-carbonyl)piperidine-2-carboxamide,
1-(1-(cyclohexylmethyl)-1*H*-indole-3-carbonyl)-*N*-(pyridin-3-ylmethyl)piperidine-2-carboxamide,
methyl (1-(1-(cyclohexylmethyl)-1*H*-indole-3-carbonyl)piperidine-2-carbonyl)glycinate,
*N*-(*tert*-butyl)-1-(1-(cyclohexylmethyl)-1*H-*indazole-3-carbonyl)piperidine-2-carboxamide,
*N*-cyclohexyl-1-(1-(cyclohexylmethyl)-1*H*-indazole-3-carbonyl)piperidine-2-carboxamide,
1-(1-(cyclohexylmethyl)-1*H*-indazole-3-carbonyl)-*N*-(pyridin-3-ylmethyl)piperidine-2-carboxamide,
1-(1-(cyclohexylmethyl)-1*H*-indazole-3-carbonyl)-*N*-(2,3-dihydrobenzo[*b*][1,4]dioxin-6-yl)piperidine-2-carboxamide,
*N*-(*tert*-butyl)-1-(1-(4-fluorobenzyl)-1*H*-indazole-3-carbonyl)pyrrolidine-2-carboxamide,
1-(1-(4-fluorobenzyl)-1*H-*indazole-3-carbonyl)-*N*-(pyridin-3-ylmethyl)pyrolidine-2-carboxamide,
1-(1-(4-fluorobenzyl)-1*H*-indazole-3-carbonyl)-*N*-(2-(methylamino)-2-oxoethyl)pyrrolidine-2-carboxamide,
*N*-cyclohexyl-1-(1-(cyclohexylmethyl)-1*H*-indole-3-carbonyl)pyrrolidine-2-carboxamide,
1-(1-(cyclohexylmethyl)-1*H*-indole-3-carbonyl)-*N*-(2,3-dihydrobenzo[*b*][1,4]dioxin-6-yl)pyrrolidine-2-carboxamide,
methyl (1-(cyclohexylmethyl)-1*H*-indole-3-carbonyl)prolylglycinate,
*N*-(*tert*-butyl)-1-(1-(cyclohexylmethyl)-1*H*-indazole-3-carbonyl)pyrrolidine-2-carboxamide,
N-benzyl-1-(1-(cyclohexylmethyl)-1H-indazole-3-carbonyl)pyrrolidine-2-carboxamide,
*N*-(*tert*-butyl)-4-(1-(4-fluorobenzyl)-1*H*-indazole-3-carbonyl)morpholine-3-carboxamide,
*N*-cyclohexyl-4-(1-(4-fluorobenzyl)-1*H*-indazole-3-carbonyl)morpholine-3-carboxamide,
*N*-(*tert*-butyl)-4-(1-(cyclohexylmethyl)-1*H*-indazole-3-carbonyl)morpholine-3-carboxamide,
methyl 3-(*tert*-butylcarbamoyl)-4-(1-(4-fluorobenzyl)-1*H*-indazole-3-carbonyl)piperazine-1-carboxylate, and
methyl 3-(*tert*-butylcarbamoyl)-4-(1-(cyclohexylmethyl)-1*H*-indazole-3-carbonyl)piperazine-1-carboxylate,
or a stereoisomer or a pharmaceutically acceptable salt thereof.

9. Pharmaceutical composition comprising a compound of formula (I), or a stereoisomer or a pharmaceutically acceptable salt thereof, as defined in any one of the preceding claims, and a pharmaceutically acceptable excipient.

10. Process for the preparation of a compound of formula (I) according to any one of claims 1 to 8, wherein R² is selected from the group consisting of C₁-C₆ alkyl, -(CH₂)ₚ-C₃-C₈ cycloalkyl, -(CH₂)ₚ-C₆-C₁₂ aryl, -(CH₂)ₚ-CH₂-heteroaryl wherein said heteroaryl is a 6- to 12-memebered monocyclic or bicyclic ring system containing one, two or three heteroatoms selected from the N and O, -(CH₂)ₚ- heterocyclyl wherein said heterocyclyl is 3- to 8-membered monocyclic ring containing one or two heteroatoms selected from N and O, -CH₂C(=O)OR^{a}, and -CH₂-C(=O)NR^{a}; wherein p is selected from 0, 1 and 2; and R^{a} is H or C₁-C₆ alkyl, and
wherein the process comprises the Ugi-Joullié reaction of a compound of formula (II) with a compound of formula (V) and a compound of formula (VI) to give the compound of formula (I), 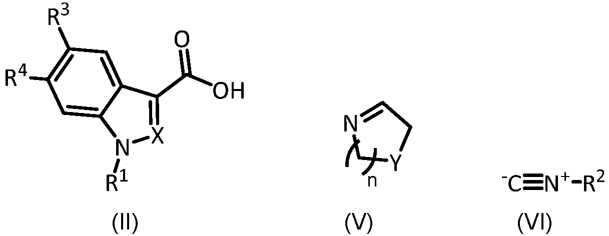 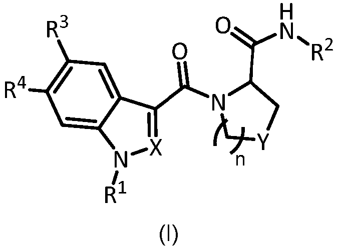
wherein R¹, R³, R⁴, X, Y and n are as defined in any one of claims 1 to 5, and R² is as defined above.

11. Process for the preparation of a compound of formula (I) according to any one of claims 1 to 8, wherein R² is H and n is 2, and wherein the process comprises:
a) amidation of a compound of formula (II) with a compound of formula (III) to give a compound of formula (IV) 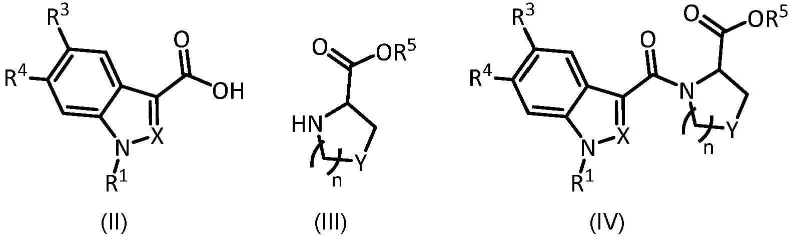 wherein R¹, R³, R⁴, X and Y are as defined in any one of claims 1 and 3 to 5, n is 2 and R⁵ is C₁-C₃ alkyl;
b) ammonolysis of the compound of formula (IV) obtained in step a) to a compound of formula (I) 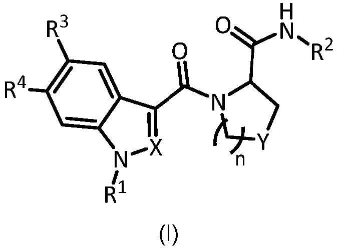 wherein R¹, R³, R⁴, X and Y are as defined in any one of claims 1 and 3 to 5, n is 2 and R² is H.

12. Compound of formula (IV) 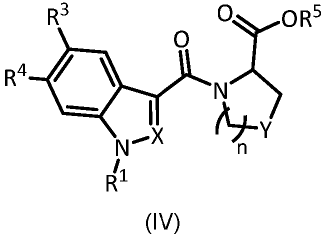 wherein R¹, R³, R⁴, X and Y are as defined in any one of claims 1 and 3 to 5, n is 2 and R⁵ is C₁-C₃ alkyl.

13. Compound according to claim 12, wherein R⁵ is methyl.

14. Compound of formula (I), or a stereoisomer or a pharmaceutically acceptable salt thereof, as defined in any one of claims 1 to 8, for use in medicine.

15. Compound of formula (I), or a stereoisomer or a pharmaceutically acceptable salt thereof, as defined in any one of claims 1 to 8, for use in the treatment and/or prevention of a disease selected from the group consisting of pathologies neuroinflammatory and neurodegenerative diseases, such as Alzheimer's disease, Parkinson's disease, multiple sclerosis, amyotrophic lateral sclerosis (ALS), Huntington's disease, and neuropathic pain; peripheral inflammatory diseases, such as rheumatoid arthritis, inflammatory bowel disease (such as Crohn's disease and ulcerative colitis), systemic lupus erythematosus, and psoriasis; cancer-related conditions associated with chronic inflammation and immune dysregulation, such as glioblastoma, pancreatic cancer, breast cancer, and colorectal cancer; chronic inflammatory diseases, such as diabetic retinopathy, chronic obstructive pulmonary disease (COPD), and atherosclerosis.
